(19) Europäisches Patentamt
European Patent Office
Office européen des brevets

(11) Veröffentlichungsnummer: **0 046 734**
**A2**

(12) # EUROPÄISCHE PATENTANMELDUNG

(21) Anmeldenummer: **81810343.4**

(22) Anmeldetag: **21.08.81**

(51) Int. Cl.³: **C 07 D 501/59**
C 07 D 501/00, A 61 K 31/545
//C07C125/065, C07C101/72

(30) Priorität: **27.08.80 CH 6446/80**

(43) Veröffentlichungstag der Anmeldung:
**03.03.82 Patentblatt 82/9**

(84) Benannte Vertragsstaaten:
**AT BE CH DE FR GB IT LI LU NL SE**

(71) Anmelder: **CIBA-GEIGY AG**
**Patentabteilung Postfach**
**CH-4002 Basel(CH)**

(72) Erfinder: **Wiederkehr, René, Dr.**
**Grenzweg 9**
**CH-4148 Pfeffingen(CH)**

(72) Erfinder: **Scartazzini, Riccardo, Dr.**
**Conrad-Ferdinand-Meyer-Strasse 38**
**CH-4059 Basel(CH)**

(54) **Aminohydroxyphenyl-Derivate von Cephalosporin, Verfahren zu ihrer Herstellung und diese enthaltende pharmazeutische Präparate.**

(57) Neue 7β-[D-2-Amino-2-(aminohydroxyphenyl)-acetylamino]-3-cephem-4-carbonsäureverbindungen der Formel

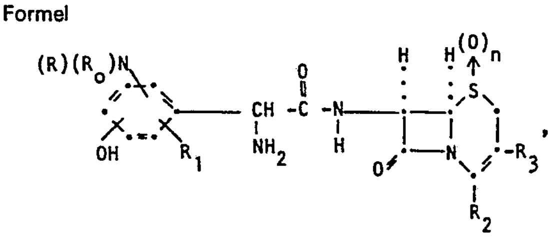

worin R Wasserstoff oder eine Acylgruppe, $R_o$ Wasserstoff oder Niederalkyl, $R_1$ Wasserstoff oder eine Gruppe der Teilformel $(R)(R_o)N$-, $R_2$ Carboxyl oder geschütztes Carboxyl und $R_3$ Wasserstoff, Halogen mit Ordnungszahl bis 35 oder Niederalkoxy bedeuten, werden hergestellt. Umfasst sind ebenfalls Zwischenprodukte.

Die neuen Verbindungen zeigen wertvolle antibiotische Eigenschaften und sind sowohl gegen grampositive als auch gegen gramnegative Mikroorganismen bei geringer Toxizität wirksam. Sie können daher zur Behandlung von entsprechenden Infektionen verwendet werden.

EP 0 046 734 A2

CIBA-GEIGY AG 4-13033/+

Basel (Schweiz)

BEZEICHNUNG GEÄNDERT
siehe Titelseite

Aminohydroxyphenylverbindungen, Verfahren zu ihrer Herstellung, pharmazeutische Präparate, welche diese Verbindungen enthalten, und Verwendung von letzteren.

Die vorliegende Erfindung betrifft neue 7β-[D-2-Amino-2-(aminohydroxyphenyl)-acetylamino]-3-cephem-4-carbonsäureverbindungen der Formel

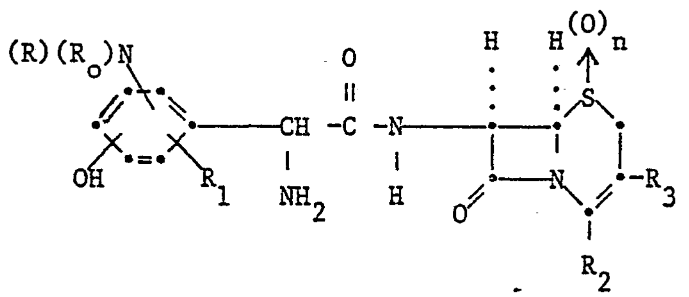

(I)

worin der Index n für 0 oder 1 steht, R Wasserstoff oder eine Acylgruppe, $R_o$ Wasserstoff oder Niederalkyl, $R_1$ Wasserstoff oder eine Gruppe der Teilformel $(R)(R_o)N-$, $R_2$ Carboxyl oder geschütztes Carboxyl und $R_3$ Wasserstoff, Halogen mit Ordnungszahl bis 35 oder Niederalkoxy bedeuten, sowie Hydrate und Salze von Verbindungen der Formel I, Verfahren zur Herstellung von Verbindungen der Formel I, pharmazeutische Präparate, die Verbindungen der Formel I enthalten, und ihre Verwendung zur Herstellung von pharmazeutischen Präparaten oder als pharmakologisch wirksame Verbindungen.

In der obigen Formel steht der Index n in erster Linie für 0. Falls n den Wert 1 hat, liegt die entsprechende 1-Oxid-Verbindung in der α- oder β-Form vor.

In der vorliegenden Beschreibung bedeutet der im Zusammenhang mit Definitionen von Substituenten oder Verbindungen verwendete Ausdruck "nieder", dass die entsprechenden Substituenten oder Verbindungen bis zu 7, bevorzugt bis zu 4, Kohlenstoffatome enthalten, sofern es nicht ausdrücklich anders definiert ist.

Die vor- und nachstehend verwendeten allgemeinen Definitionen haben im Rahmen der vorliegenden Beschreibung vorzugsweise die folgenden Bedeutungen:

Eine Acylgruppe R hat bis zu 19 Kohlenstoffatome und ist die Acylgruppe R einer Carbonsäure, eines Halbesters der Kohlensäure, der Carbaminsäure, einer substituierten Carbaminsäure, der Thiocarbaminsäure, einer substituierten Thiocarbaminsäure, einer Sulfonsäure, der Amidosulfonsäure oder einer substituierten Amidosulfonsäure.

Solche Acylgruppen R haben beispielsweise die Formeln: $R^a$-CO-, $R^a$-O-CO-, $R^aR^a$N-CO-, $R^aR^a$N-CS-, $R^a$-$SO_2$- oder $R^aR^a$N-$SO_2$-, worin $R^a$ Wasserstoff oder einen gegebenenfalls substituierten Kohlenwasserstoffrest darstellt und zwei Reste $R^a$ in einer Acylgruppe im Rahmen ihrer Bedeutungen gleich oder verschieden sein können.

Ein gegebenenfalls substituierter Kohlenwasserstoffrest $R^a$ in einer Acylgruppe R hat bis zu 18 C-Atome und ist beispielsweise

ein niederaliphatischer, cycloaliphatischer, cycloaliphatisch-aliphatischer, aromatischer oder araliphatischer Rest, der beispielsweise durch Niederalkyl, Niederalkoxy, Halogen, Nitro, Oxo, Hydroxy, gegebenenfalls funktionell abgewandeltes Carboxy, gegebenenfalls niederalkyliertes Amino oder eine entsprechende geschützte funktionelle Gruppe substituiert sein kann.

Ein niederaliphatischer Rest $R^a$ ist beispielsweise Niederalkyl, insbesondere mit 1-6, bevorzugt 1-4, C-Atomen, z.B. Methyl, Aethyl, Propyl oder Butyl, oder Niederalkenyl, insbesondere mit 2-5 C-Atomen, z.B. Vinyl, Propenyl oder Butenyl.

Ein cycloaliphatischer Rest $R^a$ ist Cycloalkyl mit 3-8, insbesondere 3-6 C-Atomen, z.B. Cyclopropyl, Cyclobutyl, Cyclopentyl oder Cyclohexyl.

Ein cycloaliphatisch-aliphatischer Rest $R^a$ ist einer der genannten aliphatischen Reste, der durch einen der genannten cycloaliphatischen Reste substituiert ist, z.B. Cyclopropylmethyl, oder -äthyl, oder Cyclohexylmethyl oder -äthyl.

Ein aromatischer Rest $R^a$ ist insbesondere Phenyl oder Naphthyl.

Ein araliphatischer Rest $R^a$ ist einer der genannten aliphatischen Reste, der durch einen bis drei der genannten aromatischen Reste substituiert ist, z.B. Benzyl, Phenäthyl, Diphenylmethyl oder Trityl.

Bevorzugte substituierte Kohlenwasserstoffreste $R^a$ sind beispielsweise Halogenniederalkyl, z.B. 2-Fluor-, 2-Chlor-, 2-Brom- und 2-Jodäthyl, Oxoniederalkyl, z.B. Acetonyl, gegebenenfalls geschütztes, z.B. acyliertes Hydroxyniederalkyl, z.B. 2-Hydroxyäthyl und Acetoxyäthyl, gegebenenfalls verestertes Carboxyniederalkyl, z.B. 2-Carboxymethyl und 2-Aethoxycarbonylmethyl, Amino- und Mono- oder Diniederalkylaminoniederalkyl, z.B. 2-Aminoäthyl, 2-Methylaminoäthyl,

2-Dimethylaminoäthyl und Acylaminoniederalkyl, z.B. 2-Acetamidoäthyl, Niederalkylphenyl, z.B. Toluyl und Xylyl, Niederalkoxyphenyl, z.B. Methoxyphenyl, Halogenphenyl, z.B. Fluor- oder Chlorphenyl, Hydroxyphenyl, Nitrophenyl, Carboxyphenyl und Aminophenyl, wobei die Substituenten in o-, m- oder p-Stellung des Phenylkernes stehen können, und Phenacetyl.

Bevorzugte Acylgruppen R sind beispielsweise Niederalkanoyl, wie Formyl, Acetyl oder Propionyl, Halogen-niederalkanoyl, wie 2-Halogenacetyl, insbesondere 2-Chlor-, 2-Brom-, 2-Jod-, 2,2,2-Trifluor- oder 2,2,2-Trichloracetyl, gegebenenfalls z.B. durch Halogen, Niederalkoxy oder Nitro substituiertes Benzoyl, z.B. Benzoyl, 4-Chlorbenzoyl, 4-Methoxybenzoyl oder 4-Nitrobenzoyl, oder in 1-Stellung des Niederalkylrestes verzweigtes oder in 1- oder 2-Stellung geeignet substituiertes Niederalkoxycarbonyl, insbesondere tert.-Niederalkoxycarbonyl, z.B. tert.-Butyloxycarbonyl, Arylmethoxycarbonyl mit einem oder zwei Arylresten, die gegebenenfalls, z.B. durch Niederalkyl, insbesondere tert.-Niederalkyl, wie tert.-Butyl, Niederalkoxy, wie Methoxy, Hydroxy, Halogen, z.B. Chlor und/oder Nitro, mono- oder polysubstituiertes Phenyl darstellen, wie gegebenenfalls substituiertes Benzyloxycarbonyl, z.B. 4-Nitrobenzyloxycarbonyl, oder gegebenenfalls substituiertes Diphenylmethoxycarbonyl, z.B. Benzhydryloxycarbonyl oder Di-(4-methoxyphenyl)-methoxycarbonyl, Aroylmethoxycarbonyl, worin die Aroylgruppe vorzugsweise gegebenenfalls, z.B. durch Halogen, wie Brom, substituiertes Benzoyl darstellt, z.B. Phenacyloxycarbonyl, 2-Halogenniederalkoxycarbonyl, z.B. 2,2,2-Trichloräthoxycarbonyl, 2-Chloräthoxycarbonyl, 2-Bromäthoxycarbonyl oder 2-Jodäthoxycarbonyl, Carbamoyl, N-Niederalkylcarbamoyl, z.B. N-Methylcarbamoyl, N,N-Diniederalkylcarbamoyl, z.B. N,N-Dimethylcarbamoyl, Thiocarbamoyl, N-Niederalkylthiocarbamoyl, z.B. N-Methylthiocarbamoyl, N,N-Diniederalkylthiocarbamoyl, z.B. N,N-Dimethylthiocarbamoyl, Niederalkylsulfonyl, z.B. Methylsulfonyl oder Aethylsulfonyl, Arylsulfonyl, z.B. Phenylsulfonyl oder Toluylsulfonyl, Sulfamoyl, N-Niederalkylsulfamoyl, z.B. N-Methylsulfamoyl, oder N,N-Diniederalkylsulfamoyl, z.B. N,N-Dimethylsulfamoyl.

$R_o$ als Niederalkyl ist z.B. Methyl, Aethyl, n-Propyl, n-Butyl, tert.-Butyl, sowie n-Pentyl, n-Hexyl oder n-Heptyl.

Wenn $R_1$ eine Teilformel $(R)(R_o)N-$ darstellt, können die zwei Gruppen der Teilformel $(R)(R_o)N-$ im Rahmen ihrer Bedeutungen gleich oder verschieden sein. Dabei nimmt am Phenylring bevorzugt eine Gruppe die 3-Stellung (meta), die andere Gruppe die 5-Stellung (meta) und die Hydroxygruppe die 4-Stellung (para) ein.

$R_2$ in der Bedeutung "geschütztes Carboxyl" ist leicht spaltbares geschütztes Carboxyl oder unter physiologischen Bedingungen spaltbares Carboxyl und ist durch die weiter unten beschriebenen Gruppen verestert.

$R_3$ als Halogen mit Ordnungszahl bis 35 steht für Fluor oder Brom, in erster Linie für Chlor.

$R_3$ als Niederalkoxy ist z.B. Aethoxy, n-Propyloxy, Isopropyloxy, n-Butyloxy, tert.-Butyloxy, sowie n-Pentyloxy, n-Hexyloxy, n-Heptyloxy, oder insbesondere Methoxy.

Die in der Verbindungen der Formel I vorhandenen funktionellen Gruppen, insbesondere die 4-Carboxyl-, die D-2-Amino- und die Hydroxygruppe am Phenylring, sind gegebenenfalls durch Schutzgruppen geschützt, die in der Penicillin-, Cephalosporin- und Peptidchemie verwendet werden.

Solche Schutzgruppen sind leicht, das heisst, ohne dass unerwünschte Nebenreaktionen stattfinden, beispielsweise solvolytisch, reduktiv, photolytisch oder auch unter physiologischen Bedingungen, abspaltbar.

Solche Schutzgruppen und ihre Abspaltung sind beispielsweise in "Protective Groups in Organic Chemistry", Plenum Press, London, New York, 1973, ferner in "The Peptides", Vol. I, Schröder and Lubke,

Academic Press, London, New York, 1965, sowie in "Methoden der organischen Chemie", Houben-Weyl, 4. Auflage, Bd. 15./1, Georg Thieme Verlag Stuttgart 1974, beschrieben.

So sind Carboxylgruppen, z.B. die Carboxylgruppe $R_2$, üblicherweise in veresterter Form geschützt, wobei solche Estergruppierungen unter schonenden Bedingungen leicht spaltbar sind. In dieser Art geschützte Carboxylgruppen enthalten als veresternde Gruppen in erster Linie in 1-Stellung verzweigte oder in 1- oder 2-Stellung geeignet substituierte Niederalkylgruppen. Bevorzugte, in veresterter Form vorliegende Carboxylgruppen sind u.a. tert.-Niederalkoxycarbonyl, z.B. tert.-Butyloxycarbonyl, Arylmethoxycarbonyl mit einem oder zwei Arylresten, wobei diese gegebenenfalls, z.B. durch Niederalkyl, wie tert.-Niederalkyl, z.B. tert.-Butyl, Niederalkoxy, wie Methoxy, Hydroxy, Halogen, z.B. Chlor, und/oder Nitro, mono- oder polysubstituierte Phenylreste darstellen, wie gegebenenfalls,z.B. wie oben erwähnt, substituiertes Benzyloxycarbonyl, z.B. 4-Nitro-benzyloxycarbonyl oder 4-Methoxy-benzyloxycarbonyl, oder gegebenenfalls, z.B. wie oben erwähnt, substituiertes Diphenylmethoxycarbonyl, z.B. Diphenylmethoxycarbonyl oder Di-(4-methoxyphenyl)-methoxycarbonyl, 1-Niederalkoxyniederalkoxycarbonyl, wie Methoxymethoxycarbonyl, 1-Methoxyäthoxycarbonyl oder 1-Aethoxymethoxycarbonyl, 1-Niederalkylthioniederalkoxycarbonyl, wie 1-Methylthiomethoxycarbonyl oder 1-Aethylthioäthoxycarbonyl, Aroylmethoxycarbonyl, worin die Aroylgruppe gegebenenfalls, z.B. durch Halogen, wie Brom, substituiertes Benzoyl darstellt, z.B. Phenacyloxycarbonyl, 2-Halogenniederalkoxycarbonyl, z.B. 2,2,2-Trichloräthoxycarbonyl, 2-Chloräthoxycarbonyl, 2-Bromäthoxycarbonyl oder 2-Jodäthoxycarbonyl, oder 2-(trisubstituiertes Silyl)-äthoxycarbonyl, worin die Substituenten unabhängig voneinander je einen gegebenenfalls substituierten, z.B. durch Niederalkyl, Niederalkoxy, Aryl, Halogen und/oder Nitro, substituierten, aliphatischen, araliphatischen, cycloaliphatischen oder aromatischen Kohlenwasserstoffrest mit z.B. bis zu 15 Kohlenstoffatomen, wie entsprechendes, gegebenenfalls substituiertes Niederalkyl, Phenylniederalkyl, Cycloalkyl oder Phenyl bedeuten, z.B. 2-Triniederalkylsilyläthoxycarbonyl, wie 2-Trimethylsilyläthoxycarbonyl oder 2-(Di-n-butylmethylsilyl)-äthoxycarbonyl, oder 2-Triarylsilyläthoxycarbonyl, wie 2-Triphenylsilyläthoxycarbonyl.

Weitere, in veresterter Form vorliegende geschützte Carboxylgruppen sind entsprechende Silyloxycarbonyl-, insbesondere organische Silyloxycarbonylgruppen, ferner entsprechende Stannyloxycarbonylgruppen. In diesen enthält das Silicium- bzw. Zinnatom vorzugsweise Niederalkyl, insbesondere Methyl, ferner Niederalkoxy, z.B. Methoxy, und/oder Halogen, z.B. Chlor, als Substituenten. Geeignete Silyl- bzw. Stannylschutzgruppen sind in erster Linie Triniederalkylsilyl, insbesondere Trimethylsilyl, ferner Dimethyl-tert.butylsilyl, Niederalkoxyniederalkylhalogensilyl, z.B. Methoxymethylchlorsilyl, oder Diniederalkylhalogensilyl, z.B. Dimethylchlorsilyl, oder entsprechend substituierte Stannylverbindungen, z.B. Tri-n-butylstannyl.

Bevorzugte geschützte Carboxylgruppen sind tert.-Niederalkoxycarbonyl, wie tert.-Butyloxycarbonyl, und in erster Linie gegebenenfalls, z.B. wie oben erwähnt, substituiertes Benzyloxycarbonyl, wie 4-Nitrobenzyloxycarbonyl, oder Diphenylmethoxycarbonyl.

Eine unter physiologischen Bedingungen spaltbare, veresterte Carboxylgruppe ist in erster Linie eine Acyloxymethyloxycarbonylgruppe, worin Acyl z.B. den Rest einer organischen Carbonsäure, in erster Linie einer gegebenenfalls substituierten Niederalkancarbonsäure bedeutet, oder worin Acyloxymethyl den Rest eines Lactons bildet, oder auch 1-Niederalkyloxycarbonyloxy-niederalkyloxycarbonyl, worin Niederalkyl Methyl, Propyl, Butyl oder insbesondere Aethyl ist. Solche Gruppen sind Niederalkanoyloxymethoxycarbonyl, z.B. Acetyloxymethyloxycarbonyl oder Pivaloyloxymethoxycarbonyl, Aminoniederalkanoyloxymethoxycarbonyl, insbesondere α-Amino-niederalkanoyloxymethoxycarbonyl, z.B. Glycyloxymethoxycarbonyl, L-Valyloxymethoxycarbonyl,

L-Leucyloxymethoxycarbonyl, Phthalidyloxycarbonyl, z.B. 2-Phthalidyloxycarbonyl, 4-Crotonolactonyl oder gamma-Butyrolacton-4-yl, Indanyloxycarbonyl, z.B. 5-Indanyloxycarbonyl oder 1-Aethyloxycarbonyloxyäthyloxycarbonyl.

Eine geschützte D-2-Aminogruppe kann z.B. in Form einer leicht spaltbaren Acylamino-, Arylmethylamino-, verätherten Mercaptoamino-, 2-Acylniederalk-1-en-ylamino-, Silyl- oder Stannylaminogruppe oder als Azidogruppe vorliegen.

In einer entsprechenden Acylaminogruppe ist Acyl beispielsweise der Acylrest einer organischen Carbonsäure mit z.B. bis zu 18 Kohlenstoffatomen, insbesondere einer gegebenenfalls, z.B. durch Halogen oder Aryl, substituierten Alkancarbonsäure, oder einer gegebenenfalls, z.B. durch Halogen, Niederalkoxy oder Nitro, substituierten Benzoesäure, oder eines Kohlensäurehalbesters. Solche Acylgruppen sind beispielsweise Niederalkanoyl, wie Formyl, Acetyl oder Propionyl, Halogenniederalkanoyl, wie 2-Halogenacetyl, insbesondere 2-Chlor-, 2-Brom-, 2-Jod-, 2,2,2-Trifluor- oder 2,2,2-Trichloracetyl, gegebenenfalls, z.B. durch Halogen, Niederalkoxy oder Nitro substituiertes Benzoyl, z.B. Benzoyl, 4-Chlorbenzoyl, 4-Methoxybenzoyl oder 4-Nitrobenzoyl, oder in 1-Stellung des Niederalkylrestes verzweigtes oder in 1- oder 2-Stellung geeignet substituiertes Niederalkoxycarbonyl, insbesondere tert.-Niederalkoxycarbonyl, z.B. tert.-Butyloxycarbonyl, Arylmethoxycarbonyl mit einem oder zwei Arylresten, die vorzugsweise gegebenenfalls, z.B. durch Niederalkyl, insbesondere tert.-Niederalkyl, wie tert.-Butyl, Niederalkoxy, wie Methoxy, Hydroxy, Halogen, z.B. Chlor und/oder Nitro, mono- oder polysubstituiertes Phenyl darstellen, wie gegebenenfalls substituiertes Benzyloxycarbonyl, z.B. 4-Nitrobenzyloxycarbonyl, oder gegebenenfalls substituiertes Diphenylmethoxycarbonyl, z.B. Benzhydryloxycarbonyl oder Di-(4-methoxyphenyl)-methoxycarbonyl, Aroylmethoxycarbonyl, worin die Aroylgruppe vorzugsweise gegebenenfalls, z.B. durch Halogen, wie Brom, substituiertes Benzoyl darstellt, z.B. Phenacyloxycarbonyl, 2-Halogenniederalkoxycarbonyl,

z.B. 2,2,2-Trichloräthoxycarbonyl, 2-Chloräthoxycarbonyl, 2-Bromäthoxycarbonyl oder 2-Jodäthoxycarbonyl, oder 2-(trisubstituiertes Silyl)-äthoxycarbonyl, worin die Substituenten unahängig voneinander je einen gegebenenfalls substituierten, z.B. durch Niederalkyl, Niederalkoxy, Aryl, Halogen oder Nitro, substituierten aliphatischen, araliphatischen, cycloaliphatischen oder aromatischen Kohlenwasserstoffrest mit z.B. bis zu 15 C-Atomen, wie entsprechendes gegebenenfalls substituiertes Niederalkyl, Phenylniederalkyl, Cycloalkyl oder Phenyl, bedeuten, z.B. 2-Triniederalkylsilyläthoxycarbonyl, wie 2-Trimethylsilyläthoxycarbonyl oder 2-(Di-n-butylmethylsilyl)-äthoxycarbonyl, oder 2-Triarylsilyläthoxycarbonyl, wie 2-Triphenylsilyläthoxycarbonyl.

Weitere, als Aminoschutzgruppen in Frage kommende Acylreste sind auch entsprechende Reste organischer Phosphor-, Phosphon- oder Phosphinsäuren, wie Diniederalkylphosphoryl, z.B. Dimethylphosphoryl, Diäthylphosphoryl, Di-n-propylphosphoryl oder Diisopropylphosphoryl, Dicycloalkylphosphoryl, z.B. Dicyclohexylphosphoryl, gegebenenfalls substituiertes Diphenylphosphoryl, z.B. Diphenylphosphoryl, gegebenenfalls, z.B. durch Nitro substituiertes Diphenylniederalkylphosphoryl, z.B. Dibenzylphosphoryl oder Di-4-nitrobenzylphosphoryl, gegebenenfalls substituiertes Phenyloxyphenylphosphonyl, z.B. Phenyloxyphenylphosphonyl, Diniederalkylphophinyl, z.B. Diäthylphosphinyl, oder gegebenenfalls substituiertes Diphenylphosphinyl, z.B. Diphenylphosphinyl.

In einer Arylmethylaminogruppe, die eine Mono-, Di- oder insbesondere Triarylmethylaminogruppe darstellt, sind die Arylreste insbesondere gegebenenfalls substituierte Phenylreste. Solche Gruppen sind beispielsweise Benzyl-, Diphenylmethyl- und insbesondere Tritylamino.

Eine verätherte Mercaptogruppe in einer mit einem solchen Rest geschützten Aminogruppe ist in erster Linie Arylthio oder Arylniederalkylthio, worin Aryl insbesondere gegebenenfalls, z.B. durch Niederalkyl, wie Methyl oder tert.-Butyl, Niederalkoxy, wie Methoxy, Halogen, wie Chlor und/oder Nitro, substituiertes Phenyl ist. Eine entsprechende Aminoschutzgruppe ist z.B. 4-Nitrophenylthio.

In einem als Aminoschutzgruppe verwendbaren 1-Acylnieder-alk-1-en-2-yl-rest ist Acyl z.B. der entsprechende Rest einer Niederalkancarbonsäure, einer gegebenenfalls, z.B. durch Niederalkyl, wie Methyl oder tert.-Butyl, Niederalkoxy, wie Methoxy, Halogen, wie Chlor, und/oder Nitro substituierten Benzoesäure, oder insbesondere eines Kohlensäurehalbesters, wie eines Kohlensäureniederalkylhalbesters. Entprechende Schutzgruppen sind in erster Linie 1-Niederalkanoyl-prop-1-en-2-yl, z.B. 1-Acetylprop-1-en-2-yl, oder 1-Niederalkoxy-carbonyl-prop-1-en-2-yl, z.B. 1-Aethoxycarbonylprop-1-en-2-yl.

Eine Silyl- oder Stannylaminogruppe ist eine organische Silyl- bzw. Stannylaminogruppe, worin das Silicium- bzw. Zinnatom vorzugsweise Niederalkyl, insbesondere Methyl, ferner Niederalkoxy, z.B. Methoxy, und/oder Halogen, z.B. Chlor, als Substituenten enthält. Entsprechende Silyl- oder Stannylgruppen sind in erster Linie Triniederalkylsilyl, insbesondere Trimethylsilyl, ferner Dimethyl-tert.-butylsilyl, Niederalkoxyniederalkylhalogensilyl, z.B. Methoxymethylchlorsilyl oder Diniederalkylhalogensilyl, z.B. Dimethylchlorsilyl, oder entsprechend substituiertes Stannyl, z.B. Tri-n-butylstannyl.

Eine Aminogruppe kann auch in protonierter Form geschützt werden; als Anionen kommen in erster Linie solche vor starken anorganischen Säuren, wie von Halogenwasserstoffsäuren, z.B. das Chlor- oder Bromanion, oder von organischen Sulfonsäuren, wie p-Toluolsulfonsäure, in Frage.

Bevorzugte Aminoschutzgruppen sind Acylreste von Kohlensäurehalbestern, insbesondere tert.-Butyloxycarbonyl, gegebenenfalls z.B. wie angegeben, substituiertes Benzyloxycarbonyl, z.B. 4-Nitrobenzyloxycarbonyl, oder Diphenylmethoxycarbonyl, oder 2-Halogenniederalkoxycarbonyl, wie 2,2,2-Trichloräthoxycarbonyl, ferner Trityl oder Formyl.

Hydroxyschutzgruppen sind z.B. Acylreste, wie gegebenenfalls z.B. durch Halogen, substituiertes Niederalkanoyl, wie 2,2-Dichloracetyl, oder insbesondere die im Zusammenhang mit einer geschützten Aminogruppe genannten Acylreste von Kohlensäurehalbestern, insbesondere 2,2,2-Trichloräthoxycarbonyl, oder organische Silyl- oder Stannylreste, ferner leicht abspaltbare veräthernde Gruppen, wie tert.-Niederalkyl, z.B. tert.-Butyl, 2-Halogenniederalkyl z.B. 2,2,2-Trichlor-, 2-Chlor-, 2-Brom- oder 2-Jodäthyl, 2-oxa- oder 2-thiaaliphatische oder -cycloaliphatische Kohlenwasserstoffreste, in erster Linie 1-Niederalkoxyniederalkyl oder 1-Niederalkylthioniederalkyl, z.B. Methoxymethyl, 1-Methoxyäthyl, 1-Aethoxyäthyl, 1-Methylthiomethyl, 1-Methylthioäthyl oder 1-Aethylthioäthyl, oder 2-Oxa- oder 2-Thiacycloalkyl mit 5-7 Ringatomen, z.B. 2-Tetrahydrofuryl oder 2-Tetrahydropyranyl oder entsprechende Thiaanaloge, sowie gegebenenfalls substituiertes 1-Phenylniederalkyl, wie gegebenenfalls substituiertes Benzyl oder Diphenylmethyl, wobei als Substituenten der Phenylreste z.B. Halogen, wie Chlor, Niederalkoxy, wie Methoxy und/oder Nitro in Frage kommen.

Salze von erfindungsgemässen Verbindungen der Formel I sind in erster Linie pharmazeutisch verwendbare, nicht toxische Salze, wie diejenigen von Verbindungen der Formel I mit sauren Gruppen, z.B. mit einer freien Carboxyl- oder Sulfogruppe. Solche Salze sind in erster Linie Metall- oder Ammoniumsalze, wie Alkalimetall- und Erdalkalimetall-, z.B. Natrium-, Kalium-, Magnesium- oder Calciumsalze, sowie Ammoniumsalze, welche mit Ammonaik oder geeigneten organischen Aminen gebildet werden, wobei in erster Linie aliphatische, cycloaliphatische, cylcoaliphatischaliphatische oder araliphatische primäre, sekundäre oder tertiäre Mono-, Di- oder Polyamine, sowie heterocyclische Basen für die Salzbildung in Frage kommen, beispielsweise Niederalkylamine, z.B. Träthylamin, Hydroxyniederalkylamine, z.B. 2-Hydroxyäthylamin, Bis-(2-hydroxyäthyl)-amin oder Tris-(2-hydroxyäthyl)-amin, basische aliphatische Ester von Carbonsäuren, z.B. 4-Aminobenzoesäure-2-diäthylaminoäthylester, Niederalkenamine, z.B. 1-Aethyl-piperidin, Cycloalkylamine, z.B. Dicyclohexylamin, oder Benzylamine, z.B. N,N'-Dibenzyläthylendiamin, ferner Basen vom Pyridintyp, z.B. Pyridin, Collidin oder Chinolin. Verbindungen der Formel I mit einer basischen Gruppe können Säureadditionssalze, z.B. mit anorganischen Säuren,wie Salzsäure, Schwefelsäure oder Phosphorsäure, oder mit geeigneten organischen Carbon- oder Sulfonsäuren, z.B. Trifluoressigsäure, sowie mit Aminosäuren, wie Arginin und Lysin, bilden. Bei Anwesenheit von mehreren sauren oder basischen Gruppen können Mono- oder Polysalze gebildet werden. Verbindungen der Formel I mit einer freien sauren Gruppe, z.B. der 4-Carboxylgruppe und mit einer freien basischen Gruppe, z.B. der D-2-Aminogruppe, können auch in Form von inneren Salzen, d.h. in zwitterionischer Form vorliegen, oder es kann ein Teil des Moleküls als inneres Salz, und ein anderer Teil als normales Salz vorliegen.

Zur Isolierung oder Reinigung von Verbindungen der Formel I können auch pharmazeutisch ungeeignete Salze Verwendung finden. Zur therapeutischen Anwendung gelangen nur pharmazeutisch verwendbare, nicht toxische Salze, die deshalb bevorzugt werden.

Die neuen Verbindungen der Formel I, worin die funktionellen Gruppen entweder in freier Form vorliegen oder die 4-Carboxylgruppe gegebenenfalls in physiologisch spaltbarer veresterter Form vorliegt, und ihre pharmazeutisch verwendbaren, nicht toxischen Salze weisen wertvolle pharmakologische Eigenschaften auf.

So sind Verbindungen der Formel I in freier Form oder in Form ihrer Salze *in vitro* gegen grampositive und gramnegative Kokken, z.B. *Staphylococcus aureus*, *Streptococcus pyogenes* oder *Neisseria spp.*, in Konzentrationen von ca. 0,1 bis ca. 32 µg/ml, gegen gramnegative Bakterien, z.B. Enterobakterien, beispielsweise *Escherichia coli*, *Klebsiella pneumoniae* oder *Proteus spp.*, oder gegen *Haemophilus influenzae* in Konzentrationen von 0,5 bis ca. 64 µg/ml wirksam.

*In vivo*, bei peroraler oder subkutaner Verabreichung an der Maus, sind Verbindungen (I) gegen systemische Infektionen, welche durch grampositive Kokken, z.B. *Staphylococcus aureus*, verursacht werden, in einem Dosisbereich von ca. 1,5 bis ca. 30 mg/kg und gegen systemische Infektionen, welche durch gramnegative Bakterien, z.B. *Escherichia coli*, *Proteus mirabilis* oder *Klebsiella spp.*, verursacht werden, in einem Dosisbereich von ca. 1,4 bis ca. 100 mg/kg bei geringer Toxizität wirksam.

## Versuchbericht

### I. Getestete Verbindungen

Für die folgenden Verbindungen ist die antibiotische Wirksamkeit getestet worden:

1. 7β-[D-2-Amino-2-(3-methylsulfonylamino-4-hydroxyphenyl)-acetylamino]-3-methoxy-ceph-3-em-4-carbonsäure (Beispiel 1a))
2. 7β-[D-2-Amino-2-(3-methylsulfonylamino-4-hydroxyphenyl)-acetylamino]-ceph-3-em-4-carbonsäure (Beispiel 2a))
3. 7β-[D-2-Amino-2-(3-methylsulfonylamino-4-hydroxyphenyl)-acetylamino]-3-chlor-ceph-3-em-4-carbonsäure (Beispiel 3a))
4. 7β-[D-2-Amino-2-(3-äthylsulfonylamino-4-hydroxyphenyl)-acetylamino]-ceph-3-em-4-carbonsäure (Beispiel 9a))
5. 7β-[D-2-Amino-2-(3-äthylsulfonylamino-4-hydroxyphenyl)-acetylamino]-3-chlor-ceph-3-em-4-carbonsäure (Beispiel 10a))

6. 7β-[D-2-Amino-2-(3-äthylsulfonylamino-4-hydroxyphenyl)-acetylamino]-3-methoxy-ceph-3-em-4-carbonsäure (Beispiel 11a))

II. Methodik

A. Die antibiotische Aktivität der Testverbindungen in vitro wurde durch die Agarverdünnungsmethode nach Ericsson, H.M. und Sherris, S.C., 1971, Acta Path. Microb. Scand. Section B, Suppl. No. 217, Bd. 1-90, in DST Agar ermittelt. Die gefundenen, das Wachstum der Testorganismen noch hemmenden Minimalkonzentrationen (MIC = minimum inhibiting concentrations) werden in Mikrogramm pro Milliliter (µg/ml) für die geprüften Verbindungen in der Tabelle 1 angegeben.

B. Die chemotherapeutsche Wirksamkeit in vivo gegen systemische Infektionen in weiblichen SPF, $MF_2$ Mäusen wurde nach der Methode von Zak, O., et al., 1979, Drugs Exptl. Clin. Res. 5, 45.59, ermittelt. Die gefundenen $ED_{50}$-Werte in Milligramm Substanz pro Kilogramm Maus (mg/kg) gegen eine Anzahl von Mikroorganismen werden für die oral (p.o.) oder subcutan (s.c.) applizierten Testverbindungen in der Tabelle 2 angegeben.

III. Resultate

Tabelle 1 (antibiotische Aktivität in vitro)

| Mikroorganismen | MIC [µg/ml] | | | | | |
|---|---|---|---|---|---|---|
| | Getestete Verbindungen | | | | | |
| | 1 | 2 | 3 | 4 | 5 | 6 |
| Streptococcus pyogenes Aronson | 0,1 | 4 | 0,5 | 1 | 0,5 | 0,5 |
| Neisseria meningitidis | 1 | 8 | 0,5 | 4 | 0,1 | 0,1 |
| Escherichia coli DC2 | 0,5 | 4 | 0,5 | 4 | 2 | 2 |
| Klebsiella pneumoniae 372 | 2 | 4 | 1 | 4 | 1 | 16 |

Tabelle 2 (chemotherapeutsche Wirksamkeit in vivo)

| Infizierende Mikroorganismen | ED$_{50}$ (mg/kg) Getestete Verbindungen | | | | | | | | | | | |
|---|---|---|---|---|---|---|---|---|---|---|---|---|
| | 1 | | 2 | | 3 | | 4 | | 5 | | 6 | |
| | s.c. | p.o. | s.c. | p.o. | s.c. | p.o. | s.c. | p.o. | s.c. | p.o. | s.c. | p.o. |
| Staphylococcus aureus 10 B | 3 | 1,5 | 3 | 1,5 | 10 | 4 | 3 | 1,5 | 10 | 7 | 15 | 6 |
| Escherichia coli 205 | 6 | 5 | n.g. | 2,2 | 3 | 2 | 3 | 4,5 | 3 | 4,5 | 8 | 6 |
| Klebsiella pneumoniae 327 | n.g. | 7 | n.g. | 3 | n.g. | 3 | n.g. | 1,4 | n.g. | 1,4 | n.g. | 10 |
| Proteus mirabilis 774 | n.g. | 22 | n.g. | 22 | n.g. | 4 | n.g. | 30 | n.g. | 30 | n.g. | 30 |

n.g.: nicht geprüft.

Die neuen Verbindungen der Formel I können deshalb entsprechend, z.B. in Form von antibiotisch wirksamen Präparaten, zur Behandlung von Infektionen Verwendung finden, welche durch grampositive oder gramnegative Bakterien verursacht werden.

Verbindungen der Formel I, worin die funktionellen Gruppen geschützt sind, werden als Zwischenprodukte zur Herstellung von Verbindungen der Formel I verwendet, worin die funktionellen Gruppen in freier Form vorliegen.

Die Erfindung betrifft bevorzugt solche Verbindungen der Formel I, worin funktionelle Gruppen in freier Form vorliegen oder worin die 4-Carboxylgruppe in physiologisch spaltbarer Form geschützt ist, und deren pharmazeutisch annehmbaren Salze, da hauptsächlich diese Verbindungen die angegebene Wirksamkeit besitzten und für den angegebenen Zweck verwendet werden können.

Hervorzuheben sind Verbindungen der Formel I, worin der Index n für 0 steht, R Wasserstoff oder eine Acylgruppe, z.B. Niederalkanoyl, Halogenniederalkanoyl, gegebenenfalls durch Halogen, Niederalkoxy oder Nitro substituiertes Benzoyl, tert.-Niederalkoxycarbonyl, Arylmethoxycarbonyl mit einem oder zwei Arylresten, die gegebenenfalls durch Niederalkyl, Niederalkoxy, Hydroxy, Halogen, und/oder Nitro mono- oder polysubstituiertes Phenyl darstellen, Aroylmethoxycarbonyl, worin die Aroylgruppe gegebenenfalls durch Halogen substituiertes Benzoyl darstellt, 2-Halogenniederalkoxycarbonyl, Carbamoyl, N-Niederalkylcarbamoyl, N,N-Diniederalkylcarbamoyl, Thiocarbamoyl, N-Niederalkylthiocarbamoyl, N,N-Diniederalkylthiocarbamoyl, Niederalkylsulfonyl, Arylsulfonyl, Sulfamoyl, N-Niederalkylsulfamoyl, oder N,N-Diniederalkylsulfamoyl, $R_o$ Wasserstoff oder Niederalkyl, $R_1$ Wasserstoff oder eine Gruppe der Teilformel $(R)(R_o)N-$, worin R und $R_o$ die vorstehend genannten Bedeutungen haben, $R_2$ Carboxyl oder Niederalkanoylmethoxycarbonyl und $R_3$ Wasserstoff, Fluor, Chlor, Brom, Methoxy, Aethoxy, Isopropyloxy oder tert.-Butyloxy bedeuten, sowie Hydrate

und Salze von Verbindungen (I), insbesondere pharmazeutisch verwendbare Salze von Verbindungen der Formel I.

Besonders hervorzuheben sind Verbindungen der Formel I, worin der Index n für 0 steht, R Wasserstoff, Niederalkanoyl mit bis zu 4 Kohlenstoffatomen, z.B. Formyl, Acetyl oder Propionyl, Halogenniederalkanoyl mit bis zu 4 Kohlenstoffatomen, z.B. 2-Chlor-, 2-Brom-, 2-Jod-, 2,2,2-Trifluor- oder 2,2,2-Trichloracetyl, gegebenenfalls durch Halogen, Niederalkoxy, z.B. Methoxy, oder Nitro substituiertes Benzoyl, z.B. 4-Chlorbenzoyl, 4-Methoxybenzoyl oder 4-Nitrobenzoyl, tert.-Niederalkoxycarbonyl, z.B. tert.-Butyloxycarbonyl, gegebenenfalls durch Nitro substituiertes Benzyloxycarbonyl, z.B. Benzyloxycarbonyl oder 4-Nitrobenzyloxycarbonyl, gegebenenfalls durch Niederalkoxy, z.B. Methoxy substituiertes Diphenylmethoxycarbonyl, z.B. Benzhydryloxycarbonyl oder Bis-(4-methoxyphenyl)-methoxycarbonyl, Phenacyloxycarbonyl, 2-Halogenniederalkoxycarbonyl mit bis zu 4 Kohlenstoffatomen, z.B. 2,2,2-Trichlor-, 2-Chlor-, 2-Brom- oder 2-Jodäthoxycarbonyl, Carbamoyl, N-Niederalkylcarbamoyl, z.B. N-Methylcarbamoyl, N,N-Diniederalkylcarbamoyl, z.B. N,N-Dimethylcarbamoyl, Thiocarbamoyl, N-Niederalkylthiocarbamoyl, z.B. N-Methylthiocarbamoyl, N,N-Diniederalkylthiocarbamyol, z.B. N,N-Dimethylthiocarbamoyl, Niederalkylsulfonyl, z.B. Methylsulfonyl oder Aethylsulfonyl, Arylsulfonyl, z.B. Phenylsulfonyl oder Toluylsulfonyl, Sulfamoyl, N-Niederalkylsulfamoyl, z.B. N-Methylsulfamoyl, oder N,N-Diniederalkylsulfamoyl, z.B. N,N-Dimethylsulfamoyl, $R_o$ Wasserstoff oder Niederalkyl, z.B. Methyl oder Aethyl, $R_1$ Wasserstoff oder eine Gruppe der Teilformel $(R)(R_o)N-$, worin R und $R_o$ die vorstehend genannten Bedeutungen haben, $R_2$ Carboxyl und $R_3$ Wasserstoff, Chlor oder Methoxy bedeuten, sowie Hydrate und Salze von Verbindungen (I), insbesondere pharmazeutisch verwendbare Salze von Verbindungen der Formel I.

Die Erfindung betrifft in erster Linie Verbindungen der Formel I, worin der Index n für 0 steht, R Wasserstoff, Niederalkanoyl mit bis zu 4 Kohlenstoffatomen, z.B. Acetyl, Niederalkylsulfonyl mit

bis zu 4 Kohlenstoffatomen, z.B. Methylsulfonyl oder Aethylsulfonyl, $R_o$ Wasserstoff, $R_1$ Wasserstoff oder eine Gruppe der Teilformel $(R)(R_o)N-$, worin R und $R_o$ die vorstehend genannten Bedeutungen haben, $R_2$ Carboxyl und $R_3$ Wasserstoff, Chlor oder Methoxy bedeuten, sowie Salze von solchen Verbindungen, insbesondere pharmazeutisch verwendbare Salze von Verbindungen der Formel I.

Die Erfindung betrifft vor allem Verbindungen der Formel I, worin der Index n für 0 steht, R Wasserstoff, Niederalkanoyl mit bis zu 4 Kohlenstoffatomen, z.B. Acetyl, Niederalkylsulfonyl mit bis zu 4 Kohlenstoffatomen, z.B. Methylsulfonyl oder Aethylsulfonyl, $R_o$ Wasserstoff, $R_1$ Wasserstoff oder eine Gruppe der Teilformel $(R)(R_o)N-$, worin R und $R_o$ die vorstehend genannten Bedeutungen haben, $R_2$ Carboxyl und $R_3$ Wasserstoff, Chlor oder Methoxy bedeuten, und worin die beiden Gruppen der Teilformel $(R)(R_o)N-$ die 3- und 5-Stellung (meta) und die Hydroxygruppe die 4-Stellung (para) am Phenylring einnehmen, sowie Hydrate und Salze von solchen Verbindungen, insbesondere pharmazeutisch verwendbare Salze von Verbindungen der Formel I.

Von allen die Erfindung betreffenden Verbindungen sind vor allem die in den Beispielen beschriebenen Verbindungen der Formel I, sowie Salze, insbesondere pharmazeutisch verwendbare Salze von solchen Verbindungen mit salzbildenden Gruppen hervorzuheben.

Die Verbindungen der Formel I der vorliegenden Erfindung werden nach an sich bekannten Verfahren erhalten.

Verbindungen der Formel I, sowie Hydrate und Salze von solchen Verbindungen, die eine salzbildende Gruppe aufweisen, werden beispielsweise hergestellt, indem man

a) in einer Verbindung der Formel

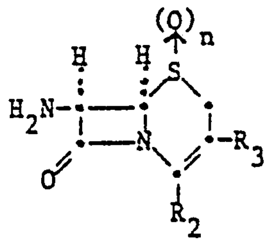

(II),

worin der Index n für 0 oder 1 steht und die 7β-Aminogruppe gegebenenfalls durch eine die Acylierung erlaubende Gruppe geschützt ist, und $R_2$ und $R_3$ die unter Formel I genannten Bedeutungen haben, die 7β-Aminogruppe durch Umsetzung mit einem den Acylrest einer Carbonsäure der Formel

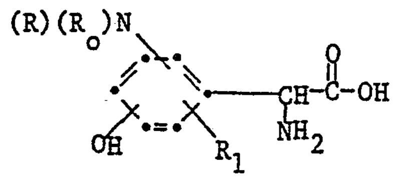

(III)

einführenden Acylierungsmittel, worin R, $R_o$ und $R_1$ die unter Formel I genannten Bedeutungen haben, und worin die Hydroxygruppe am Phenylring und/oder die Aminogruppe(n) gegebenenfalls in geschützter Form vorliegen und weitere vorhandene funktionelle Gruppen gegebenenfalls geschützt sind, acyliert, oder

b) eine 2-Cephemverbindung der Formel

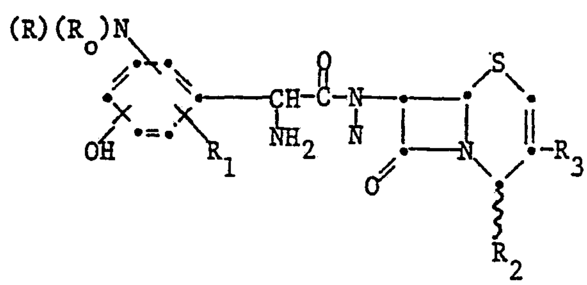

(IV),

worin R, $R_o$, $R_1$, $R_2$ und $R_3$ die unter Formel I genannten Bedeutungen haben und worin die Hydroxygruppe am Phenylring und/oder die Aminogruppe(n) gegebenenfalls in geschützter Form vorliegen und weitere vorhandene funktionelle Gruppen gegebenenfalls geschützt sind, isomerisiert, oder

c) in einer Verbindung der Formel

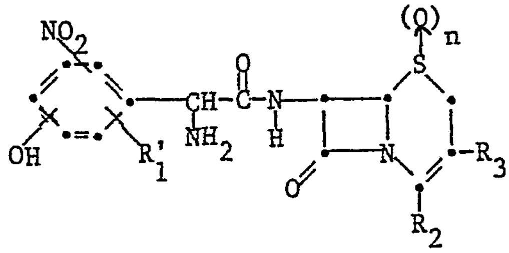

(V),

worin der Index n für 0 oder 1 steht und $R_1'$ Nitro oder den Rest $R_1$ darstellt, welcher die unter Formel I genannten Bedeutungen hat, $R_2$ und $R_3$ die unter Formel I genannten Bedeutungen haben, und worin die Hydroxygruppe am Phenylring und/oder die Aminogruppe(n) gegebenenfalls in geschützter Form vorliegen und weitere vorhandene funktionelle Gruppen gegebenenfalls geschützt sind, die Nitrogruppe(n) am Phenylring durch Umsetzen mit einem Nitrogruppen in Aminogruppen überführenden Mittel reduziert, und, wenn erwünscht, in einer erhältlichen Verbindung eine freie Aminogruppe der Teilformel (R)($R_o$)N-, worin R und $R_o$ Wasserstoff bedeuten, in eine substituierte Aminogruppe überführt und/oder, wenn erwünscht, eine erhältliche Verbindung, worin n 0 bedeutet, in eine Verbindung überführt, worin n 1 bedeutet, und/oder eine erhältliche Verbindung, worin n 1 bedeutet, in eine Verbindung überführt, worin n 0 bedeutet, und/oder in einer erhältlichen Verbindung in geschützter Form vorliegende funktionelle Gruppen in die freien funktionellen Gruppen überführt, und/oder ein erhältliches Salz in die freie Verbindung oder in ein anderes Salz überführt, und/oder eine erhältliche Verbindung mit einer salzbildenden Gruppe in ein Salz überführt, und/oder ein erhältliches Gemisch von isomeren Verbindungen in die einzelnen Isomeren auftrennt.

Verfahren a) (Acylierung):

Im Ausgangsmaterial der Formel II kann die 4-Carboxylgruppe vorzugsweise in veresterter Form, z.B. wie oben beschrieben, in erster Linie in Form eines Silyl-, wie Trimethylsilylesters, der meistens unmittelbar vor der Acylierungsreaktion durch Behandeln mit einem entsprechenden Silylierungsmittel, z.B. Trimethylchlorsilan oder N,O-Bis-(trimethylsilyl)-acetamid, hergestellt wird, geschützt sein. Das Carbonsäure-Ausgangsmaterial der Formel II kann jedoch auch in Salzform, z.B. in Ammoniumsalzform, wie in Form eines Triäthylammoniumsalzes, oder in geschützter Form eingesetzt werden, die durch Umsetzen des Carbonsäureausgangsmaterials II mit einer geeigneten organischen Phosphorhalogenidverbindung, wie mit einem Niederalkyl- oder Niederalkoxyphosphordihalogenid, wie Methylphosphordichlorid, Aethylphosphordibromid oder Methoxyphosphordichlorid, erhältlich ist. Ausgangsstoffe der Formel II, worin die 7β-Aminogruppe durch eine die Acylierungsreaktion erlaubende Gruppe geschützt ist, sind z.B. mit einem Silylrest, wie einem der oben genannten Silylreste geschützt.

Weitere Gruppen, durch die die 7β-Aminogruppe in reaktionsfähiger Form geschützt sein kann, sind die Carbonylgruppe -(C=O)- sowie gegebenenfalls 1-substituierte 1-Halogen- oder 1-Alkoxymethylengruppen. In solchen Methylengruppen ist Halogen Brom oder insbesondere Chlor und Alkoxy ist insbesondere gegebenenfalls substituiertes Niederalkoxy, wie Aethoxy, Propoxy, Butoxy oder bevorzugt Methoxy. Der gegebenenfalls vorhandene andere Substituent in 1-Stellung einer solchen Methylengruppe ist irgendein sterisch wenig gehinderter organischer Rest, beispielsweise gegebenenfalls substituiertes Niederalkyl, wie Methyl, 4-Amino-4-carboxybutyl, worin die Amino- und die Carboxygruppe geschützt sein können, Benzyl, Phenyloxymethyl, Thienylmethyl oder auch Furylmethyl, wie 2-Thienyl- oder 2-Furylmethyl.

In einem Acylrest einer Säure der Formel III ist die D-2-Aminogruppe gegebenenfalls durch übliche Aminoschutzgruppen, aber auch in ionischer Form geschützt, d.h. das Ausgangsmaterial der Formel III mit der D-2-Aminogruppe kann auch in Form eines Säureadditionssalzes, vorzugsweise mit einer starken anorganischen Säure, wie einer Halogenwasserstoffsäure, z.B. Salzsäure, verwendet werden, oder die D-2-Aminogruppe ist gegebenenfalls in maskierter Form, z.B. als Azidogruppe, geschützt.

In einem Ausgangsmaterial der Formel III ist in substituierten Aminogruppen der Teilformel $(R)(R_o)N-$, worin R eine Acylgruppe und $R_o$ Wasserstoff bedeutet, der Wasserstoff $R_o$ gegebenenfalls durch eine weitere Acylgruppe ersetzt.

Die Hydroxygruppe am Phenylring ist durch eine der oben genannten üblichen Hydroxyschutzgruppen gegebenenfalls geschützt.

Vorhandene freie Aminogruppen Am am Phenylring sind gegebenenfalls durch die oben beschriebenen üblichen Aminoschutzgruppen geschützt.

Den Acylrest einer Carbonsäure der Formel III einführende Acylierungsmittel sind beispielsweise die Carbonsäure selbst oder ihre reaktionsfähige, funktionelle Derivate.

Falls eine Carbonsäure der Formel III, worin vorhandene funktionelle Gruppen ausser der an der Reaktion teilnehmenden 4-Carboxylgruppe geschützt sein können, zur Acylierung eingesetzt wird, wird die Reaktion üblicherweise in Gegenwart von geeigneten Kondensationsmitteln, wie Carbodiimiden, beispielsweise N,N'-Diäthyl-, N,N'-Dipropyl-, N,N'-Dicyclohexyl- oder N-Aethyl-N'-3-dimethylaminopropylcarbodiimid, geeigneten Carbonylverbindungen, beispielsweise Carbonyldiimidazol, oder 1,2-Oxazoliumverbindungen, wie 2-Aethyl-5-phenyl-1,2-oxazolium-3'-sulfonat oder 2-tert.-Butyl-5-methyl-1,2-oxazoliumperchlorat, oder einer geeigneten Acylaminoverbindung, z.B. 2-Aethoxy-1-äthoxycarbonyl-1,2-dihydrochinolin, durchgeführt.

Die Kondensationsreaktion wird vorzugsweise in einem wasserfreien Reaktionsmedium, vorzugsweise in Gegenwart eines Lösungs- oder Verdünnungsmittels, z.B. Methylenchlorid, Dimethylformamid, Acetonitril oder Tetrahydrofuran, wenn erwünscht oder notwendig, unter Kühlen oder Erwärmen, z.B. in einem Temperaturbereich von etwa -40°C bis etwa +100°C, bevorzugt von etwa -20°C bis etwa +50°C und/oder in einer Inertgas-, z.B. Stickstoffatmosphäre durchgeführt.

Ein reaktionsfähiges, funktionelles Derivate einer Carbonsäure der Formel III, worin vorhandene funktionelle Gruppen ausser der an der Reaktion beteiligten Carboxylgruppe geschützt sein können, ist in erster Linie ein Anhydrid einer solchen Säure, inklusive und vorzugsweise ein gemischtes Anhydrid. Gemischte Anhydride sind z.B. diejenigen mit anorganischen Säuren, wie Halogenwasserstoffsäuren, d.h. die entsprechenden Säurehalogenide, z.B. -chloride oder -bromide, ferner mit Stickstoffwasserstoffsäure, d.h. die entsprechenden Säureazide, mit einer phosphorhaltigen Säure, z.B. Phosphorsäure, Diäthylphosphorsäure oder Phosphoriger Säure, oder mit einer schwefelhaltigen Säure, z.B. Schwefelsäure, oder mit Cyanwasserstoffsäure. Weitere gemischte Anhydride sind z.B. diejenigen mit organischen Carbonsäuren, wie mit gegebenenfalls, z.B. durch Halogen, wie Fluor oder Chlor, substituierten Niederalkancarbonsäuren, z.B. Pivalinsäure oder Trichloressigsäure, oder mit Halbestern, insbesondere Niederalkylhalbestern der Kohlensäure, wie dem Aethyl- oder Isobutylhalbester der Kohlensäure, oder mit organischen, insbesondere aliphatischen oder aromatischen, Sulfonsäuren, z.B. p-Toluolsulfonsäure.

Weitere zur Reaktion mit der Aminogruppe geeignete Derivate einer Säure der Formel III, worin vorhandene funktionelle Gruppen ausser der an der Reaktion teilnehmenden Carboxylgruppe geschützt sein können, sind aktivierte Ester, wie Ester mit vinylogen Alkoholen, d.h. mit Enolen, wie vinylogen Niederalkenolen oder Iminomethylesterhalogeniden, wie Dimethyliminomethylesterchlorid, hergestellt aus der Carbonsäure der Formel III und z.B. Dimethyl-(1-chlor-äthyliden)-iminium-chlorid der Formel $(CH_3)_2N^{\oplus}=C(Cl)CH_3\ Cl^{\ominus}$, das man z.B. aus N,N-Dimethylacetamid und Phosgen erhalten kann, Arylester, z.B. durch Halogen, wie Chlor, und/oder Nitro, substituierte Phenylester, z.B. 4-Nitrophenyl-, 2,3-Dinitrophenyl- oder Pentachlorphenylester, N-heteroaromatische Ester, wie N-Benztriazol-, z.B. 1-Benztriazolester, oder N-Diacyliminoester, wie N-Succinylimino- oder N-Phthalyliminoester.

Die Acylierung mit einem reaktionsfähigen, funktionellen Derivat einer Säure der Formel III, wie einem entsprechenden Anhydrid, insbesondere einem Säurehalogenid, wird bevorzugt in Anwesenheit eines säurebindenden Mittels, beispielsweise einer organischen Base, wie eines organischen Amins, z.B. eines tertiären Amins, wie Triniederalkylamin, z.B. Trimethylamin, Triäthylamin oder Aethyl-diisopropylamin, oder N,N-Diniederalkylanilin, z.B. N,N-Dimethylanilin, oder eines cyclischen tertiären Amins, wie eines N-niederalkylierten Morpholins, wie N-Methylmorpholin, oder einer Base vom Pyridin-Typ, z.B. Pyridin, einer anorganischen Base, beispielsweise eines Alkalimetall- oder Erdalkalimetallhydroxids, -carbonats oder -hydrogencarbonats, z.B. Natrium-, Kalium- oder Calciumhydroixd, -carbonat oder -hydrogencarbonat, oder eines Oxirans, beispielsweise eines 1,2-Niederalkylenoxids, wie Aethylenoxid oder Propylenoxid, durchgeführt.

Die obigen Acylierungen werden vorzugsweise in einem inerten, vorzugsweise wasserfreien,Lösungsmittel oder Lösungsmittelgemisch vorgenommen, beispielsweise in einem Carbonsäureamid, wie einem Formamid, z.B. Dimethylformamid, einem halogenierten Kohlenwasserstoff, z.B. Methylenchlorid, Tetrachlorkohlenstoff oder Chlorbenzol, einem Keton, z.B. Aceton, einem Ester, z.B. Essisäureäthylester, oder einem Nitril, z.B. Acetonitril, oder einer Mischung davon, wenn notwendig oder erwünscht, bei erniedrigter oder erhöhter Temperatur, z.B. in einem Temperaturbereich von etwa -40°C bis etwa +100°C, bevorzugt von etwa -10°C bis etwa +50°C, und/oder in einer Inertgas-, z.B. Stickstoffatmosphäre.

Die Acylierung kann auch durch Verwendung eines geeigneten Derivates der Säure der Formel III in Gegenwart einer geeigneten Acylase erfolgen. Solche Acylasen sind bekannt und können von einer Anzahl von Mikroorganismen gebildet werden, z.B. von Pseudomonas melanogenum ATCC 17 808 oder von zahlreichen anderen acylierenden Stämmen wie Xanthomonas, Acetobacter, z.B. Acetobacter aurantium, Achromobacter, z.B. Achromobacter Aeris, Benechea hyperoptica, Micrococcus urea, Mycobacterium smegmatis, Nocardia globerula oder Bacillus

z.B. Bacillus megaterium 400. In einer solchen enzymatischen Acylierung werden insbesondere Amide, Ester oder Thioester, wie Niederalkyl-, z.B. Methyl- oder Aethylester, der Carbonsäure der Formel III als entsprechende Derivate eingesetzt. Ueblicherweise wird eine solche Acylierung in einem den entsprechenden Mikroorganismus enthaltenden Nährmedium, in einem Filtrat der Kulturbrühe oder, gegebenenfalls nach Isolierung der Acylase, inklusive nach Adsorption an einen Träger, in einem wässrigen, gegebenenfalls einen Puffer enthaltenden Medium, z.B. in einem Temperaturbereich von etwa +20°C bis etwa +40°C, bevorzugt bei etwa +37°C, durchgeführt.

Ein in der Acylierungsreaktion eingesetztes reaktionsfähiges, funktionelles Derivat einer Säure der Formel III kann, wenn erwünscht, in situ gebildet werden. So kann man z.B. ein gemischtes Anyhdrid herstellen, indem man eine Säure der Formel III, gegebenenfalls mit entsprechend geschützten funktionellen Gruppen, oder ein geeignetes Salz davon, z.B. ein Ammoniumsalz, welches z.B. mit einer organischen Base, wie Pyridin oder 4-Methylmorpholin gebildet wird oder ein Metall-, z.B. Alkalimetallsalz, mit einem geeigneten Säurederivat, wie einem entsprechenden Säurehalogenid einer gegebenenfalls substituierten Niederalkancarbonsäure, z.B. Trichloracetylchlorid, mit einem Ester eines Kohlensäurehalbhalogenids, z.B. Chlorameisensäureäthylester oder -isobutylester, oder mit einem Halogenid einer Diniederalkylphosphorsäure, z.B. Diäthylphosphorbromidat, das man durch Umsetzen von Triäthylphosphit mit Brom bilden kann, umsetzt und das so erhältliche gemischte Anhydrid ohne Isolierung in der Acylierungsreaktion verwendet.

Wird bei der Acylierung eine racemische Mischung eines Ausgangsmaterials der Formel III eingesetzt, so erhält man eine racemische Mischung einer Verbindung der Formel I. Diese lässt sich anschliessend mit den üblichen in der Literatur für die Auftrennung von racemischen Mischungen beschriebenen Methoden in die gewünschten D- und L-konfigurierten Antipoden auftrennen.

Bevorzugt werden jedoch bei der Acylierung die D-konfigurierten Antipoden eines Ausgangsmaterials der Formel III eingesetzt.

Verfahren b) (Isomerisierung): In einem 2-Cephem-Ausgangsmaterial der Formel IV weist die gegebenenfalls geschützte Carboxylgruppe $R_2$ in 4-Stellung vorzugsweise die α-Konfiguration auf.

2-Cephem-Verbindungen der Formel IV können isomerisiert werden, indem man sie mit einem schwach-basischen Mittel behandelt und die entsprechende 3-Cephem-Verbindung isoliert. Geeignete Isomerisierungsmittel sind z.B. organische, stickstoffhaltige Basen, insbesondere tertiäre heterocyclische Basen aromatischen Charakters, in erster Linie Basen des Pyridin-Typs, wie Pyridin selber, sowie Picoline, Collidine oder Lutidine, ferner Chinolin, tertiäre aromatische Basen, z.B. solche des Anilin-Typs, wie N,N-Diniederalkylaniline, z.B. N,N-Dimethylanilin oder N,N-Diäthylanilin, oder tertiäre aliphatische, azacycloaliphatische oder araliphatische Basen, wie N,N,N-Triniederalkylamine, z.B. N,N,N-Trimethylamin oder N,N-Diisopropyl-N-äthylamin, N-Niederalkylazacycloalkane, z.B. N-Methylpiperidin, oder N-Phenylniederalkyl-N,N-diniederalkylamine, z.B. N-Benzyl-N,N-dimethylamin, sowie Gemische von solchen basischen Mitteln, wie das Gemisch einer Base vom Pyridintyp und eines N,N,N-Tri-niederalkylamins, z.B. Pyridin und Triäthylamin. Ferner können auch anorganische oder organische Salze von Basen, insbesondere von mittelstarken bis starken Basen mit schwachen Säuren, wie Alkalimetall-, oder Ammoniumsalze von Niederalkancarbonsäuren, z.B. Natriumacetat, Triäthylammoniumacetat oder N-Methyl-piperidinacetat, sowie andere analoge Basen oder Gemische von solchen basischen Mitteln verwendet werden.

Bei der Isomerisierung von 2-Cephem-Verbindungen der Formel IV mit basischen Mitteln arbeitet man vorzugsweise in wasserfreiem Medium, in An- oder Abwesenheit eines Lösungsmittels, wie eines gegebenenfalls halogenierten, z.B. chlorierten, aliphatischen, cycloaliphatischen oder aromatischen Kohlenwasserstoffs, oder eines

Lösungsmittelgemisches, wobei als Reaktonsmittel verwendete unter den Reaktonsbedingungen flüssige Basen gleichzeitig auch als Lösungsmittel dienen können, gegebenenfalls unter Kühlen oder unter Erwärmen, vorzugsweise in einem Temperaturbereich von etwa -30°C bis etwa +100°C, in einer Inertgas-, z.B. Stickstoffatmosphäre, und/oder in einem geschlossenen Gefäss.

So erhältliche 3-Cephem-Verbindungen der Formel I lassen sich in an sich bekannter Weise, z.B. durch Adsorption und/oder Kristallisation, von gegebenenfalls noch vorhandenen 2-Cephem-Ausgangsstoffen abtrennen.

Die Isomerisierung von 2-Cephem-verbindungen der Formel IV zur entsprechenden 3-Cephem-Verbindung wird vorzugsweise durchgeführt, indem man diese in 1-Stellung oxydiert, wenn erwünscht, gegebenenfalls Isomerengemische der gebildeten 1-Oxide trennt, und die so erhältlichen 1-Oxide der entsprechenden 3-Cephemverbindungen reduziert.

Als geeignete Oxydationsmittel für die Oxydation in 1-Stellung von 2-Cephem-verbindungen der Formel IV kommen anorganische Persäuren, die ein Reduktionspotential von wenigstens +1,5 Volt aufweisen und aus nicht-metallischen Elementen bestehen, organische Persäuren oder Gemische aus Wasserstoffperoxid und Säuren, insbesondere organische Carbonsäuren, mit einer Dissoziationskonstante von wenigstens $10^{-5}$ in Frage. Geeignete anorganische Persäuren sind Perjod- und Perschwefelsäure. Organische Persäuren sind entsprechende Percarbon- und Persulfonsäuren, die als solche zugesetzt oder durch Verwendung von wenigstens einem Aequivalent Wasserstoffperoxid und einer Carbonsäure in situ gebildet werden können. Dabei ist es zweckmässig, einen grossen Ueberschuss der Carbonsäure anzuwenden, wenn z.B. Essigsäure als Lösungsmittel verwendet wird. Geeignete Persäuren sind z.B. Perameisensäure, Peressigsäure, Trifluorperessigsäure, Permaleinsäure, Perbenzoesäure, 3-Chlorperbenzoesäure, Monoperphthalsäure oder p-Toluolpersulfonsäure.

Die Oxydation kann ebenfalls unter Verwendung von Wasserstoffperoxid mit katalytischen Mengen einer Säure mit einer Dissoziationskonstante von wenigstens $10^{-5}$ durchgeführt werden, wobei man niedrige Konzentrationen, z.B. 1-2% und weniger, aber auch grössere Mengen der Säure einsetzen kann. Dabei hängt die Wirksamkeit des Gemisches in erster Linie von der Stärke der Säure ab. Geeignete Gemische sind z.B. solche von Wasserstoffperoxid mit Essigsäure, Perchlorsäure oder Trifluoressigsäure.

Die obige Oxydation kann in Gegenwart von geeigneten Katalysatoren durchgeführt werden. So kann z.B. die Oxydation mit Percarbonsäuren durch die Anwesenheit einer Säure mit einer Dissoziationskonstante von wenigstens $10^{-5}$ katalysiert werden, wobei ihre Wirksamkeit von ihrer Stärke abhängt. Als Katalysatoren geeignete Säuren sind z.B. Essigsäure, Perchlorsäure und Trifluoressigsäure. Ueblicherweise verwendet man mindestens äquimolare Mengen des Oxydationsmittels, vorzugsweise einen geringen Ueberschuss von etwa 10% bis etwa 20%, wobei man auch grössere Ueberschüsse, d.h. bis zur 10-fachen Menge des Oxidationsmittels oder darüber, verwenden kann. Die Oxydation wird unter milden Bedingungen , z.B. bei Temperaturen von etwa -50°C bis etwa +100°C, vorzugsweise von etwa -10°C bis etwa +40°C durchgeführt.

Die Reduktion der 1-Oxide von 3-Cephem-Verbindungen kann in an sich bekannter Weise durch Behandeln mit einem Reduktionsmittel, wenn notwendig, in Anwesenheit eines aktivierenden Mittels, durchgeführt werden. Als Reduktionsmittel kommen beispielsweise in Betracht: Katalytisch aktivierter Wasserstoff, wobei Edelmetallkatalysatoren verwendet werden, welche z.B. Palladium, Platin oder Rhodium enthalten, und die man gegebenenfalls zusammen mit einem geeigneten Trägermaterial, wie Kohle oder Bariumsulfat, einsetzt; reduzierende Zinn-, Eisen-, Kupfer- oder Mangankationen, welche in Form von entsprechenden Verbindungen oder Komplexen anorganischer oder organischer Art, z.B. als Zinn-II-chlorid, -fluorid, -acetat oder -formiat, Eisen-II-chlorid, -sulfat, -oxalat oder -succinat, Kupfer-I-chlorid, -benzoat oder -oxyd, oder Mangan-II-chlorid, -sulfat, -acetat oder -oxyd, oder als Komplexe, z.B. mit Aethylendiamintetraessigsäure oder Nitrolotriessigsäure, verwendet werden; reduzierende Dithionit-, Jod- oder Eisen-II-cyanid-anionen, welche in Form von entsprechenden anorganischen oder organischen Salzen, wie Alkalimetall-, z.B. Natrium- oder Kaliumdithionit, Natrium- oder Kaliumjodid oder -eisen-II-cyanid, oder in Form der entsprechenden Säuren, wie Jodwasserstoffsäure, verwendet werden; reduzierende trivalente anorganische oder organische Phosphorverbindungen, wie Phosphine, ferner Ester, Amide und Halogenide der phosphinigen, phosphonigen oder phosphorigen Säure, sowie diesen Phosphorsauerstoffverbindungen entsprechende Phosphor-Schwefelverbindungen, worin organische Reste in erster Linie aliphatische, aromatische oder araliphatische Reste, z.B. gegebenenfalls substituiertes Niederalkyl, Phenyl oder Phenylniederalkyl darstellen, wie z.B. Triphenylphosphin, Tri-n-butylphosphin, Diphenylphosphinigsäuremethylester, Diphenylchlorphosphin, Phenyldichlorphosphin, Benzolphosphonigsäuredimethylester, Butanphosphonigsäuremethylester, Phosphorigsäuretriphenylester, Phosphorigsäuretrimethylester, Phosphortrichlorid, Phosphortribromid, etc.; reduzierende Halogensilanverbindungen, die mindestens ein an das Siliciumatom gebundenes Wasserstoffatom aufweisen, und die ausser Halogen, wie Chlor, Brom oder Jod, auch organische Reste, wie aliphatische oder aromatische Gruppen, z.B. gegebenenfalls substituiertes Niederalkyl oder Phenyl, aufweisen können, wie Chlorsilan, Bromsilan, Di- oder Trichlorsilan, Di- oder Tribromsilan, Diphenylchlorsilan, oder Dimethylchlorsilan, sowie auch Halogensilanverbindungen, worin alle Wasserstoffe durch organische Reste ersetzt sind, wie Triniederalkylhalogensilan, z.B. Trimethylchlorsilan oder Trimethyljodsilan, oder cyclische, Schwefelenthaltende Silane, wie 1,3-Dithia-2,4-disilacyclobutane oder 1,3,5-Trithia-2,4,6-trisilacyclohexane, deren Siliciumatome durch Kohlenwasserstoffreste, wie insbesondere Niederalkyl substituiert sind, z.B. 2,2,4,4-Tetramethyl-1,3-dithia-2,4-disilacyclobutan oder 2,2,4,4,6,6-Hexamethyl-1,3,5-trithia-2,4,6-trisilacyclohexan, etc.; reduzierende quaternäre Chlormethyleniminiumsalze, insbesondere

-chloride oder -bromide, worin die Iminiumgruppe durch einen bivalenten oder zwei monovalente organische Reste, wie gegebenenfalls substituiertes Niederalkylen bzw. Niederalkyl substituiert ist, wie N-Chlormethylen-N,N-diäthyliminiumchlorid oder N-Chlormethylenpyrrolidiniumchlorid; oder komplexe Metallhydride, wie Natriumborhydride, in Gegenwart von geeigneten Aktivierungsmitteln, wie Cobalt-II-chlorid, sowie Borandichlorid.

Als aktivierende Mittel, die zusammen mit denjenigen der obgenannten Reduktionsmittel verwendet werden, welche selber nicht Lewissäure-Eigenschaften aufweisen, d.h. in erster Linie zusammen mit den Dithionit-, Jod- oder Eisen-II-cyanid- und den nicht-halogenhaltigen trivalenten Phosphor-Reduktionsmitteln oder bei der katalytischen Reduktion eingesetzt werden, sind insbesondere organische Carbon- und Sulfonsäurehalogenide, ferner Schwefel-, Phosphor- oder Siliciumhalogenide mit gleicher oder grösserer Hydrolysenkonstante zweiter Ordnung als Benzoylchlorid, z.B. Phosgen, Oxalylchlorid, Essigsäurechlorid oder -bromid, Chloressigsäurechlorid; Pivalinsäurechlorid, 4-Methoxybenzoesäurechlorid, 4-Cyanbenzoesäurechlorid, p-Toluolsulfonsäurechlorid, Methansulfonsäurechlorid, Thionylchlorid, Phosphoroxychlorid, Phosphortrichlorid, Phosphortribromid, Phenyldichlorphosphin, Benzolphosphonigsäuredichlorid, Dimethylchlorsilan oder Trichlorsilan, ferner geeignete Säureanhydride, wie Trifluoressigsäureanhydrid, oder cyclische Sultone, wie Aethansulton, Propansulton, 1,3-Butansulton oder 1,3-Hexansulton zu erwähnen.

Die Reduktion wird vorzugsweise in Gegenwart von Lösungsmitteln oder Gemischen davon durchgeführt, deren Auswahl in erster Linie durch die Löslichkeit der Ausgangsstoffe und die Wahl des Reduktionsmittels bestimmt wird, so z.B. Niederalkancarbonsäuren oder Ester davon, wie Essigsäure und Essigsäureäthylester, bei der katalytischen Reduktion, und z.B. gegebenenfalls durch Halogen oder Nitro substituierte, aliphatische, cycloaliphatische, aromatische oder araliphatische Kohlenwasserstoffe, z.B. Benzol, Methylenchlorid, Chloroform oder Nitromethan, geeignete Säurederivate, wie Niederalkancarbonsäureester oder -nitrile, z.B. Essigsäureäthylester oder Acetonitril, oder Amide von anorganischen oder organischen Säuren, z.B. Dimethylformamid oder Hexamethylphosphoramid, Aether, z.B. Diäthyläther, Tetrahydrofuran oder Dioxan, Ketone, z.B. Aceton, oder Sulfone, insbesondere aliphatische Sulfone, z.B. Dimethylsulfon oder Tetramethylensulfon, etc., zusammen mit den chemischen Reduktionsmitteln, wobei diese Lösungsmittel vorzugsweise kein Wasser enthalten. Dabei arbeitet man gewöhnlicherweise bei Temperaturen von etwa -20°C bis etwa 100°C, wobei bei Verwendung von sehr reaktionsfähigen Aktivierungsmitteln die Reaktion auch bei tieferen Temperaturen durchgeführt werden kann.

Wird bei der Isomerisierung eine racemische Mischung eines Ausgangsmaterials der Formel IV eingesetzt, so erhält man eine racemische Mischung einer Verbindung der Formel I. Diese lässt sich anschliessend mit den üblichen in der Literatur für die Auftrennung von racemischen Mischungen beschriebenen Methoden in die gewünschte D- und die L-konfigurierten Antipoden auftrennen. Bevorzugt werden jedoch bei der Isomerisierung die D-konfigurierten Antipoden eines Ausgangsmaterials der Formel IV eingesetzt.

Verfahren c) (Reduktion der Nitrogruppe(n)):

In einer Verbindung der Formel V, worin die D-2-Aminogruppe, die Hydroxygruppe am Phenylring und die 4-Carboxylgruppe gegebenenfalls in geschützter Form vorliegen, wird (werden) die aromatische(n) Nitrogruppe(n) am Phenylring durch Umsetzung mit einem geeigneten Nitrogruppen in Aminogruppen überführenden Mittel reduziert.

Geeignete Mittel, welche Nitrogruppen am Phenylring in Aminogruppen überführen, sind übliche Reduktionsmittel, welche gegebenenfalls in Gegenwart eines Katalysators und/oder geeigneten Trägermaterials eingesetzt werden.

Als übliche Reduktionsmittel kommen in erster Linie in Betracht: Katalytisch aktivierter Wasserstoff, wobei als Hydrierkatalysatoren beispielsweise Edelmetalle, z.B. Palladium, Platin oder Rhodium verwendet werden und welche man gegebenenfalls mit einem geeigneten Trägermaterial wie Kohle, Bariumsulfat oder -carbonat oder Calciumcarbonat einsetzt, reduzierende Zinn(II)- oder Eisen(II)-kationen, welche vorzugsweise in Form ihrer Chloride und letztere auch in Form ihrer Sulfate eingesetzt werden, reduzierende Dithionit- oder Sulfitanionen, welche als anorganische Salze, z.B. mit Alkalimetallkationen, in Form von Alkalimetallsalzen, z.B. Natriumdithionit oder Natriumsulfit, oder Alkalimetallhydrogensalzen, z.B. Natriumhydrogensulfit, verwendet werden, unedle Metalle, z.B. Eisen, Zinn, Zink oder Aluminium, welche gegebenenfalls in Gegenwart ihrer entsprechenden Metallsalze oder mit anderen Neutralsalzen, z.B. Calcium-, Magnesium-, Kalium- oder Natriumchlorid zur Anwendung gelangen, ferner Sulfide, z.B. Schwefelwasserstoff, Di- oder Polysulfide, z.B. Natriumdisulfid oder Natriumpolysulfide, Alkalimetall- oder Alkalimetallhydrogen-, z.B. Natriumsulfid oder Natriumhydrogensulfid, Ammoniumsulfid oder Ammoniumpolysulfid, Wasserstoff-abgebende Mittel, z.B. substituiertes oder unsubstituiertes Hydrazin, beispielsweise Phenylhydrazin, welches gegebenenfalls in Form seines Säureadditionssalzes eingesetzt wird, oder molekularer Wasserstoff, welcher durch elektrolytische Reduktion an der Kathode entladen wird.

Die Reduktion mit katalytisch aktiviertem Wasserstoff erfolgt bei Normaldruck oder mässig erhöhtem Druck bis zu etwa 5 atü. Die Reduktion mit Zink, Aluminium, Zinn oder Zinn(II)-Ionen erfolgt im allgemeinen in saurer, z.B. in salzsaurer Lösung. Verwendet man Eisen(II)-Kationen als Reduktionsmittel arbeitet man üblicherweise unter leicht basischen Bedingungen. Dabei setzt man ein beliebiges lösliches Eisen(II)-Salz, z.B. Eisen(II)-sulfat zur Reaktionsmischung hinzu, worauf das reduzierend wirkende Eisen(II)-hydroxyd ausfällt. Die Reduktion mit elementarem Eisen, welches z.B. in Form von Eisenpulver zugesetzt werden kann, erfolgt im allgemeinen unter leicht sauren, z.B. salzsauren oder neutralen Bedingungen. Im letzteren Fall setzt man

zwecks Aktivierung noch Salze, z.B. Eisen(II)-chlorid, Eisen(II)-sulfat, Eisen(III)-chlorid, Calciumchlorid, Magnesiumchlorid, Kaliumchlorid oder Natriumchlorid hinzu.

Ueblicherweise werden die Reduktionen mit Dithionitanionen in neutralem oder schwach alkalischem Medium, welches man beispielsweise durch Zugabe von tertiären aromatischen Basen, z.B. Pyridin, erzeugt, durchgeführt. Die Reduktionen mit Sulfitanionen finden in leicht saurem, z.B. essigsaurem, oder neutralem Medium statt.

Die Reduktion mit Sulfiden kann in leicht saurem, z.B. essigsaurem, neutralem oder schwach basischem wässrigem Medium, welches bei der Reduktion mit Schwefelwasserstoff in Gegenwart von Ammoniak oder von tertiären aromatischen Basen, z.B. Pyridin, erzeugt wird, durchgeführt werden. Natriumsulfid oder Natriumpolysulfid wird vor der Reduktion durch Eintragen der jeweils äquivalenten Menge an Schwefel in eine Natriumsulfidlösung frisch hergestellt. Natriumhydrogensulfid wird durch Einleiten von Schwefelwasserstoff in eine Natriumsulfidlösung hergestellt. Ammoniumsulfid oder Ammoniumpolysulfid wird vorteilhaft in situ erzeugt, indem man in eine ammoniakalische wässrige Reaktionslösung Schwefelwasserstoff einleitet und gegebenenfalls elementaren Schwefel zusetzt.

Die Reduktion mit wasserstoffabgebenden Mitteln, z.B. Hydrazinen, wird durch die oben genannten Hydrierkatalysatoren, z.B. Raney-Nickel, Palladium-Kohle oder Platin beschleunigt. Die elektrolytische Reduktion der Nitrogruppen zum Amin erfordert ein hohes Elektrodenpotential und wird an Kathoden aus Metallen mit grosser Ueberspannung, wie Blei, Zinn, Nickel, Kupfer oder Zink durchgeführt. Die Elektrolyse erfolgt meist in schwefelsaurer oder salzsaurer Lösung.

Die oben erwähnten Reduktionsmittel werden in äquimolarer Menge oder bevorzugt im Ueberschuss zugesetzt. Die Zugabe eines Ueberschusses an Reduktionsmittel soll die Bildung von unerwünschten Zwischenprodukten, z.B. Nitrosoverbindungen oder Hydroxylaminen, unterbinden.

Die Reduktion wird vorzugsweise in Gegenwart von Lösungsmitteln oder Gemischen davon durchgeführt, deren Auswahl in erster Linie durch die Löslichkeit der Ausgangsstoffe, die Wahl des Reduktionsmittels und der Fähigkeit das bei der Reduktion entstandene Reaktionswasser zu binden bestimmt wird, so z.B. Niederalkanole, z.B. Methanol oder Aethanol, Niederalkyläther von Glykolen, z.B. Aethylenglykol-monomethyläther oder -äthyläther, Niederalkancarbonsäuren oder Ester davon, wie Essigsäure und Essigsäureäthylester, bei der katalytischen Reduktion, und z.B. gegebenenfalls durch Halogen oder Nitro substituierte, aliphatische, cycloaliphatische, aromatische oder araliphatische Kohlenwasserstoffe, z.B. Benzol, Methylenchlorid, Chloroform oder Nitromethan, geeignete Säurederivate, wie Niederalkancarbonsäureester oder -nitrile, z.B. Essigsäureäthylester oder Acetonitril, oder Amide von anorganischen oder organischen Säuren, z.B. Dimethylformamid oder Hexamethylphosphorsäuretriamid, Aether, z.B. Diäthyläther, Tetrahydrofuran oder Dioxan, Ketone, z.B. Aceton, oder Sulfone, insbesondere aliphatische Sulfone, z.B. Dimethylsulfon oder Tetramethylensulfon, etc.

Um die Löslichkeit vor allem der salzartigen Reduktionsmittel im Reaktionsgemisch zu erhöhen, wird zum Reaktionsgemisch nach Bedarf Wasser hinzugesetzt. Man arbeitet gewöhnlicherweise bei Temperaturen von etwa -20°C bis etwa 100°C, wobei bei Verwendung von sehr reaktionsfähigen Aktivierungsmitteln die Reaktion auch bei tieferen Temperaturen durchgeführt werden kann.

Wird bei der Reduktion der Nitrogruppe(n) eine racemische Mischung eines Ausgangsmaterials der Formel V eingesetzt, so erhält man eine racemische Mischung einer Verbindung der Formel I. Diese lässt sich anschliessend mit den üblichen in der Literatur für die Auftrennung von racemischen Mischungen beschriebenen Methoden in die D- und L-konfigurierten Antipoden auftrennen. Bevorzugt werden bei der Reduktion die D-konfigurierten Antipoden eines Ausgangsmaterials der Formel V eingesetzt.

Nachoperationen:

In einer Verbindung der Formel I können die Substituenten $(R)(R_o)N-$, $R_1$ und $R_2$ im Rahmen ihrer Bedeutungen und in beliebiger Reihenfolge in andere Substituenten $(R)(R_o)N-$, $R_1$ und $R_2$ überführt werden. So kann eine freie Aminogruppe der Teilformel $(R)(R_o)N-$, worin R und $R_o$ Wasserstoff bedeuten, in eine Acylaminogruppe der Teilformel $(R)(R_o)N-$ umgewandelt werden, worin R eine Acylgruppe und $R_o$ Wasserstoff oder Niederalkyl bedeuten. Eine freie Carboxylgruppe kann verestert oder eine veresterte Carboxylgruppe $R_2$ kann in eine freie Carboxylgruppe übergeführt werden. Gegebenenfalls in einer Verbindung der Formel I vorhandene Schutzgruppen, z.B. eine D-2-Aminoschutzgruppe oder eine Hydroxyschutzgruppe, werden abgespalten. Diese nachträglichen Operationen werden in an sich bekannter Weise durchgeführt, beispielsweise wie folgt:

Acylierung der Aminogruppe(n) am Phenylring:

Wenn in einer erhältlichen Verbindung der Formel I R und $R_o$ Wasserstoff bedeuten, und/oder der Substituent $R_1$ eine Gruppe der Teilformel $(R)(R_o)N-$ darstellt, worin R und $R_o$ ebenfalls Wasserstoff bedeuten, kann (können) die freie(n) Aminogruppe(n) am Phenylring in an sich bekannter Weise durch eine Acylgruppe R oder einen Niederalkylrest $R_o$ substituiert werden.

Diese Substitution kann beispielsweise durch Acylierung mit einem den entsprechenden Acylrest R einführenden geeigneten Acylierungsmittel erfolgen.

Die Aminogruppe(n) am Phenylring liegt (liegen) gegebenenfalls in reaktionsfähiger, d.h. die Acylierung erlaubender, beispielsweise durch die oben beschriebenen üblichen Silylreste geschützter, Form vor. Die Hydroxygruppe am Phenylring ist durch eine der oben beschriebenen üblichen Hydroxyschutzgruppen und die D-2-Aminogruppe durch eine der oben beschriebenen üblichen Aminoschutzgruppen gegebenenfalls

geschützt.

Wird (werden) die Aminogruppe(n) durch einen Acylrest $R^a$-CO- substituiert, verwendet man als Acylierungsmittel beispielsweise die entsprechende Carbonsäure oder ein reaktionsfähiges Derivat davon, insbesondere ein Anhydrid, inkl. ein gemischtes oder inneres Anhydrid einer solchen Säure. Gemischte Anhydride von Carbonsäuren sind z.B. diejenigen mit Halogenwasserstoffsäuren, d.h. die entsprechenden Carbonsäurehalogenide, insbesondere -chloride, ferner mit Cyanwasserstoffsäure, oder diejenigen mit geeigneten Kohlensäurehalbderivaten, wie entsprechenden -halbestern, z.B. die mit einem Halogenameisensäureniederalkyl-, wie Chlorameisensäureäthylester oder -isobutylester, gebildeten gemischten Anhydride, oder mit gegebenenfalls substituierten, z.B. Halogen, wie Chlor, enthaltenden Niederalkancarbonsäuren, wie die mit Pivalinsäurechlorid oder Trichloressigsäurechlorid gebildeten gemischten Anhydride. Innere Anhydride sind z.B. diejenigen von organischen Carbonsäuren, d.h. Ketene, wie Keten oder Diketen, oder diejenigen von Carbamin- oder Thiocarbaminsäuren, d.h. Isocyanate oder Isothiocyanate. Weitere reaktionsfähige, als Acylierungsmittel verwendbare Derivate von organischen Carbonsäuren sind aktivierte Ester, wie geeignet substituierte Phenyl-, z.B. Pentachlorphenyl- oder 4-Nitrophenylester.

Wird (werden) die Aminogruppe(n) am Phenylring in einer Verbindung der Formel I durch einen Acylrest $R^a-SO_2-$ substituiert, verwendet man als Acylierungsmittel beispielsweise die entsprechende Sulfonsäure oder ein reaktionsfähiges Derivat davon, in erster Linie ein Anhydrid davon, inklusive, und vorzugsweise, ein gemischtes Anhydrid. Gemischte Anhydride von Sulfonsäuren sind z.B. diejenigen mit anorganischen Säuren, insbesondere mit Halogenwasserstoffsäuren, d.h. die entsprechenden Sulfonsäurehalogenide, z.B. das Sulfonsäurechlorid oder -bromid.

Weitere, zur Substitution der Aminogruppe(n) am Phenylring geeignete Sulfonsäurederivate sind aktivierte Ester der Niederalkylsulfonsäuren, wie Ester mit vinylogen Alkoholen (d.h. Enolen), wie vinylogen Niederalkenolen, oder Arylester, wie vorzugsweise, z.B. durch Nitro oder Halogen, wie Chlor, substituierte Phenylester, z.B. Pentachlorphenyl-, 4-Nitrophenyl- oder 2,4-Dinitrophenylester, heteroaromatische Ester, wie Benztriazolester, oder Diacyliminoester, wie Succinylimino- oder Phthalyliminoester.

Wird (werden) die Aminogruppe(n) am Phenylring durch einen Acylrest R mit den Bedeutungen $R^a$-O-CO-, $R^aR^a$N-CS- oder $R^aR^aN-SO_2$-substituiert, verwendet man als Acylierungsmittel ein reaktionsfähiges Derivat des entsprechenden Kohlensäurehalbesters, der entsprechenden Carbaminsäure, Thiocarbaminsäure oder Amidosulfonsäure. Solche reaktionsfähigen Derivate sind beispielsweise Anhydride, gemischte Anhydride, z.B. mit anorganischen Säuren wie Halogenwasserstoffsäuren, oder im Falle der Carbaminsäure oder Thiocarbaminsäure innere Anhydride, z.B. die Cyanate oder Thiocyanate.

Die Acylierungsreaktionen können, wenn notwendig, in Gegenwart von geeigneten Kondensationsmitteln, bei Verwendung von freien Carbonsäuren oder Sulfonsäuren beispielsweise in Gegenwart von Carbodiimiden, z.B. N,N'-Diäthyl-, N,N'-dipropyl-, N,N'-Diisopropyl-, N,N'-Dicyclohexyl- oder N-Aethyl-N'-3-dimethylaminopropyl-carbodiimid, geeigneten Carbonylverbindungen, z.B. Carbonyldiimidazol, oder Isoxazoliniumsalzen, z.B. N-Aethyl-5-phenyl-isoxazolinium-3'-sulfonat und N-tert.-Butyl-5-methyl-isoxazoliniumperchlorat, oder einer geeigneten Acylaminoverbindung, z.B. 2-Aethoxy-1-äthoxycarbonyl-1,2-dihydrochinolin. Solche Kondensationsreaktionen werden vorzugsweise in einem wasserfreien Reaktionsmedium, beispielsweise in Methylenchlorid, Dimethylformamid oder Acetonitril, durchgeführt.

Die Acylierungsreaktion mit einer dem Rest R entsprechenden Sulfonsäure oder einem reaktionsfähigen Derivat davon, kann auch in Anwesenheit eines säurebindenden Mittels, beispielsweise einer organischen Base, wie eines organischen Amins, z.B. eines tertiären Amins,

wie Triniederalkylamin, z.B. Triäthylamin, N,N-Diniederalkyl-anilin, z.B. N,N-Dimethyl-anilin, oder einer Base vom Pyridin-Typ, z.B. Pyridin, einer anorganischen Base, beispielsweise eines Alkalimetall- oder Erdalkalimetallhydroxids, -carbonats, oder -hydrogencarbonats, z.B. Natrium-, Kalium- oder Calciumhydroxid, -carbonat oder -hydrogencarbonat, oder eines Oxirans, beispielsweise eines niederen 1,2-Alkylenoxids, wie Aethylenoxid oder 1,2-Propylenoxid, durchgeführt werden.

Die Acylierungsreaktionen können in Abwesenheit oder in Gegenwart eines Lösungsmittels oder Lösungsmittelgemisches, unter Kühlen, bei Raumtemperatur oder unter Erwärmen, und, wenn notwendig, in einem geschlossenen Gefäss und/oder in einer Inertgas-, z.B. Stickstoffatmosphäre, durchgeführt werden. Geeignete Lösungsmittel sind die oben genannten oder beispielsweise gegebenenfalls substituierte, insbesondere gegebenenfalls chlorierte, aliphatische, cycloaliphatische oder aromatische Kohlenwasserstoffe, wie Benzol und Toluol, wobei man geeignete Veresterungsreagentien, wie Essigsäureanhydrid, auch als Verdünnungsmittel verwenden kann.

Alkylierung der Aminogruppe(n) am Phenylring:

Wenn in einer erhältlichen Verbindung der Formel I R und $R_o$ Wasserstoff bedeuten und/oder der Substituent $R_1$ eine Gruppe der Teilformel (R)($R_o$)N- darstellt, worin R und $R_o$ ebenfalls Wasserstoff bedeuten, kann (können) die freie(n) Aminogruppe(n) am Phenylring durch ein den Niederalkylrest $R_o$ einführendes geeignetes Alkylierungsmittel, z.B. ein Alkylhalogenid wie Methylbromid, zur (zu) $R_o$ (= Niederalkyl)-substituierten Aminogruppe(n) alkyliert werden. Solche $R_o$ substituierte(n) Aminogruppe(n) lässt (lassen) sich anschliessend in der oben geschilderten Weise mit einem den Acylrest R einführenden Acylierungsmittel substituieren.

Man gelangt so zu Verbindungen der Formel I, worin die Gruppe der Teilformel (R)($R_o$)N- durch einen Niederalkylrest und durch Acyl substituiert ist und worin $R_1$ Wasserstoff oder eine durch Niederalkyl und Acyl substituierte Gruppe der Teilformel (R)($R_o$)N- ist.

Solche Verbindungen lassen sich auch bevorzugt durch Substitution der Aminogruppe(n) am Phenylring durch einen den oben gegebenen Bedeutungen entsprechenden Acylrest R und anschliessende Metallisierung der so gebildeten sekundäre(n) Aminogruppe(n) am Phenylring mit einem geeigneten Metallierungsreagenz, gefolgt von einer dem Rest $R_o$ entsprechenden reaktionsfähigen Verbindung, herstellen.

Geeignete Metallierungsreagenzien sind beispielsweise Lithiumdiisopropylamid oder Butyllithium. Eine dem Rest $R_o$ entsprechende reaktionsfähige Verbindung ist beispielsweise eine Verbindung der Formel $R_o$-X, worin X eine nukleofuge Abgangsgruppe, beispielsweise ein Halogenatom, z.B. Chlor, Brom oder Jod, oder eine Sulfonyloxygruppe, z.B. Mesyloxy oder Tosyloxy, darstellt.

Umwandlung zum 1-Oxid, bzw. 1-Sulfid:

Eine Verbindung der Formel I, worin der Index n 0 bedeutet, kann mit den unter Verfahren b) beschriebenen Oxydationsmitteln in das entsprechende 1-Oxid, worin der Index n den Wert 1 hat, umgewandelt werden.

Ein 1-Oxid der Formel I, worin der Index n den Wert 1 hat, kann mit den unter Verfahren b) beschriebenen Reduktionsmitteln in das entsprechende 1-Sulfid, worin der Index n den Wert 0 hat, umgewandelt werden.

Veresterung einer freien Carboxylgruppe:

Die Umwandlung von freiem Carboxyl, in erster Linie einer entsprechenden Gruppe $R_2$ in einer Verbindung der Formel (I) in verestertes Carboxyl, insbesondere in eine veresterte Carboxylgruppe, die unter physiologischen Bedingungen spaltbar ist, erfolgt nach an sich bekannten Veresterungsmethoden, beispielsweise indem man eine Verbindung der Formel I worin andere, gegebenenfalls vorhandene funktionelle Gruppen gegebenenfalls in geschützter Form vorliegen, oder ein reaktionsfähiges funktionelles Carboxyderivat, inkl. ein Salz davon, mit

einem entsprechenden Alkohol oder einem reaktionsfähigen funktionellen Derivat davon, umsetzt.

Die Veresterung der freien Carboxylgruppe mit dem gewünschten Alkohol wird in Gegenwart eines geeigneten Kondensationsmittels durchgeführt. Uebliche Kondensationsmittel sind z.B. Carbodiimide, beispielsweise N,N'-Diäthyl-, N,N'-Dipropyl-, N,N'-Dicyclohexyl- oder N-Aethyl-N'-(3-dimethylaminopropyl)-carbodiimid, geeignete Carbonylverbindungen, beispielsweise Carbonyldiimidazol, oder 1,2-Oxazoliumverbindungen, z.B. 2-Aethyl-5-phenyl-1,2-oxazolium-3'-sulfonat und 2-tert.-Butyl-5-methylisoxazoliumperchlorat, oder eine geeignete Acylaminoverbindung, z.B. 2-Aethoxy-1-äthoxycarbonyl-1,2-dihydrochinolin. Die Kondensationsreaktion wird vorzugsweise in einem wasserfreien Reaktionsmedium, vorzugsweise in Gegenwart eines Lösungs- oder Verdünnungsmittel, z.B. Methylenchlorid, Dimethylformamid, Acetonitril oder Tetrahydrofuran und, wenn notwendig, unter Kühlen oder Erwärmen und/oder in einer Inertgasatmosphäre durchgeführt.

Geeignete reaktionsfähige funktionelle Derivate der zu veresternden Carboxylverbindungen der Formel I sind z.B. Anhydride, insbesondere gemischte Anhydride, und aktivierte Ester.

Gemischte Anhydride sind z.B. diejenigen mit anorganischen Säuren, wie Halogenwasserstoffsäuren, d.h. die entsprechenden Säurehalogenide, z.B. -chloride oder -bromide, ferner Stickstoffwasserstoffsäure, d.h. die entsprechenden Säureazide, sowie phosphorhaltigen Säuren, z.B. Phosphorsäure, Diäthylphosphorsäure oder phosphoriger Säure, oder schwefelhaltigen Säuren, z.B. Schwefelsäure, oder Cyanwasserstoffsäure. Gemischte Anhydride sind weiter z.B. diejenigen mit organischen Carbonsäuren, wie mit gegebenenfalls, z.B. durch Halogen, wie Fluor oder Chlor, substituierten Niederalkancarbonsäuren, z.B. Pivalinsäure oder Trichloressigsäure, oder mit Halbestern, insbesondere Niederalkylhalbestern der Kohlensäure, wie Aethyl- oder Isobutylhalbestern der Kohlensäure, oder mit organischen, insbesondere aliphatischen oder aromatischen, Sulfonsäure, z.B. p-Toluolsulfonsäure.

Zur Reaktion mit dem Alkohol geeignete aktivierte Ester sind z.B. Ester mit vinylogen Alkoholen (d.h. Enolen), wie vinylogen Niederalkenolen, oder Iminomethylesterhalogeniden, wie Dimethyliminomethylesterchlorid, hergestellt aus der freien Carbonsäure und Dimethylchlormethylideniminiumchlorid der Formel $[(CH_3)_2N{=}CHCl]^{\oplus}Cl^{\ominus}$, oder Arylester, wie Pentachlorphenyl-, 4-Nitrophenyl- oder 2,3-Dinitrophenylester, heteroaromatische Ester, wie Benztriazol-, z.B. 1-Benztriazolester, oder Diacyliminoester, wie Succinylimino- oder Phthalyliminoester.

Die Veresterung mit einem solchen Säurederivat, wie einem Anhydrid, insbesondere mit einem Säurehalogenid wird bevorzugt in Anwesenheit eines säurebindenden Mittels, beispielsweise einer organischen Base, wie eines organischen Amins, z.B. eines tertiären Amins, wie Triniederalkylamin, z.B. Trimethylamin, Triäthylamin oder Aethyldiisopropylamin, oder N,N-Diniederalkylanilin, z.B. N,N-Dimethylanilin, oder eines cyclischen tertiären Amins, wie eines N-niederalkylierten Morpholins, wie N-Methylmorpholin, oder einer Base vom Pyridin-Typ, z.B. Pyridin, einer anorganischen Base, beispielsweise eines Alkalimetall- oder Erdalkalimetallhydroxids, -carbonats oder -hydrogencarbonats, z.B. Natrium-, Kalium- oder Calciumhydroxid, -carbonat oder -hydrogencarbonat, oder eines Oxirans, beispielsweise eines niederen 1,2-Alkylenoxids, wie Aethylenoxid oder Propylenoxid, durchgeführt.

Ein reaktionsfähiges funktionelles Derivat des veresternden Alkohols ist in erster Linie ein entsprechender Ester, vorzugsweise mit einer starken anorganischen oder organischen Säure und stellt insbesondere ein entsprechendes Halogenid, z.B. Chlorid, Bromid oder Jodid, oder eine entsprechende Niederalkyl- oder Aryl-, wie Methyl- oder 4-Methylphenylsulfonyloxyverbindung dar.

Ein solcher reaktionsfähiger Ester eines Alkohols kann mit der freien Carboxylverbindung der Formel I oder einem Salz, wie einen Alkalimetall- oder Ammoniumsalz davon umgesetzt werden, wobei man bei Verwendung der freien Säure vorzugsweise in Gegenwart eines säurebindenden Mittels arbeitet.

Die obigen Veresterungsreaktionen werden in einem inerten, üblicherweise wasserfreien Lösungsmittel oder Lösungsmittelgemisch vorgenommen, beispielsweise in einem Carbonsäureamid, wie einem Formamid, z.B. Dimethylformamid, einem halogenierten Kohlenwasserstoff, z.B. Methylenchlorid, Tetrachlorkohlenstoff oder Chlorbenzol, einem Keton, z.B. Aceton, einem Ester, z.B. Essigsäureäthylester, oder einem Nitril, z.B. Acetonitril, oder Mischungen davon, wenn notwendig, unter Kühlen oder Erwärmen, z.B. in einem Temperaturbereich von etwa bei -40°C bis etwa +100°C, vorzugsweise bei etwa -10°C bis etwa +40°C, und/oder in einer Inertgas-, z.B. Stickstoffatmosphäre.

Ferner kann das Säurederivat, wenn erwünscht, in situ gebildet werden. So erhält man z.B. ein gemischtes Anhydrid durch Behandeln der Carbonsäureverbindung mit entsprechend geschützten funktionellen Gruppen oder eines geeigneten Salzes davon, wie eines Ammoniumsalzes, z.B. mit einem organischem Amin, wie Piperidin oder 4-Methylmorpholin, oder eines Metall-, z.B. Alkalimetallsalzes mit einem geeigneten Säurederivat, wie dem entsprechenden Säurehalogenid einer gegebenenfalls substituierten Niederalkancarbonsäure, z.B. Trichloracetylchlorid, mit einem Halbester eines Kohlensäure-halbhalogenids, z.B. Chloramiesensäueräthylester oder -isobutylester, oder mit einem Halogenid einer Diniederalkylphosphorsäure, z.B. Diäthylphosphorbromidat, und verwendet das so erhältliche gemischte Anhydrid ohne Isolierung.

Abspaltung von Schutzgruppen: In einer erhaltenen Verbindung der Formel I, worin eine oder mehrere funktionelle Gruppen geschützt sind, können diese, z.B. geschützte Carboxyl-, Amino-, Hydroxy- und/oder Sulfogruppen, in an sich bekannter Weise, mittels Solvolyse, insbesondere Hydrolyse, Alkoholyse oder Acidolyse, oder mittels

Reduktion, insbesondere Hydrogenolyse oder chemische Reduktion, gegebenenfalls stufenweise oder gleichzeitig freigesetzt werden.

So kann man tert.-Niederalkoxycarbonyl oder in 2-Stellung durch eine organische Silylgruppe oder in 1-Stellung durch Niederalkoxy oder Niederalkylthio substituiertes Niederalkoxycarbonyl oder gegebenenfalls substituiertes Diphenylmethoxycarbonyl z.B. durch Behandeln mit einer geeigneten Säure, wie Ameisensäure oder Trifluoressigsäure, gegebenenfalls unter Zugabe einer nucleophilen Verbindung, wie Phenol oder Anisol, in freies Carboxyl überführen. Gegebenenfalls substituiertes Benzyloxycarbonyl kann z.B. mittels Hydrogenolyse, d.h. durch Behandeln mit Wasserstoff in Gegenwart eines metallischen Hydrierkatalysators, wie eines Palladiumkatalysators, freigesetzt werden. Ferner kann man geeignet substituiertes Benzyloxycarbonyl, wie 4-Nitrobenzyloxycarbonyl, auch mittels chemischer Reduktion, z.B. durch Behandeln mit einem Alkalimetall, z.B. Natriumdithionit, oder mit einem reduzierenden Metall, z.B. Zink, oder Metallsalz, wie einem Chrom-II-salz, z.B. Chrom-II-chlorid, üblicherweise in Gegenwart eines Wasserstoff-abgebenden Mittels, das zusammen mit dem Metall nascierenden Wasserstoff zu erzeugen vermag, wie einer Säure, in erster Linie einer geeigneten Carbonsäure, wie einer gegebenenfalls, z.B. durch Hydroxy, substituierten Niederalkancarbonsäure, z.B. Essigsäure, Ameisensäure, Glycolsäure, Diphenylglycolsäure, Milchsäure, Mandelsäure, 4-Chlormandelsäure oder Weinsäure, oder eines Alkohols oder Thiols, wobei man vorzugsweise Wasser zugibt, in freies Carboxyl überführt. Durch Behandeln mit einem reduzierenden Metall oder Metallsalz, wie oben beschrieben, kann man auch 2-Halogen-niederalkoxycarbonyl, gegebenenfalls nach Umwandlung einer 2-Bromniederalkoxycarbonylgruppe in eine entsprechende 2-Jodniederalkoxycarbonylgruppe, oder Aroylmethoxycarbonyl in freies Carboxyl umwandeln, wobei Aroylmethoxycarbonyl ebenfalls durch Behandeln mit einem nucleophilen, vorzugsweise salzbildenden Reagens, wie Natriumthiophenolat oder Natriumjodid, gespalten werden kann. Substituiertes 2-Silyläthoxycarbonyl kann auch durch Behandeln mit einem, das Fluoridanion liefernden Salz

der Fluorwasserstoffsäure, wie einem Alkalimetallfluorid, z.B. Natrium- oder Kaliumfluorid, in Anwesenheit eines macrocyclischen Polyäthers ("Kronenäther"), oder mit einem Fluorid einer organischen quaternären Base, wie Tetraniederalkylammoniumfluorid oder Triniederalkylarylammoniumfluorid, z.B. Tetraäthylammoniumfluorid oder Tetrabutylammoniumfluorid, in Gegenwart eines aprotischen Lösungsmittels, wie Dimethylsulfoxid oder N,N-Dimethylacetamid, in freies Carboxyl übergeführt werden. Mit einer organischen Silyl- oder Stannylgruppe, wie Triniederalkylsilyl oder -stannyl, z.B. Trimethylsilyl, verestertes Carboxyl kann in üblicher Weise solvolytisch, z.B. durch Behandeln mit Wasser, einem Alkohol oder Säure freigesetzt werden.

Eine geschützte Aminogruppe setzt man in an sich bekannter und je nach Art der Schutzgruppen in verschiedenartiger Weise, vorzugsweise mittels Solvolyse oder Reduktion, frei. 2-Halogenniederalkoxycarbonylamino, gegebenenfalls nach Umwandlung einer 2-Bromniederalkoxycarbonylaminogruppe in eine 2-Jodniederalkoxycarbonylaminogruppe, Aroylmethoxycarbonylamino oder 4-Nitrobenzyloxycarbonylamino kann z.B. durch Behandeln mit einem geeigneten chemischen Reduktionsmittel, wie Zink, in Gegenwart einer geeigneten Carbonsäure, wie wässriger Essigsäure, gespalten werden. Aroylmethoxycarbonylamino kann auch durch Behandeln mit einem nucleophilen, vorzugsweise salzbildenden Reagens, wie Natriumthiophenolat, und 4-Nitro-benzyloxycarbonylamino auch durch Behandeln mit einem Alkalimetall-, z.B. Natriumdithionit, gespalten werden. Gegebenenfalls substituiertes Diphenylmethoxycarbonylamino, tert.-Niederalkoxycarbonylamino oder 2-trisubstituiertes Silyläthoxycarbonylamino kann durch Behandeln mit einer geeigneten Säure, z.B. Ameisen- oder Trifluoressigsäure, gegebenenfalls substituiertes Benzyloxycarbonylamino z.B. mittels Hydrogenolyse, d.h. durch Behandeln mit Wasserstoff in Gegenwart eines geeigneten Hydrierkatalysators, wie eines Palladiumkatalysators, gegebenenfalls substituiertes Triarylmethylamino, Formylamino oder 2-Acyl-niederalk-1-en-yl-amino, z.B. durch Behandeln mit einer Säure, wie Mineralsäure, z.B. Chlorwasserstoffsäure, oder einer organischen Säure, z.B. Ameisen-, Essig- oder Trifluoressigsäure, gegebenenfalls in Gegenwart von Wasser, und eine mit einer organischen Silyl- oder Stannylgruppe geschützte Aminogruppe z.B. mittels Hydrolyse oder Alkoholyse, freigesetzt werden. Eine durch 2-Halogenacetyl, z.B. 2-Chloracetyl, geschützte Aminogruppe kann durch Behandeln mit Thioharnstoff in Gegenwart einer Base, oder mit einem Thiolatsalz, wie einem Alkalimetallthiolat, des Thioharnstoffs und anschliessende Solvolyse, wie Alkoholyse oder Hydrolyse, des entstandenen Kondensationsproduktes freigesetzt werden. Eine durch 2-substituiertes Silyläthoxycarbonyl geschützte Aminogruppe kann auch durch Behandeln mit einem Fluoridanionen liefernden Salz der Fluorwasserstoffsäure, wie oben im Zusammenhang mit der Freisetzung einer entsprechend geschützten Carboxylgruppe angegeben, in die freie Aminogruppe übergeführt werden. Eine Phosphor-, Phosphon- oder Phosphin-amidogruppe kann z.B. durch Behandeln mit einer Phosphorhaltigen Säure, wie einer Phosphor-, Phosphon- oder Phosphinsäure, z.B. Orthophosphorsäure oder Polyphosphorsäure, einem sauren Ester, z.B. Monomethyl-, Monoäthyl-, Dimethyl- oder Diäthylphosphat oder Monomethylphosphonsäure, oder einem Anhydrid davon, wie Phosphorpentoxid, in die freie Aminogruppe übergeführt werden.

Ein in Form einer Azidogruppe maskiertes Amino wird z.B. durch Reduktion in freies Amino übergeführt, beispielsweise durch katalytische Hydrierung mit Wasserstoff in Gegenwart eines Hydrierkatalysators, wie Platinoxid, Palladium oder Raney-Nickel, oder auch durch Behandeln mit Zink in Gegenwart einer Säure, wie Essigsäure. Die katalytische Hydrierung wird vorzugsweise in einem inerten Lösungsmittel, wie einem halogenierten Kohlenwasserstoff, z.B. Methylenchlorid, oder auch in Wasser oder einem Gemisch von Wasser und einem organischen Lösungsmittel, wie einem Alkohol oder Dioxan, bei etwa 20 bis 25°C, oder auch unter Kühlen oder Erwärmen, durchgeführt.

Eine durch eine geeignete Acylgruppe, eine organische Silyl- oder Stannylgruppe oder durch gegebenenfalls substituiertes 1-Phenylniederalkyl geschützte Hydroxygruppe wird wie eine entsprechend geschützte Aminogruppe freigesetzt. Eine durch 2,2-Dichloracetyl geschützte Hydroxygruppe wird z.B. durch basische Hydrolyse, eine durch tert.-Niederalkyl oder durch einen 2-oxa- oder 2-thiaaliphatischen oder -cycloaliphatischen Kohlenwasserstoffrest verätherte Hydroxygruppe durch Acidolyse, z.B. durch Behandeln mit einer Mineralsäure oder einer starken Carbonsäure, z.B. Trifluoressigsäure, freigesetzt. Eine 2-Halogenniederalkylgruppe wird reduktiv abgespalten.

Eine geschützte, insbesondere veresterte, Sulfogruppe wird analog einer geschützten Carboxylgruppe freigesetzt.

Die beschriebenen Spaltungsreaktionen werden unter an sich bekannten Bedingungen durchgeführt, wenn notwendig unter Kühlen oder Erwärmen, in einem geschlossenen Gefäss und/oder in einer Inertgas-, z.B. Stickstoffatmosphäre.

Die an sich bekannten reduktiven Spaltungsmethoden von 2-Halogenniederalkoxycarbonyl- und 2-Halogenniederalkoxygruppen können in vorteilhafter Weise unter besonders milden Bedingungen in Anwesenheit einer katalytischen Menge eines Uebergangsmetall-Komplexes einer Corrin- oder Porphin-Verbindung durchgeführt werden, wobei auch üblicherweise schwer spaltbare Gruppen, z.B. 2-Chloräthoxycarbonyl- und 2-Chloräthoxygruppen, gespalten werden können.

Uebergangsmetall-Komplexe von Corrin- und Porphin-Verbindungen, die man als Katalysatoren der reduktiven Abspaltung einsetzt, leiten sich von solchen Uebergangsmetallen ab, die in mehr als einer Wertigkeitsstufe auftreten, wie Kupfer, Palladium, Rhodium, und vor allem Kobalt. Bevorzugt als Katalysator sind Modellverbindungen zur Synthese von Vitamin $B_{12}$, z.B. Derivate, welche sich vom 2,2,3,3,7,7,8,8,12,12,13,13,17,17,18,18-Hexadecamethyl-10,20-diazahexahydroporphin-Kobalt(III)-Kation ableiten, und insbesondere Vitamine der $B_{12}$-Gruppe, darunter vor allem Cyanocobalamin, Aquocobalamin und Hydroxycobalamin, sowie ihre Derivate und Abbauprodukte,

wie Cobyrinsäure und ihre Ester, Corphyrinsäure, Corphinsäure, Cobaminsäure und Cobamid. Die Menge des Katalysators beträgt im Normalfall etwa 0,1 bis etwa 0,001, vorzugsweise etwa 0,03 bis etwa 0,003 Moläräquivalent, bezogen auf die zu reduzierende Komponente, bei einer günstigen Versuchsanordnung, z.B. bei einer optimalisierten Elektroreduktion, kann man aber die unterste Grenze noch weit unterschreiten.

Die Reduktion wird in an sich bekannter Weise durch konventionelle Methoden in Anwesenheit einer Protonenquelle durchgeführt. Als diese können beliebige anorganische und organische Säuren sowie gebräuchliche protische Lösungsmittel dienen; Säuren werden mit Vorteil in einer gepufferten Form oder als Salze eingesetzt, wobei der pH-Wert vorzugsweise zwischen etwa 4,0 bis 9,5, insbesondere zwischen etwa 5,5 bis 7,5 und vor allem in der Nähe des Neutralpunktes liegt.

Als eine der schonendsten Reduktionsmethoden empfiehlt sich die kathodische Elektroreduktion unter konventionellen Bedingungen, wie an der Quecksilber- oder einer anderen für die Elektroreduktion allgemein verwendbaren Kathode, wie Kohle-Kathode, in Anwesenheit von üblichen Hilfselektrolyten, wie Ammonium- und/oder Alkalimetallsalzen, insbesondere Lithiumsalzen, starker anorganischer Säuren, wie insbesondere Halogeniden und Perchloraten, vorzugsweise solchen, die sich durch eine gute Löslichkeit im verwendeten wässrig-organischen Milieu auszeichnen. Als Lösungsmittel dienen, neben Wasser, übliche mit Wasser mischbare organische Lösungsmittel, z.B. Niederalkanole, wie Methanol, Aethanol und Isopropylalkohol, oder Dimethylformamid, und ähnliche niederaliphatische Amide, ferner auch Nitrile, wie Acetonitril, Aether, wie Diäthyläther, 1.2-Dimethoxy- und 1,2-Diäthoxy-äthan und cyclische Aether vom Typ Tetrahydrofuran, Ketone, wie Aceton, Carbonate, wie Dimethyl- und Diäthylcarbonat, sowie Sulfoxide, wie insbesondere Dimethylsulfoxid. Die Temperatur der Elektroreduktion kann in einem breiten Bereich variiert werden, d.h. von etwa -20°C bis zur Siedetemperatur des Reaktionsgemisches, vorzugsweise

liegt sie bei Raumtemperatur oder darunter.

Als eine weitere geeignete Variante der reduktiven Spaltung kommt die Reduktion mit einem Metall, vor allem mit Zink oder auch mit Magnesium, in Betracht. Dabei kann man unter Umständen die üblichen energischen Bedingungen, z.B. etwa 90%ige wässrige Essigsäure als Milieu und/oder erhöhte Temperatur bis zum Siedepunkt des Gemisches bei entsprechend verkürzter Reaktionszeit anwenden. Vorzugsweise arbeitet man aber unter möglichst schonenden Bedingungen, z.B. im oben erwähnten bevorzugten pH-Bereich in der Nähe des Neutralpunktes und bei Temperaturen von etwa 0° bis etwa 40°C, vorzugsweise etwa 10-25°C; üblicherweise arbeitet man mit einem etwa zehnfachen molaren Ueberschuss am Metall, vorzugsweise Zink in Form von Zinkstaub, den man mit Vorteil noch unmittelbar vor der Reaktion in konventioneller Weise, z.B. durch Verrühren mit verdünnter Salzsäure und sorgfältiges Neutralwaschen, aktiviert. Zum Erzielen der gewünschten Pufferwirkung können mit Vorteil Ammoniumsalze von Halogenwasserstoffsäuren dienen, insbesondere von denjenigen, die in der jeweiligen abzuspaltenden Gruppe der Formel I vorkommen. Die Reduktion erfolgt in den oben erwähnten organischen Lösungsmitteln oder deren Gemischen; protische Lösungsmittel sind bevorzugt. Auch andere herkömmliche Varianten des Reduktionsverfahrens sind anwendbar: als Beispiel sei die Reduktion mit Metall-Kationen in tiefen Wertigkeitsstufen, z.B. mit Chrom(II)-Salzen, wie Chrom(II)-chlorid oder -acetat, ferner auch die Reduktion mit komplexen Metallhydriden, z.B. mit Natriumborhydrid und verwandten Niederalkoxy-natriumborhydriden und Niederalkoxy-lithiumborhydriden, sowie die katalytische Hydrierung, z.B. an einem Palladium-Katalysator, erwähnt.

Bevorzugt werden beim Vorhandensein von mehreren geschützten funktionellen Gruppen die Schutzgruppen so gewählt, dass gleichzeitig mehr als eine solche Gruppe abgespalten werden kann, beispielsweise acidolytisch, wie durch Behandeln mit Trifluoressigsäure oder Ameisensäure, oder reduktiv, wie durch Behandeln mit Zink und Essigsäure, oder mit Wasserstoff und einem Hydrierkatalysator, wie einem Palladium/Kohle Katalysator.

Salzbildung: Salze von Verbindungen der Formel I mit salzbildenden Gruppen können in an sich bekannter Weise hergestellt werden. So kann man Salze von Verbindungen der Formel I mit sauren Gruppen, z.B. durch Behandeln mit Metallverbindungen, wie Alkalimetallsalzen von geeigneten organischen Carbonsäuren, z.B. dem Natriumsalz der α-Aethyl-capronsäure, oder mit anorganischen Alkali- oder Erdalkalimetallsalzen, z.B. Natriumhydrogencarbonat, oder mit Ammoniak oder einem geeigneten organischen Amin bilden, wobei man vorzugsweise stöchiometrische Mengen oder nur einen kleinen Ueberschuss des salzbildenden Mittels verwendet. Säureadditionssalze von Verbindungen der Formel I erhält man in üblicher Weise, z.B. durch Behandeln mit einer Säure oder einem geeigneten Anionenaustauschreagens. Innere Salze von Verbindungen der Formel I, welche z.B. eine freie Carboxylgruppe enthalten, können z.B. durch Neutralisieren von Salzen, wie Säureadditionssalzen, auf den isoelektrischen Punkt, z.B. mit schwachen Basen, oder durch Behandeln mit flüssigen Ionenaustauschern gebildet werden.

Salze können in üblicher Weise in die freien Verbindungen übergeführt werden, Metall- und Ammoniumsalze z.B. durch Behandeln mit geeigneten Säuren, und Säureadditionssalze, z.B. durch Behandeln mit einem geeigneten basischen Mittel.

Das 1-Oxid der Formel I, worin der Index n den Wert 1 hat, kann in der a- oder β-Form oder als Gemisch der beiden Isomeren bei den vorstehend beschriebenen nachträglichen Reaktionen eingesetzt werden.

Zur Trennung von racemischen Mischungen eignen sich die in der Literatur beschriebenen Methoden, z.B. fraktionierte Kristallisation, Chromatographie etc.

Das Verfahren umfasst auch diejenigen Ausführungsformen, wonach als Zwischenprodukte anfallende Verbindungen als Ausgangsstoffe verwendet und die restlichen Verfahrensschritte mit diesen durchgeführt werden, oder das Verfahren auf irgendeiner Stufe abgebrochen wird; ferner können Ausgangsstoffe in Form von Derivaten verwendet oder während der Reaktion gebildet werden.

Vorzugsweise werden solche Ausgangsstoffe verwendet und die Reaktionsbedingungen so gewählt, dass man zu den vorstehend als besonders bevorzugt aufgeführten Verbindungen gelangt.

Ausgangsstoffe:

Die im Verfahren zur Herstellung der Verbindungen der vorliegenden Erfindung verwendeten Ausgangsstoffe sind bekannt oder können, falls neu, in an sich bekannter Weise hergestellt werden.

Die Ausgangsverbindungen vom Typ der Formel II, sowie entsprechende Verbindungen mit geschützten funktionellen Gruppen, sind bekannt .

Folgende Verbindungen der Formel III, sowie Verfahren zu ihrer Herstellung,sind aus der Deutschen Offenlegungsschrift 2 432 190 bekannt oder können in an sich bekannter Weise hergestellt werden:
R $R_o$ und $R_1$ bedeuten Wasserstoff; R stellt einen Acylrest der Formel $R^a-SO_2-$ dar, worin $R^a$ Niederalkyl bedeutet, $R_o$ bedeutet Wasserstoff oder Niederalkyl und $R_1$ Wasserstoff; R stellt einen Acylrest der Formel $R^aR^aN-CO-$, $R^aR^aN-CS-$ oder $R^aR^aN-SO_2-$ dar, worin jeweils ein

Rest $R^a$ Niederalkyl und der andere Rest $R^a$ Wasserstoff bedeutet, $R_o$ und $R_1$ bedeuten Wasserstoff.

Verbindungen der Formel III, worin R einen Acylrest der Formel $R^a$-O-CO- darstellt, worin $R^a$ die oben gegebenen Bedeutungen hat, oder Verbindungen der Formel III, worin R einen Acylrest der Formel $R^aR^aN$-CO-, $R^aR^aN$-CS- oder $R^aR^aN-SO_2$- darstellt, worin jeweils beide Reste $R^a$ gegebenenfalls substituierte Kohlenwasserstoffreste oder beide Reste $R^a$ Wasserstoff bedeuten, lassen sich beispielsweise durch Acylierung einer Verbindung der Formel III, worin R, $R_o$ und $R_1$ Wasserstoff bedeuten, mit dem Acylrest R eines entsprechend substituierten Kohlensäurehalbesters, einer entsprechend substituierten Carbaminsäure, Thiocarbaminsäure oder Amidosulfonsäure, bzw. einem reaktionsfähigen Derivat von solchen Säuren, z.B. einem Säureanhydrid, wie einem Säurechlorid, analog Verfahren a) herstellen.

Verbindungen der Formel III, worin R eine Acylgruppe mit den oben genannten Bedeutungen und $R_o$ Wasserstoff bedeuten und $R_1$ eine Gruppe der Teilformel (R)($R_o$)N- darstellt, worin R eine Acylgruppe und $R_o$ Wasserstoff bedeuten, lassen sich durch Acylierung von freien Aminogruppen einer entsprechenden Verbindung der Formel III mit dem Acylrest einer entsprechend substituierten Carbonsäure, Sulfonsäure, eines Kohlensäurehalbesters, einer Carbaminsäure, Thiocarbaminsäure oder Amidosulfonsäure, bzw. einem reaktionsfähigen Derivat von solchen Säuren, z.B. einem Säureanhydrid wie einem Säurechlorid, analog Verfahren a) herstellen.

Verbindungen der Formel III, worin R und $R_o$ Wasserstoff bedeuten und $R_1$ eine Gruppe der Teilformel (R)($R_o$)N- darstellt, worin R und $R_o$ ebenfalls Wasserstoff bedeuten, lassen sich durch Reduktion der entsprechenden Dinitrohydroxyphenyl-D-2-aminoessigsäuren, z.B. von 3,5-Dinitro-4-hydroxyphenyl-D-2-aminoessigsäure, mit Raney-Nickel oder einem der oben genannten Nitrogruppen in Aminogruppen überführenden Reduktionsmittel, z.B. mit Palladium-Kohle, herstellen.

In Verbindungen der Formel III, worin R und $R_o$ Wasserstoff bedeuten, $R_1$ Wasserstoff oder eine Gruppe der Teilformel (R)($R_o$)N- mit den oben für R und $R_o$ gegebenen Bedeutuungen darstellt, kann (können) die Aminogruppe(n) am Phenylring in an sich bekannter Weise durch ein, z.B. einen Niederalkylrest $R_o$ einführendes geeignetes Alkylierungsmittel, z.B. ein Alkylhalogenid, wie Methylbromid, alkyliert werden.

Solche alkylierte(n) Aminogruppe(n) am Phenylring lässt (lassen) sich anschliessend in der oben geschilderten Weise mit einem den Acylrest R einführenden Acylierungsmittel substituieren.

Man gelangt so zu Verbindungen der Formel III, worin R eine Acylgruppe und $R_o$ einen Niederalkylrest bedeuten und $R_1$ Wasserstoff oder eine Gruppe der Teilformel (R)($R_o$)N- darstellt, worin R ebenfalls eine Acylgruppe und $R_o$ einen Niederalkylrest bedeuten.

Solche Verbindungen lassen sich bevorzugt durch Substitution der Aminogruppe(n) am Phenylring durch einen den oben gegebenen Bedeutungen entsprechenden Acylrest R und anschliessende Metallierung der so gebildeten sekundäre(n) Aminogruppe(n) am Phenylring mit einem geeigneten Metallierungsreagenz, gefolgt von einer dem Rest $R_o$ entsprechenden reaktionsfähigen Verbindung, herstellen.

Geeignete Metallierungsreagenzien sind beispielsweise Lithiumdiisopropylamid oder Butyllithium. Eine dem Rest $R_o$ entsprechende reaktionsfähige Verbindung ist beispielsweise eine Verbindung der Formel $R_o$-X, worin X eine nukleofuge Abgangsgruppe, beispielsweise ein Halogenatom, z.B. Chlor, Brom oder Jod, oder eine Sulfonyloxygruppe, z.B. Mesyloxy oder Tosyloxy darstellt.

Bei den jeweiligen Acylierungs- und Alkylierungsreaktionen der Aminogruppe(n) am Phenylring ist die Hydroxygruppe am Phenylring durch die oben beschriebenen üblichen Hydroxyschutzgruppen und die D-2-Aminogruppe durch die oben beschriebenen üblichen Aminoschutzgruppen gegebenenfalls geschützt.

Verbindungen der Formel III besitzen am Kohlenstoffatom in 2- oder α-Stellung des Acetylrests ein Chiralitätszentrum.

Für das obige Acylierungsverfahren gemäss a) zur Herstellung von Verbindungen der Formel I werden bevorzugt Verbindungen der Formel III eingesetzt, worin die Aminogruppe, welche sich in 2- oder α-Stellung des Acylrests befindet, die D-Konfiguration besitzt. Solche Verbindungen sind beispielsweise durch Auftrennen von racemischen Mischungen von Verbindungen der Formel III in die gewünschten D-konfigurierten Antipoden erhältlich. Dabei können eine Vielzahl üblicher in der Literatur beschriebener, insbesondere solche für die Spaltung von racemischen α-Aminosäuren angewandten Methoden, Verwendung finden.

Ausgangsstoffe der Formel IV können u.a. als Nebenprodukt bei der Herstellung von Verbindungen vom Typ der Formel I, z.B. wenn diese unter basischen Bedingungen gebildet werden, gebildet werden. Sie brauchen nicht in reiner Form vorzuliegen, sondern können z.B. auch im Gemisch mit entsprechenden Verbindungen der Formel I eingesetzt werden.

Verbindungen der Formel V, worin $R_1'$ Wasserstoff, Nitro oder eine Gruppe der Teilformel $(R)(R_o)N-$ darstellt, worin R Wasserstoff oder eine Acylgruppe und $R_o$ Wasserstoff oder Niederalkyl bedeuten, $R_2$ Carboxyl oder geschütztes Carboxyl und $R_3$ Wasserstoff, Halogen mit Ordnungszahl bis 35 oder Niederalkoxy bedeuten, sowie Hydrate und Salze von Verbindungen der Formel V und Verfahren zu ihrer Herstellung sind ebenfalls Gegenstand der vorliegenden Erfindung. Verbindungen der Formel V werden beispielsweise hergestellt, indem man in einer Verbindung der Formel

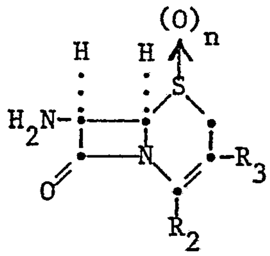

(II),

worin der Index n für 0 oder 1 steht und die 7β-Aminogruppe gegebenenfalls durch eine die Acylierung erlaubende Gruppe geschützt ist, und $R_2$ und $R_3$ die unter Formel V genannten Bedeutungen haben, die 7β-Aminogruppe durch Umsetzung mit einem Acylrest einer Carbonsäure der Formel

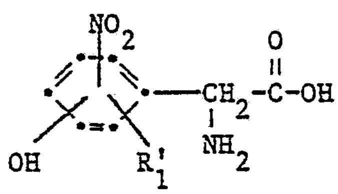

(VI)

einführenden Acylierungsmittel, worin die Hydroxygruppe am Phenylring und/oder die D-2-Aminogruppe gegebenenfalls in geschützter Form vorliegen und worin weitere vorhandene funktionelle Gruppen gegebenenfalls

geschützt sind, acyliert und, wenn erwünscht, eine erhältliche Verbindung, worin der Index n 1 bedeutet, in eine Verbindung umwandelt, worin der Index n 0 bedeutet und/oder, wenn erwünscht, in einer erhältlichen Verbindung in geschützter Form vorliegende funktionelle Gruppen in freie funktionelle Gruppen und/oder ein erhältiches Salz in die freie Verbindung oder in ein anderes Salz überführt und/oder eine erhältliche freie Verbindung mit einer salzbildenden Gruppe in ein Salz überführt und/oder ein erhältliches Gemisch von isomeren Verbindungen in die einzelnen Isomeren auftrennt.

Die neuen Verbindungen der Formel V, worin die funktionellen Gruppen entweder in freier Form vorliegen oder die 4-Carboxylgruppe

gegebenenfalls in physiologisch spaltbarer veresterter Form vorliegt, und ihre pharmazeutisch verwendbaren, nicht toxischen Salze weisen wertvolle pharmakologische Eigenschaften auf.

So sind Verbindungen der Formel V in freier Form oder in Form ihrer Salze in vitro gegen grampositive und gegen gramnegative Kokken, z.B. Staphylococcus aureus, Streptococcus pyogenes oder Neisseria spp. in Konzentrationen von ca. 0,1 bis ca. 32 µg/ml, gegen gramnegative Bakterien, beispielsweise Enterobakterien, z.B. Escherichia coli, Klebsiella pneumoniae, Salmonella typhimurium, Proteus spp., in Konzentrationen von ca. 2 bis ca. 64 µg/ml, gegen Anaerobien, z.B. Chlostridium perfringens, bei einer Konzentration von ca. 0,1 µg/ml wirksam.

In vivo, bei peroraler Verabreichung an der Maus, sind Verbindungen der Formel V gegen systemische Infektionen, welche durch grampositive Kokken, z.B. Staphylococcus aureus, sowie gramnegative Bakterien, z.B. Escherichia coli oder Klebsiella pneumoniae verursacht werden, in einem Dosisbereich von ca. 7 bis ca. 18 mg/kg bei geringer Toxizität wirksam.

In Verbindungen der Formel V haben R, $R_o$, $R_2$ und $R_3$ die oben unter Formel I genannten bevorzugten Bedeutungen.

Die in Verbindungen der Formel V vorhandenen funktionellen Gruppen, insbesondere die 4-Carboxyl-, die D-2-Amino- und die Hydroxygruppe am Phenylring, sind gegebenenfalls durch die oben unter Formel I genannten Schutzgruppen geschützt.

Salze von Verbindungen der Formel V sind die oben unter Formel I genannten Salze.

Verbindungen der Formel V besitzen am Kohlenstoffatom in 2- oder α-Stellung des Acetylrests ein Chiralitätszentrum. Für die Herstellung von Verbindungen der Formel V werden bei der Acylierung bevorzugt Verbindungen der Formel III eingesetzt, worin die Aminogruppe in 2-Stellung des Acetylrests die D-Konfiguration besitzt. Diese Verbindungen sind beispielsweise durch Auftrennen von racemischen Mischungen von Verbindungen der Formel VI mit Hilfe der üblichen Methoden erhältlich.

Das Acylierungsverfahren erfolgt analog den oben unter a) beschriebenen Verfahren.

Carbonsäuren der Formel VI worin $R_1'$ Nitro oder Wasserstoff bedeuten, sind aus der Deutschen Offenlegungsschrift 2 444 762 bekannt.

Verbindungen der Formel VI, worin $R_1'$ eine Gruppe der Teilformel $(R)(R_o)N-$ darstellt, worin R eine Acylgruppe mit den oben genannten Bedeutungen und $R_o$ Wasserstoff bedeutet, lassen sich beispielsweise durch Acylierung einer Carbonsäure der Formel VI, worin $R_1'$ eine Gruppe der Teilformel $(R)(R_o)N-$ darstellt, worin R und $R_o$ Wasserstoff bedeuten, mit dem Acylrest einer entsprechend substituierten Carbonsäure, Sulfonsäure, eines Kohlensäurehalbesters, Carbaminsäure, Thiocarbaminsäure oder Amidosulfonsäure, bzw. einem reaktionsfähigen Derivat von solchen Säuren, z.B. einem Säureanhydrid wie einem Säurechlorid, herstellen. Verbindungen der Formel VI, worin $R_1'$ eine Gruppe der Teilformel $(R)(R_o)N-$ darstellt, worin R eine Acylgruppe mit den oben genannten Bedeutungen und $R_o$ Niederalkyl darstellt, lassen sich beispielsweise durch Alkylierung einer freien Aminogruppe im Phenylring durch ein den Rest $R_o$ einführendes geeignetes Alkylierungsmittel, z.B. ein Alkylhalogenid, gegebenenfalls in Gegenwart von Basen wie Pyridin, zur sekundären Aminogruppe und anschliessende Acylierung herstellen.

Neue Ausgangsverbindungen, sowie Zwischenprodukte und Verfahren zu ihrer Herstellung, sind ebenfalls Gegenstand der vorliegenden Erfindung.

Die neuen Verbindungen der Formeln I und V der vorliegenden Erfindung können z.B. zur Herstellung von pharmazeutischen Präparaten verwendet werden, welche eine wirksame Menge der Aktivsubstanz zusammen oder im Gemisch mit anorganischen oder organischen, festen oder flüssigen, pharmazeutisch verwendbaren Trägerstoffen enthalten, die sich zur enteralen oder parenteralen Verabreichung eignen. So verwendet man Tabletten oder Gelatinekapseln, welche den Wirkstoff zusammen mit Verdünnungsmitteln, z.B. Laktose, Dextrose, Sukrose, Mannitol, Sorbitol, Cellulose und/oder Glycin, und Schmiermitteln, z.B. Kieselerde, Talk, Stearinsäure oder Salze davon, wie Magnesium- oder Calciumstearat, und/oder Polyäthylenglykol, aufweisen; Tabletten enthalten ebenfalls Bindemittel, z.B. Magnesiumaluminiumsilikat, Stärken, wie Mais-, Weizen-, Reis- oder Pfeilwurzstärke, Gelatine, Traganth, Methylcellulose, Natriumcarboxymethylcellulose und/oder Polyvinylpyrrolidon, und, wenn erwünscht, Sprengmittel, z.B. Stärken, Agar, Alginsäure oder ein Salz davon, wie Natriumalginat, und/oder Brausemischungen, oder Adsorptionsmittel, Farbstoffe, Geschmackstoffe und Süssmittel. Ferner kann man die neuen pharmakologisch wirksamen Verbindungen in Form von injizierbaren, z.B. intravenös verabreichbaren Präparaten oder von Infusionslösungen verwenden. Solche Lösungen sind vorzugsweise isotonische wässrige Lösungen oder Suspensionen, wobei diese z.B. aus lyophilisierten Präparaten, welche die Wirksubstanz allein oder zusammen mit einem Trägermaterial, z.B. Mannit, enthalten, vor Gebrauch hergestellt werden können. Die pharmazeutischen Präparate können sterilisiert sein und/oder Hilfsstoffe, z.B. Konservier-, Stabilisier-, Netz- und/oder Emulgiermittel, Löslichkeitsvermittler, Salze zur Regulierung des osmotischen Druckes und/oder Puffer enthalten. Die vorliegenden pharmazeutischen Präparate, die, wenn erwünscht, weitere pharmakologisch wertvolle Stoffe enthalten können, werden in an sich bekannter Weise, z.B. mittels konventioneller Misch-, Granulier-, Dragier-, Lösungs- oder

Lyophilisierungsverfahren, hergestellt und enthalten von etwa 0,1% bis 100%, insbesondere von etwa 1% bis etwa 50%, Lyophilisate bis zu 100% des Aktivstoffes.

Verbindungen der Formel I und V, insbesondere diejenigen, in welchen $R_2$ eine physiologisch spaltbare veresterte Carboxylgruppe, z.B. Pivaloyloxymethoxycarbonyl, darstellt, können auch oral, z.B. in Form von Kapseln, verabreicht werden. Diese enthalten den Wirkstoff, gegebenenfalls zusammen mit geeigneten Trägerstoffen, in Form eines Granulats und zwar in Dosen von etwa 0,2 g bis etwa 0,5 g pro Dosiseinheit.

Je nach Art der Infektion und Zustand des infizierten Organismus verwendet man tägliche Dosen von 0,5 bis 5 g s.c. oder p.o. zur Behandlung von Warmblütern von ca. 70 kg Gewicht.

Die folgenden Beispiele dienen zur Illustration der Erfindung. Temperaturen werden in Celsiusgraden angegeben.

Beispiel 1: a) Eine auf 0° gekühlte Lösung von 3,5 g (4,7 mMol) 7β-[D-2-tert.-Butyloxycarbonylamino-2-(3-methylsulfonylamino-4-hydroxyphenyl)-acetylamino]-3-methoxy-ceph-3-em-4-carbonsäurediphenylmethylester und 3,5 ml Anisol in 18 ml absolutem Methylenchlorid wird mit 18 ml kalter Trifluoressigsäure versetzt, während 45 Min. bei 0° unter Stickstoffatmosphäre gerührt und anschliessend mit 100 ml Diäthyläther bei 0° versetzt. Der beige Niederschlag wird abfiltriert, mit wenig Diäthyläther gewaschen und unter vermindertem Druck getrocknet. Das so erhaltene Trifluoressigsäuresalz der 7β-[D-2-Amino-2-(3-methylsulfonylamino-4-hydroxyphenyl)-acetylamino]-3-methoxy-ceph-3-em-4-carbonsäure wird in 20 ml Eiswasser gelöst und mit Essigsäureäthylester (5x10 ml) extrahiert. Die saure wässrige Phase (pH~2) wird durch tropfenweise Zugabe von 2N Natriumhydroxidlösung auf pH 4.5 gestellt und bei 0° mit Aethanol (100 ml) versetzt. Der gebildete Niederschlag wird abfiltriert, mit 20 ml Wasser-Aethanol 1:4 gewaschen, zur Entfernung des organischen Lösungsmittels in ca. 10 ml Wasser aufgenommen und am Rotationsverdampfer eingeengt. Nach dem Trocknen (16 Stunden, Raumtemperatur, 0.05 Torr ) wird 7β-[D-2-Amino-2-(3-methylsulfonylamino-4-hydroxyphenyl)-acetylamino]-3-methoxy-ceph-3-em-4-carbonsäure in Form eines Monohydrates erhalten.

Smp. ab 150° (unter Zersetzung);

DC (Silikagel, Entwickeln mit Ninhydrin): Rf~0.46
(System: sek. Butanol-Essigsäure-Wasser 67:10:23);

UV-Spektrum (in 0.1 N wässriger Salzsäure): 278 nm (ε=7700);

$[\alpha]_D$ = +144 ± 1° (in 0.1 N wässriger Salzsäure, c=1.340%).

Das Ausgangsmaterial kann wie folgt erhalten werden:

b) Eine auf -15° gekühlte Lösung von 5,26 g (14,6 mMol) D-2-tert.-Butyloxycarbonylamino-2-(3-methylsulfonylamino-4-hydroxyphenyl)-essigsäure und 1,6 ml N-Methylmorpholin in 100 ml absolutem Tetrahydrofuran wird unter Stickstoffatmosphäre mit 1,9 ml Chlorameisensäureisobutylester und nach einer Stunde mit 6.3 g (14,6 mMol) 7β-Amino-3-

methoxy-ceph-3-em-4-carbonsäurediphenylmethylester-hydrochlorid und 1,6 ml N-Methylmorpholin versetzt. Nach 3 Stunden Rühren bei Raumtemperatur wird das Reaktionsgemisch mit Essigsäureäthylester verdünnt, je zweimal mit Eiswasser und gesättigter Natriumchloridlösung gewaschen, über Natriumsulfat getrocknet und am Rotationsverdampfer vom Lösungsmittel befreit. Der Rückstand wird an Silikagel mit Diäthyläther-Essigsäureäthylester 1:1 als Eluiermittel gereinigt, woraus 7β-[D-2-tert.-Butyloxycarbonylamino-2-(3-methylsulfonylamino-4-hydroxyphenyl)-acetylamino]-3-methoxy-ceph-3-em-4-carbonsäurediphenylmethylester als farbloses amorphes Pulver erhalten wird.
DC (Silikagel, Identifikation mit Jod): Rf~0,26 (Diäthyläther-Essigsäureäthylester 1:1).

Beispiel 2: a) In analoger Weise zu Beispiel 1 a) erhält man durch Behandeln von 2,13 g (3 mMol) 7β-[D-2-tert.-Butyloxycarbonylamino-2-(3-methylsulfonylamino-4-hydroxyphenyl)-acetylamino]-ceph-3-em-4-carbonsäurediphenylmethylester mit 10 ml Trifluoressigsäure und 2 ml Anisol in 10 ml absolutem Methylenchlorid 7β-[D-2-Amino-2-(3-methylsulfonylamino-4-hydroxyphenyl)-acetylamino]-ceph-3-em-4-carbonsäure in Form eines 1.5 Hydrates.
Smp. ab 200° unter Zersetzung;
DC (Silikagel, Entwickeln mit Ninhydrin): Rf~0,19 (System: sek. Butanol-Essigsäure-Wasser 67:10.23);
UV-Spektrum (in 0,1 N wässeriger Salzsäure): 232 nm (ε = 11600), 270 nm (Schulter);
$[\alpha]_D$ = +122 ±1° (0,1 N wässrige Salzsäure ,. c=1.353%).

Das Ausgangsmaterial kann wie folgt erhalten werden:

b) In analoger Weise zu Beispiel 1 b) erhält man durch Behandeln des gemischten Anhydrids, erhalten aus 3,6 g (10,0 mMol) D-2-tert.-Butyloxycarbonylamino-2-(3-methylsulfonylamino-4-hydroxyphenyl)-essigsäure und 1,3 ml Chlorameisensäureisobutylester in Gegenwart von 1,1 ml N-Methylmorpholin und 100 ml abs. Tetrahydrofuran bei -15°, mit 3,66 g (10 mMol) 7β-Amino-ceph-3-em-4-carbonsäure-diphenylmethylester nach

dem in Beispiel 1 b) beschriebenen Verfahren den 7β-[D-2-tert.-Butyloxycarbonylamino-2-(3-methylsulfonylamino-4-hydroxyphenyl)-acetylamino]-ceph-3-em-4-carbonsäurediphenylmethylester.
DC (Silikagel, Identifikation mit Jod): Rf ~0,48 (Essigsäureäthylester).

Beispiel 3: a) In analoger Weise zu Beispiel 1 a) erhält man durch Behandeln von 2,3 g (3,1 mMol) 7β-[D-2-tert.-Butyloxycarbonylamino-2-(3-methylsulfonylamino-4-hydroxyphenyl)-acetylamino]-3-chlor-ceph-3-em-4-carbonsäurediphenylmethylester mit 11 ml Trifluoressigsäure und 2,3 ml Anisol in 11 ml absolutem Methylenchlorid nach dem beschriebenen Verfahren 7β-[D-2-Amino-2-(3-methylsulfonylamino-4-hydroxyphenyl)-acetylamino]-3-chlor-ceph-3-em-4-carbonsäure in Form des Monohydrates. Smp. ab 172°C unter Zersetzung;
DC (Silikagel, Entwickeln mit Ninhydrin): Rf ~0,16 (System: sek.-Butanol-Essigsäure-Wasser 67:10:23);
UV-Spektrum (in 0,1 N wässeriger Salzsäure): 235 nm (Schulter), 270 nm ($\varepsilon$ = 8600);
$[\alpha]_D$ = +96 ±1° (0,1 N wässrige Salzsäure , c=1.313%).

Das Ausgangsmaterial kann wie folgt erhalten werden:

b) In analoger Weise zu Beispiel 1 b) erhält man durch Behandeln des gemischten Anhydrids, erhalten aus 3,0 g (8,3 mMol) D-2-tert.-Butyloxycarbonylamino-2-(3-methylsulfonylamino-4-hydroxyphenyl)-essigsäure und 1,1 ml Chlorameisensäureisobutylester in Gegenwart von 0,92 ml N-Methylmorpholin und 100 ml abs. Tetrahydrofuran bei -15°, mit 3,33 g (8,3 mMol) 7β-Amino-3-chlor-ceph-3-em-4-carbonsäurediphenylmethylester nach dem im Beispiel 1 b) beschriebenen Verfahren den 7β-[D-2-tert.-Butyloxycarbonylamino-2-(3-methylsulfonylamino-4-hydroxyphenyl)-methylamino]-3-chlor-ceph-3-em-4-carbonsäurediphenylmethylester.
DC (Silikagel, Identifikation mit Jod): Rf ~0,38 (Toluol-Essigsäureäthylester 1:1).

Beispiel 4: a) Eine auf 0° gekühlte Lösung von 3,8 g (5,75 mMol) 7β-[D-2-tert.-Butyloxycarbonylamino-2-(3-amino-4-hydroxyphenyl)-acetylamino—3-methoxy-ceph-3-em-4-carbonsäurediphenylmethylester und 3,8 ml Anisol in 20 ml absolutem Methylenchlorid wird mit 20 ml kalter Trifluoressigsäure versetzt, während 45 Min. bei 0° unter Stickstoffatmosphäre gerührt und anschliessend mit 200 ml Petroläther-Diäthyläther 1:1 versetzt. Der beige Niederschlag wird abfiltriert, mit wenig Diäthyläther gewaschen, darauf in 40 ml Eiswasser gelöst und mit Essigsäureäthylester (4x20 ml) extrahiert. Die wässrige Phase (pH~2) wird mit 1N Natronlauge auf pH = 5 gestellt, unter vermindertem Druck auf ca, die Hälfte des Volumens eingeengt und tropfenweise bei 0° mit Aethanol (80 ml) versetzt. Das so erhaltene Rohprodukt an 7β-[D-2-Amino-2-(3-amino-4-hydroxyphenyl)-acetylamino]-3-methoxy-ceph-3-em-4-carbonsäure wird an Amberlite XAD-2 mit Wasser-Isopropanol 88:12 als Eluiermittel gereinigt; Smp. ab 150° unter Zersetzung.
DC (Silikagel, Entwickeln mit Ninhydrin): Rf~0,13 (sek.-Butanol-Essigsäure-Wasser 67:10:23).

Das Ausgangsmaterial kann wie folgt hergestellt werden:
b) Eine Lösung von 8 g (11.6 mMol) 7β-[D-2-tert.-Butyloxycarbonylamino-2-(3-nitro-4-hydroxyphenyl)-acetylamino]-3-methoxy-ceph-3-em-4-carbonsäurediphenylmethylester in Essigsäureäthylester (150 ml) wird in Gegenwart von 2 g Palladium-Kohle (10%-ig) als Katalysator hydriert. Nach der Aufnahme der theoretischen Menge (778 ml) Wasserstoff wird das Reaktionsgemisch über Celite filtriert und unter vermindertem Druck vom Lösungsmittel befreit. Der Rückstand wird darauf an Silikagel mit Essigsäureäthylester als Eluiermittel gereinigt, woraus 7β-[D-2-tert.-Butyloxycarbonylamino-2-(3-amino-4-hydroxyphenyl)-acetylamino]-3-methoxy-ceph-3-em-4-carbonsäurediphenylmethylester als amorphes beiges Pulver erhalten wird;
DC (Silikagel, Entwickeln mit Jod): Rf~0,35 (Essigsäureäthylester).

c) Eine auf -15° gekühlte Lösung von 10,9 g (35 mMol) D-2-tert.-Butyloxycarbonylamino-2-(3-nitro-4-hydroxyphenyl)-essigsäure und 3,9 ml N-Methylmorpholin in 350 ml absolutem Methylenchlorid wird unter Stickstoffatmosphäre mit 4,6 ml Chlorameisensäureisobutylester und später mit 15,15 g (35 mMol) 7β-Amino-3-methoxy-ceph-3-em-4-carbonsäurediphenylmethylesterhydrochlorid und 3,9 ml N-Methylmorpholin versetzt. Nach 3 Stunden Rühren bei Raumtemperatur wird das Reaktionsgemisch mit Essigsäureäthylester verdünnt, je zweimal mit Eiswasser und gesättigter Natriumchloridlösung gewaschen, über Natriumsulfat getrocknet und am Rotationsverdampfer vom Lösungsmittel befreit. Der Rückstand wird an Silikagel mit Diäthyläther-Essigsäureäthylester 3:1 als Eluiermittel gereinigt, woraus 7β-[D-2-tert.-Butyloxycarbonylamino-2-(3-nitro-4-hydroxyphenyl)-acetylamino]-3-methoxy-ceph-3-em-4-carbonsäure diphenylmethylester als farbloses amorphes Pulver erhalten wird.
DC (Silikagel, Identifikation mit Jod): Rf~0,65 (Diäthyläther-Essigsäureäthylester 1:1).

d) Eine auf 0° gekühlte Suspension von 30 g (0,14 Mol) D-2-Amino-2-(3-nitro-4-hydroxyphenyl)-essigsäure in 140 ml Wasser wird unter Rühren mit 2 N Natronlauge auf pH 7 gestellt, anschliessend mit 280 ml Dioxan verdünnt und mit 15 g (0,14 Mol) Natriumcarbonat und 33,8 g (0,15 Mol) Di-tert.-Butyldicarbonat versetzt. Nach einer Reaktionszeit von 3 Stunden bei Raumtemperatur wird das wieder auf 0° gekühlte Reaktionsgemisch mit 4 N Salzsäure auf pH 7 gebracht und am Rotationsverdampfer von den Lösungsmitteln befreit. Der ölige Rückstand wird in 1 N Natriumbicarbonatlösung (200 ml) aufgenommen und mit Diäthyläther (4 x 100 ml) extrahiert. Die wässerige Phase wird darauf mit 4 N Salzsäure auf pH 2 gebracht und mit Essigsäureäthylester (4 x 150 ml) extrahiert. Die über Natriumsulfat getrockneten vereinigten organischen Phasen werden unter vermindertem Druck vom Lösungsmittel befreit, woraus D-2-tert.-Butyloxycarbonylamino-2-(3-nitro-4-hydroxyphenyl)-essigsäure erhalten wird.
DC (Silikagel, Identifikation mit Ninhydrin): Rf~0,73 (sek. Butanol-Essigsäure-Wasser 67:10:23).

e) 33,4 g (0,2 Mol) D-2-Amino-2-(4-hydroxyphenyl)-essigsäure werden portionenweise unter Rühren auf 0° gekühlte 40%-ige Salpetersäure (86 ml) gegeben. Nach 3 Stunden bei Raumtemperatur wird das wiederum auf 0° gekühlte Reaktionsgemisch mit 40 ml Eiswasser verdünnt und mit 2 N Natronlauge (250 ml) auf pH 3,5 gestellt. Der entstandene Niederschlag wird darauf abfiltriert, mit wenig Eiswasser gewaschen und am Hochvakuum bis zur Gewichtskonstanz getrocknet. Dabei wird D-2-Amino-2-(3-nitro-4-hydroxyphenyl)-essigsäure als gelbes Pulver erhalten, das ohne weitere Reinigung im nächsten Reaktionsschritt eingesetzt werden kann.
DC (Silikagel, Identifikation mit Ninhydrin) Rf~0,38 (sek. Butanol-Essigsäure-Wasser 67:10:23).

Beispiel 5: In analoger Weise zu Beispiel 4 a) erhält man durch Behandeln von 3,45 g (5,0 mMol) 7β-[D-2-tert.-Butyloxycarbonylamino-2-(3-nitro-4-hydroxyphenyl)-acetylamino]-3-methoxy-ceph-3-em-4-carbonsäurediphenylmethylester mit 17 ml Trifluoressigsäure und 3,5 ml Anisol in 17 ml absolutem Methylenchlorid 7β-[D-2-Amino-2-(3-nitro-4-hydroxyphenyl)-acetylamino]-3-methoxy-ceph-3-em-4-carbonsäure; Smp. ab 160° unter Zersetzung.
DC (Silikagel, Entwickeln mit Ninhydrin):Rf~0,31 (n-Butanol-Essigsäure-Wasser 45:45:10);
UV (0,1 N $NaHCO_3$-Lösung): 216 nm (ε = 17100), 268 nm (ε = 7200), 343 nm (ε = 2280).

Beispiel 6: a) Eine auf 0° gekühlte Lösung von 0,95 g (1,4 mMol) 7β-[D-2-tert.-Butyloxycarbonylamino-2-(3,5-diamino-4-hydroxyphenyl)-acetylamino]-3-methoxy-ceph-3-em-4-carbonsäurediphenylmethylester und 1,0 ml Anisol, in 5 ml absolutem Methylenchlorid wird mit 5 ml kalter Trifluoressigsäure versetzt, während 45 Min. bei 0° unter einer Stickstoffatmosphäre gerührt und anschliessend mit 100 ml Diäthyläther versetzt. Der beige Niederschlag wird abfiltriert, mit wenig Diäthyläther gewaschen, darauf in 10 ml Eiswasser gegeben und mit Essigsäureäthylester (4 x 10 ml) extrahiert. Die wässrige Phase (pH~2) wird mit 1 N Natronlauge auf pH = 5 gestellt, und tropfenweise bei

0° mit 50 ml Isopropanol versetzt. Das so erhaltene Rohprodukt 7β-[D-2-Amino-2-(3,5-diamino-4-hydroxyphenyl)-acetylamino]-3-methoxy-ceph-3-em-4-carbonsäure wird an Amberlite XAD-2 mit Wasser-Isopropanol 88:12 als Eluiermittel gereinigt.
DC (Silikagel, Entwickeln mit Ninhydrin): Rf~0,05 (sek. Butanol-Essigsäure-Wasser 67:10:23);
UV-Spektrum (in 0,1 N wässriger Salzsäure): 250 nm (Schulter), 270 nm (7800).

Das Ausgangsmaterial kann wie folgt hergestellt werden:
b) Eine Lösung von 2,5 g (3,4 mMol) 7β-[D-2-tert.-Butyloxycarbonylamino-2-(3,5-dinitro-4-hydroxyphenyl)-acetylamino]-3-methoxy-ceph-3-em-4-carbonsäurediphenylmethylester in Essigsäureäthylester (50 ml) wird in Gegenwart von 2 g Palladium-Kohle (10%-ig) als Katalysator hydriert. Nach der Aufnahme der theoretischen Menge Wasserstoff wird das Reaktionsgemisch über Celite filtriert und unter vermindertem Druck vom Lösungsmittel befreit. Der Rückstand wird darauf an Silikagel mit Essigsäureäthylester als Eluiermittel gereinigt, woraus 7β-[D-2-tert.-Butyloxycarbonylamino-2-(3,5-diamino-4-hydroxyphenyl)-acetylamino]-3-methoxy-ceph-3-em-4-carbonsäurediphenylmethylester als amorphes braunes Pulver erhalten wird.
DC (Silikagel, Entwickeln mit Jod): Rf~0,10 (Essigsäureäthylester).

c) Eine auf -15° gekühlte Lösung von 7,14 g (20 mMol) D-2-tert.-Butyloxycarbonylamino-2-(3,5-dinitro-4-hydroxyphenyl)-essigsäure und 2,2 ml N-Methylmorpholin in 200 ml absolutem Methylenchlorid wird unter Stickstoffatmosphäre mit 2,6 ml Chlorameisensäureisobutylester und nach einer Stunde mit 8,66 g (20 mMol) 7β-Amino-3-methoxy-ceph-3-em-4-carbonsäurediphenylmethylesterhydrochlorid und 2,2 ml N-Methylmorpholin versetzt. Nach 3 Stunden bei Raumtemperatur wird das Reaktionsgemisch mit Essigsäureäthylester verdünnt, je zweimal mit Eiswasser und gesättigter Natriumchloridlösung gewaschen, über Natriumsulfat getrocknet und am Rotationsverdampfer vom Lösungsmittel befreit. Der Rückstand wird an Silikagel mit Essigsäureäthylester als Eluiermittel gereinigt, woraus 7β-[D-2-tert.-Butyloxycarbonylamino-2-(3,5-dinitro-4-hydroxyphenyl)-acetylamino]-3-methoxy-ceph-3-em-4-carbonsäurediphenylmethylester als gelbes amorphes Pulver erhalten wird; DC (Silikagel, Identifikation mit Jod): Rf~0,25 (Essigsäureäthylester).

d) Zu einer Suspension von 33,44 g (0,2 Mol) D-2-Amino-2-(4-hydroxyphenyl)-essigsäure in 56 ml konz. Schwefelsäure werden bei 0-5° unter Rühren 56 ml Salpetersäure zugetropft. Nach 2 Stunden Reaktionszeit bei 20-25° wird das Reaktionsgemisch mit 160 ml Eiswasser verdünnt, mit 2 N Natronlauge auf pH 7 gestellt und unter vermindertem Druck auf ca. 200 ml eingeengt. Nach der Zugabe von 400 ml Dioxan und 21,2 g Natriumcarbonat werden 48 g Di-tert.-Butyl-dicarbonat zugegeben. Nach 3 Stunden wird zur Entfernung des organischen Lösungsmittels das Reaktionsgemisch eingeengt, wieder mit Wasser (200 ml) verdünnt und mit Diäthyläther (2.200 ml) extrahiert. Die wässrige Phase wird darauf mit 4 N Salzsäure auf pH 2 gestellt und mit Essigsäureäthylester (4x200 ml) extrahiert. Nach Trocknen der vereinigten organischen Phasen über Natriumsulfat wird das Lösungsmittel am Rotationsverdampfer entfernt. Die so erhaltene D-2-tert.-Butyloxycarbonylamino-2-(3,5-dinitro-4-hydroxyphenyl)-essigsäure ist ein blassgelbes Kristallisat. Smp. 96-98°;
DC (Silikagel, Identifikation mit Jod):Rf~0,68 (sek.-Butanol-Essigsäure-Wasser 67:10:23).

Beispiel 7: Eine auf 0° gekühlte Lösung von 2,94 g (4 mMol) 7β-[D-2-tert.-Butyloxycarbonylamino-2-(3,5-dinitro-4-hydroxyphenyl)-acetylamino]-3-methoxy-ceph-3-em-4-carbonsäurediphenylmethylester (Beispiel 6 c)) und 3 ml Anisol in 15 ml absolutem Methylenchlorid wird mit 15 ml kalter Trifluoressigsäure versetzt, während 45 Min. bei 0° unter Stickstoffatmosphäre gerührt und anschliessend mit 150 ml Diäthyläther versetzt. Der entstandene gelbe Niederschlag wird abfiltriert, mit wenig Diäthyläther gewaschen, darauf in 20 ml Eiswasser aufgenommen. Die mit 2 N Natronlauge auf pH 4.5 gestellte wässrige Phase wird tropfenweise mit Isopropanol (80 ml) versetzt und ca. 30 Min. gerührt.

Nach Filtration wird die 7β-[D-2-Amino-2-(3,4-dinitro-4-hydroxyphenyl)-acetylamino]-3-methoxy-ceph-3-em-4-carbonsäure zur Entfernung des organischen Lösungsmittels zweimal in wenig Wasser suspendiert und unter vermindertem Druck zur Trockne eingeengt;
DC (Silikagel, Identifikation mit Ninhydrin):Rf~0,15 (sek. Butanol-Essigsäure-Wasser 67:10:23);
$[\alpha]_D$ = +165 ±1° (in 0,1 N wässriger Salzsäure), c=0.179%).

Beispiel 8: In analoger Weise zu Beispiel 6 a) erhält man durch Behandeln von 1,7 g (2,6 mMol) 7β-[D-2-tert.-Butyloxycarbonylamino-2-(3,5-diamino-4-hydroxyphenyl)-acetylamino]-ceph-3-em-4-carbonsäurediphenylmethylester mit 1,7 ml Anisol und 8,5 ml Trifluoressigsäure in 8,5 ml absolutem Methylenchlorid nach dem beschriebenen Verfahren 7β-[D-2-Amino-2-(3,4-diamino-4-hydroxyphenyl)-acetylamino]-ceph-3-em-4-carbonsäure. Smp. ab 190° (Zersetzung);
DC (Silikagel, Identifikation mit Ninhydrin): Rf~0,05 (sek. Butanol-Essigsäure-Wasser 67:10:23);
$[\alpha]_D$ = +141 ±1° (in 0,1 N wässriger Salzsäure, c=0.181%).

Beispiel 9: a) In analoger Weise zu Beispiel 1 a) erhält man durch Behandeln von 2 g (2,77 mMol) 7β-[D-2-tert.-Butyloxycarbonylamino-2-(3-äthylsulfonylamino-4-hydroxyphenyl)-acetylamino]-ceph-3-em-4-carbonsäurediphenylmethylester mit 10 ml Trifluoressigsäure und 2 ml Anisol in 10 ml absolutem Methylenchlorid 7β-[D-2-Amino-2-(3-äthylsulfonylamino-4-hydroxyphenyl)-acetylamino]-ceph-3-em-4-carbonsäure in Form des Dihydrates. Schmelzpunkt: ab 181° unter Zersetzung.

Das Ausgangsmaterial kann wie folgt hergestellt werden:
b) 3,664 g (10 mMol) 7β-Amino-3-cephem-4-carbonsäurediphenylmethylester und 3,743 g (10 mMol) D-2-tert.-Butyloxycarbonylamino-2-(3-äthylsulfonylamino-4-hydroxyphenyl)-essigsäure werden zu 80 ml Tetrahydrofuran gegeben und auf 0 -5° gekühlt. Zu der gelben Lösung werden tropfenweise 2,063 g (10 mMol) N,N'-Dicyclohexylcarbodiimid gelöst in 20 ml Tetrahydrofuran gegeben.

Man lässt eine Stunde bei 0-5° und 3 Stunden bei Raumtemperatur rühren. Die gelbe Suspension wird abgenutscht und mit THF gewaschen. Man entfernt das Lösungsmittel bei 30° im Vakuum und erhält einen gelben, schaumigen Rückstand, den man mit 500 ml Essigsäureäthylester versetzt. Anschliessend wird die organische Phase 4 mal mit je 60 ml Eiswasser gewaschen und einmal mit 50 ml gesättigter Kochsalzlösung geschüttelt. Die Essigsäureäthylesterphase wird über Natriumsulfat getrocknet und filtriert. Man entfernt das Lösungsmittel bei 30° im Vakuum und erhält den 7β-[D-2-tert.-Butyloxycarbonylamino-2-(3-äthylsulfonylamino-4-hydroxyphenyl)-acetylamino]-ceph-3-em-4-carbonsäurediphenylmethylester als gelben, schaumigen Rückstand.

c) 2,823 g (10 mMol) D-2-tert.-Butyloxycarbonylamino-2-(3-Amino-4-hydroxyphenyl)-essigsäure werden zu 56 ml Methylenchlorid gegeben. Dazu tropft man während 10 Min. 5,64 ml (0,02 Mol) 87%-iges N,O-Bis-(trimethylsilyl)-acetamid und lässt eine halbe Stunde bei Raumtemperatur rühren. Anschliessend werden 1,61 ml (0,02 Mol) Pyridin zugegeben und die Mischung auf 0-5° abgekühlt. Man gibt 1,89 ml (0,02 Mol) Aethansulfochlorid hinzu und hält die Mischung eine halbe Stunde lang auf 0° und anschliessend nach Erwärmen zwei Stunden lang auf Raumtemperatur. Man dampft das Lösungsmittel ab, versetzt den Rückstand mit Essigsäureäthylester und wäscht anschliessend zwei Mal mit verdünnter Salzsäurelösung. Die Essigsäureäthylesterphase wird mehrmals mit verdünnter 5%-iger Natriumhydrogencarbonatlösung extrahiert. Man vereinigt die wässrigen Phasen und stellt mit ca. 25 ml 2 N Salzsäure auf pH~2. Das so ausgefällte Produkt wird mehrmals mit Essigsäureäthylester extrahiert. Die organischen Phasen werden vereinigt, mit gesättigter Kochsalzlösung gewaschen, über Natriumsulfat getrocknet, filtriert und eingeengt. Man erhält D-2-tert.-Butyloxycarbonylamino-2-(3-äthylsulfonylamino-4-hydroxyphenyl)-essigsäure als beigerosaroten Schaum.

DC (Silikagel, Identifikation mit Jod): Rf ~ 0.78 (sek. Butanol-Essigsäure-Wasser 67:10:23).

Beispiel 10: a) In analoger Weise zu Beispiel 1 a) erhält man durch Behandeln von 3,4 g (4.5 mMol) 7β-[D-2-tert.-Butyloxycarbonylamino-2-(3-äthylsulfonylamino-4-hydroxyphenyl)-acetylamino]-3-chlor-ceph-3-em-4-carbonsäurediphenylmethylester mit 17 ml Trifluoressigsäure und 3.4 ml Anisol in 17 ml absolutem Methylenchlorid nach dem beschriebenen Verfahren 7β-[D-2-Amino-2-(3-äthylsulfonylamino-4-hydroxyphenyl)-acetylamino]-3-chlor-ceph-3-em-4-carbonsäure in Form des 1.5 Hydrates. Smp. ab 165° unter Zersetzung;
DC (Silikagel, Entwickeln mit Ninhydrin): Rf~0,34 (System: sek.-Butanol-Essigsäure-Wasser 67:10:23);
$[\alpha]_D$ = +95 ±1° (0,1 N wässrige Salzsäure, c=1.163%).

b) In analoger Weise zu Beispiel 9 b) erhält man aus 3,74 g (10 mMol) D-2-tert.-Butyloxycarbonylamino-2-(3-äthylsulfonylamino-4-hydroxyphenyl)-essigsäure und 4,0 g (10 mMol) 7β-Amino-3-chlor-ceph-3-em-4-carbonsäurediphenylmethylester in Gegenwart von 2,06 g (10 mMol) N,N'-Dicyclohexylcarbodiimid in Tetrahydrofuran nach dem beschriebenen Verfahren 7β-[D-2-tert.-Butyloxycarbonylamino-2-(3-äthylsulfonylamino-4-hydroxyphenyl)-acetylamino]-3-chlor-ceph-3-em-4-carbonsäurediphenylmethylester.

Beispiel 11: a) In analoger Weise zu Beispiel 1 a) erhält man durch Behandeln von 2,74 g (3,6 mMol) 7β-[D-2-tert.-Butyloxycarbonylamino-2-(3-äthylsulfonylamino-4-hydroxyphenyl)-acetylamino]-3-methoxy-ceph-3-em-4-carbonsäurediphenylmethylester mit 14 ml Trifluoressigsäure und 2,7 ml Anisol in 14 ml absolutem Methylenchlorid nach dem beschriebenen Verfahren 7β-[D-2-Amino-2-(3-äthylsulfonylamino-4-hydroxyphenyl)-acetylamino]-3-methoxy-ceph-3-em-4-carbonsäure in der Form des Monohydrates. Smp. ab 190° unter Zersetzung;
DC (Silikagel, Entwickeln mit Ninhydrin): Rf~0,32 (System: sek.-Butanol-Essigsäure-Wasser 67:10:23);
UV-Spektrum (in 0,1 N wässriger Salzsäure): 279 nm ($\mathcal{E}$= 8250);
$[\alpha]_D$ = +138 ±1° (0,1 N wässrige Salzsäure, c = 0.967%).

b) In analoger Weise zu Beispiel 9 b) erhält man aus 2.28 g (6,1 mMol) D-2-tert.-Butyloxycarbonylamino-2-(3-äthylsulfonylamino-4-hydroxyphenyl)-essigsäure und 2,42 g (6,1 mMol) 7β-Amino-3-methoxy-ceph-3-em-4-carbonsäurediphenylmethylester in Gegenwart von 1,26 g (6,1 mMol) N,N'-Dicyclohexylcarbodiimid in Tetrahydrofuran nach dem beschriebenen Verfahren 7β-(D-2-tert.-Butyloxycarbonylamino-2-(3-äthyl-sulfonylamino-4-hydroxyphenyl)-acetylamino]-3-methoxy-ceph-3-em-4-carbonsäurediphenylmethylester.
DC (Silikagel, Identifikation mit Jod): Rf∼0,34 (Diäthyläther-Essigsäureäthylester 1:1).

Beispiel 12: a) In analoger Weise zu Beispiel 1 a) erhält man durch Behandeln von 1,32 g (1,88 mMol) 7β-[D-2-tert.-Butyloxycarbonylamino-2-(3-acetylamino-4-hydroxyphenyl)-acetylamino]-3-methoxy-ceph-3-em-4-carbonsäurediphenylmethylester mit 6,60 ml Trifluoressigsäure und 1,32 ml Anisol in 6,60 ml absolutem Methylenchlorid 7β-[D-2-Amino-2-(3-acetylamino-4-hydroxyphenyl)-acetylamino]-3-methoxy-ceph-3-em-4-carbonsäure in Form eines Monohydrates. Smp. ab 149° unter Zersetzung; DC (Silikagel, Identifikation mit Ninhydrin): Rf∼0,22 (sek. Butanol-Essigsäure-Wasser 67:10:23);
$[\alpha]_D$ = +156 ±1° (0,1 N wässrige Salzsäure , c = 0,900%).

Das Ausgangsmaterial kann wie folgt hergestellt werden:
b) 2,38 g (5,5 mMol) des Hydrochlorids des 7β-Amino-3-methoxy-ceph-3-em-4-carbonsäurediphenylmethylesters, 1,78 g (5,5 mMol) D-2-tert.-Butyloxycarbonylamino-2-(3-acetylamino-4-hydroxyphenyl)-essigsäure und 0,64 ml (5,5 mMol) 2,6-Lutidin werden zu 80 ml Tetrahydrofuran gegeben und auf 0° gekühlt. Zu der weissen Suspension werden tropfenweise 1,13 g (5,5 mMol) N,N'-Dicyclohexylcarbodiimid gelöst in 10 ml Tetrahydrofuran gegeben. Man lässt eine Stunde bei Raumtemperatur rühren. Die beige Suspension wird abgenutscht und mit THF gewaschen. Man entfernt das Lösungsmittel bei 30° im Vakuum und erhält einen gelben, schaumigen Rückstand, den man mit 250 ml Essigsäureäthylester versetzt. Anschliessend wird mit 25 ml 5%-iger Natriumhydrogencarbonatlösung und

Pufferlösung (pH=2,06) mehrmals geschüttelt. Die Essigsäureäthylesterphase wird mehrmals mit gesättigter Kochsalzlösung gewaschen,über Natriumsulfat getrocknet und abfiltriert. Man entfernt das Lösungsmittel bei 30° im Vakuum und erhält den 7β-[D-2-tert.-Butyloxycarbonylamino-2-(3-acetylamino-4-hydroxyphenyl)-acetylamino]-3-methoxy-ceph-3-em-4-carbonsäurediphenylmethylester.
DC (Silikagel, Identifikation mit Jod): Rf~0,39 (Essigsäureäthylester).

c) 4,23 g (15 mMol) D-2-tert.-Butyloxycarbonylamino-2-(3-Amino-4-hydroxyphenyl)-essigsäure werden zu 85 ml Methylenchlorid gegeben. Dazu tropft man 7,35 ml (30 mMol) 87%-iges N,O-Bis-(trimethylsilyl)-acetamid hinzu und lässt eine halbe Stunde bei Raumtemperatur rühren. Anschliessend werden 2,42 ml (30 mMol) Pyridin zugegeben und die dunkelgelbe Lösung auf 0-5° abgekühlt. Man gibt 2,13 ml (30 mMol) Acetylchlorid hinzu und hält die Mischung eine halbe Stunde lang auf 0° und anschliessend nach Erwärmen zwei Stunden lang auf Raumtemperatur. Man dampft das Lösungsmittel ab, versetzt den Rückstand mit Essigsäureäthylester und wäscht anschliessend zweimal mit verdünnter Salzsäurelösung. Die organische Phase wird mehrmals mit 5%-iger Natriumhydrogencarbonatlösung extrahiert. Man vereinigt die wässrigen Phasen und stellt mit ca. 30 ml 2 N Salzsäure auf pH~2. Man versetzt die wässrige Phase mit Essigsäureäthylester. Die organische Phase wird anschliessend mit gesättigter Kochsalzlösung gewaschen, mit Natriumsulfat getrocknet und eingeengt. Man erhält D-2-tert.-Butyloxycarbonylamino-2-(3-acetylamino-4-hydroxyphenyl)-essigsäure als gelbes Pulver.
DC (Silikagel, Identifikation mit Jod) Rf~0,75 (sek. Butanol-Essigsäure-Wasser 67:10:23).

Beispiel 13: a) Eine auf 0° gekühlte Lösung von 4,1 g (4,9 mMol) 7β-[D-2-tert.-Butyloxycarbonylamino-2-(3,5-bis-(methylsulfonylamino)-4-hydroxyphenyl)-acetylamino]-ceph-3-em-4-carbonsäurediphenylmethylester und 4,1 ml Anisol in 20 ml absolutem Methylenchlorid wird mit 20 ml kalter Trifluoressigsäure versetzt, während einer Stunde bei 0° unter Stickstoffatmosphäre gerührt und anschliessend mit 300 ml Diäthyläther bei 0° versetzt. Der beigefarbene Niederschlag wird abfiltriert, mit etwas Diäthyläther gewaschen und unter vermindertem Druck getrocknet. Man löst das so erhaltene Essigsäuresalz der 7β-[D-2-tert.-Butyloxycarbonylamino-2-(3,5-bis-(methylsulfonylamino)-4-hydroxyphenyl)-acetylamino]-ceph-3-em-4-carbonsäure in 30 ml Essigsäure und extrahiert mit 4 mal 15 ml Essigsäureäthylester. Man stellt die saure wässrige Phase (pH ca. 2) durch tropfenweise Zugabe von 2N Natriumhydroxidlösung auf pH 4,8, konzentriert im Rotationsverdampfer auf ca. 20 ml und versetzt bei 0° mit 60ml Isopropanol. Der gebildete Niederschlag wird abfiltriert, und mit je 20 ml Wasser-Isopropanol (1:4) und mit Isopropanol gewaschen. Das Rohprodukt wird anschliessend säulenchromatographisch (Kieselgel: Opti-UP $C_{12}$ der Firma Antec) mit Wasser-Acetonitril (19:1) als Eluiermittel gereinigt, woraus nach Entfernen des Lösungsmittels und nach Trocknen (16 Stunden bei Raumtemperatur und 0,05 Torr) die 7β-[D-2-Amino-2-(3,5-bis-(methylsulfonylamino)-4-hydroxyphenyl)-acetylamino]-3-methoxy-ceph-3-em-4-carbonsäure in Form eines Dihydrates erhalten wird.

Smp. ab 150° (unter Zersetzung), DC (Silicagel, Entwickeln mit Ninhydrin) $R_f$: ca. 0,18 (System: sek.Butanol-Essigsäure-Wasser 67:10:23), UV-Spektrum (in 0,1 N wässriger Salzsäure): 281 nm ($\mathcal{E}$= 8120), $[\alpha]_D$ = +107±1° (in 0,1 N wässriger Salzsäure, c = 1,054%).

Das Ausgangsmaterial kann wie folgt erhalten werden:

b) Eine auf 0-5° gekühlte Suspension von 4,76 g (12 mMol) 7β-Amino-3-methoxy-ceph-3-em-4-carbonsäurediphenylmethylester und 5,44 g (12 mMol) D-2-tert.-Butyloxycarbonylamino-2-[3,5-bis-(methylsulfonylamino)-4-hydroxyphenyl]-essigsäure in 50 ml Tetrahydrofuran wird tropfenweise mit einer Lösung von 2,476 g (12 mMol) N,N'-Dicyclohexylcarbodiimid in 20 ml Tetrahydrofuran versetzt. Nach einer Reaktionszeit von einer Stunde bei 0-5° und 3 Stunden bei Raumtemperatur wird der gebildete Niederschlag von N,N'-Dicyclohexylharnstoff abfiltriert und mit wenig Tetrahydrofuran gewaschen. Nach der Entfernung des Lösungsmittels wird der gelbe Rückstand in 500 ml Essigsäureäthylester gelöst, je 4 mal mit 50 ml 5%iger Natriumhydrogencarbonatlösung (pH = 2) und gesättigter Natriumchloridlösung gewaschen. Nach Trocknen der organischen Phase über Natriumsulfat und Entfernen des Lösungsmittels am Rotationsverdampfer wird das Rohprodukt säulenchromatographisch (Kieselgel) mit Diäthyläther-Essigsäureäthylester (2:1) als Eluiermittel gereinigt, woraus der 7β-[D-2-tert.-Butyloxycarbonylamino-2-(3,5-bis-(methylsulfonylamino)-4-hydroxyphenyl)-acetylamino]-3-methoxy-ceph-3-em-4-carbonsäurediphenylmethylester erhalten wird. DC (Silicagel, Identifikation mit Jod): $R_f$~0.28 (Essigsäureäthylester).

c) Man versetzt eine Lösung von 5,4 g (18.2 mMol) D-2-tert.Butyloxycarbonylamino-2-(3,5-diamino-4-hydroxyphenyl)-essigsäure in 100 ml absolutem Methylenchlorid unter Feuchtigkeitsausschluss und unter Rühren mit 6,7 ml (27,3 mMol) N,O-Bis-(trimethylsilyl)-acetamid und lässt eine Stunde reagieren. Nach Zugabe von 4,4 ml Pyridin und 4,24 ml (54.6 mMol) Methansulfochlorid zu der auf 0° gekühlten Lösung wird das Reaktionsgemisch 30 Min. bei 0° und 3 Stunden bei Raumtemperatur gerührt. Darauf wird das Reaktionsgemisch mit 260 ml Essigsäureäthylester verdünnt,und je dreimal mit 30 ml Eiswasser und gesättigter Natriumchloridlösung gewaschen. Die organische Phase wird über Natriumsulfat getrocknet und am Rotationsverdampfer vom Lösungsmittel befreit. Das Rohprodukt wird säulenchromatographisch (Silicagel) mit Diäthyläther-Essigsäureäthylester 9:1 als Eluiermittel gereinigt, woraus D-2-tert.Butyloxycarbonylamino-2-(3,5-bis-(methylsulfonylamino-4-hydroxyphenyl)-essigsäure als amorphes gelbes Pulver erhalten wird.
DC (Silicagel, Identifikation mit Ninhydrin): $R_f$~0.68 (sek. Butanol-Essigsäure-Wasser 67:10:23), $[\alpha]_D$ = 83±1° (abs. Aethanol, c = 0.60%).

d) Eine Lösung von 11,5 g (32.2 mMol) D-2-tert.Butyloxycarbonylamino-2-(3,5-dinitro-4-hydroxyphenyl)-essigsäure in 200 ml Methanol wird in Gegenwart von 1,5 g Palladium-Kohle (10%-ig) als Katalysator hydriert. Nach der Aufnahme der theoretischen Menge Wasserstoff wird das Reaktionsgemisch über Celite filtriert und am Rotationsverdampfer vom Lösungsmittel befreit, woraus D-2-tert.Butyloxycarbonylamino-2-(3,5-diamino-4-hydroxy-

phenyl)-essigsäure erhalten wird, die ohne weitere Reinigung im Reaktionsschritt c) eingesetzt wird.
DC (Silicagel, Identifikation mit Ninhydrin) $R_f$~0,50 (sek.-Butanol-Essigsäure-Wasser 67:10:23).

Beispiel 14: a) In analoger Weise zu Beispiel 13a) erhält man durch Behandeln von 2,3 g (2,87 mMol) 7β-[D-2-tert.-Butyloxycarbonylamino-2-(3,5-bis-(methylsulfonylamino-4-hydroxyphenyl)-acetylamino]-ceph-3-em-4-carbonsäurediphenylmethylester mit 11,5 ml Trifluoressigsäure und 2,3 ml Anisol in 11,5 ml absolutem Methylenchlorid 7β-[D-2-Amino-2-(3,5-bis-methylsulfonylamino-4-hydroxyphenyl)-acetylamino]-ceph-3-em-4-carbonsäure in Form des Dihydrates.

Smp. ab 150° (unter Zersetzung). DC (Silicagel, Identifikation mit Ninhydrin) $R_f$~0.13 (sek.Butanol-Essigsäure-Wasser 67:10:23), $[\alpha]_D = +91 \pm 1°$ (0,1 N wässerige Salzsäure, c = 0,89%).

Das Ausgangsmaterial kann wie folgt erhalten werden:

b) In analoger Weise zu Beispiel 13b erhält man aus 4,532 g (10 mMol) D-2-tert.-Butyloxycarbonylamino-2-(3,5-bis-(methylsulfonylamino)-4-hydroxyphenyl]-essigsäure und 3,654 g (10 mMol) 7β-Amino-ceph-3-em-4-carbonsäurediphenylmethylester in Gegenwart von 3,063 g (10 mMol) N,N'-Dicyclohexylcarbodiimid in Tetrahydrofuran nach dem beschriebenen Verfahren 7β-[D-2-tert.-Butyloxycarbonylamino-2-(3,5-bis-(methylsulfonylamino)-4-hydroxyphenyl)-acetylamino]-ceph-3-em-4-carbonsäurediphenylmethylester.

Beispiel 15: a) In analoger Weise zu Beispiel 13a) erhält man durch Behandeln von 4,47 g (5,35 mMol) 7β-[D-2-tert.-Butyloxycarbonylamino-2-(3,5-bis-(methylsulfonylamino)-4-hydroxyphenyl)-acetylamino]-3-chlor-ceph-3-em-4-carbonsäurediphenylmethylester mit 20 ml Trifluoressigsäure und 4 ml Anisol in 20 ml absolutem Methylenchlorid nach dem beschriebenen Verfahren 7β-[D-2-Amino-2-(3,5-bis-(methylsulfonylamino)-4-hydroxyphenyl)-acetylamino]-3-chlor-ceph-3-em-4-carbonsäure in Form des Dihydrates.

Smp. ab 150° unter Zersetzung, DC (Silicagel, Entwickeln mit Ninhydrin): $R_f$ 0,23 (System: sek.-Butanol-Essigsäure-Wasser 67:10:23), UV-Spektrum (in 0.1N wässeriger Salzsäure): 270 nm ($\varepsilon$= 7600), $[\alpha]_D$ = +62±1° (0,1N wässerige Salzsäure, c = 0,980%).

b) In analoger Weise zu Beispiel 13b) erhält man aus 4,53 g (10 mMol) D-2-tert.-Butyloxycarbonylamino-2-[3,5-bis-(methylsulfonylamino)-4-hydroxyphenyl]-essigsäure und 4,0 g (10 mMol) 7β-Amino-3-chlor-cheph-3-em-4-carbonsäurediphenylmethylester in Gegenwart von 2,06 g (10 mMol) N,N'-Dicyclohexylcarbodiimid in Tetrahydrofuran nach dem beschriebenen Verfahren 7β-[D-2-tert.-Butyloxycarbonylamino-2-(3,5-bis-(methylsulfonyl-amino-4-hydroxyphenyl)-acetylamino]-3-chlor-ceph-3-em-4-carbonsäurediphenylmethylester.
DC (Silikagel, Identifikation mit Jod): $R_f$~0.43 (Diäthyläther-Essigsäureäthylester 1:1).

Beispiel 16: Eine Suspension von 4,75 g 7β-[D-2-Amino-2-(3-methylsulfonyl-amino-4-hydroxyphenyl)-acetylamino]-3-methoxy-3-cephem-4-carbonsäure in 50 ml Wasser und 2 ml Alkohol wird unter Rühren und pH-Kontrolle bei einem pH-Wert kleiner als 7,6 langsam mit insgesamt ca. 5 ml 2N Natronlauge in Lösung gebracht. Die von wenig Restkörpern durch Filtration befreite Lösung ergibt nach Lyophilisieren 4,9 g 7β-[D-2-Amino-2-(3-methyl-sulfonylamino-4-hydroxyphenyl)-acetylamino]-3-methoxy-3-cephem-4-carbonsäure-natriumsalz; UV-Spektrum in 0.1 N HCl: 278 nm ($\varepsilon$= 7600)
DC: $R_f$~ 0.46.

Beispiel 17: Analog Beispiel 16 können hergestellt werden: 7β-[D-2-Amino-2-(3-methylsulfonylamino-4-hydroxyphenyl)-acetylamino]-3-chlor-3-cephem-4-carbonsäure-natriumsalz, UV-Spektrum in 0.1 N HCl: 235 nm (Schulter), 270 nm ($\varepsilon$= 8500) DC $R_f$~0.16;
7β-[D-2-Amino-2-(3-methylsulfonylamino-4-hydroxyphenyl)-acetylamino] -3-cephem-4-carbonsäure-natriumsalz, UV-Spektrum in 0.1 N HCl: 235 nm ($\varepsilon$= 11400) DC: $R_f$~0.19;

7β-[D-2-Amino-2-(3-amino-4-hydroxyphenyl)-acetylamino]-3-methoxy-ceph-3-em-4-carbonsäure-natriumsalz;

7β-[D-2-Amino-2-(3,5-diamino-4-hydroxyphenyl)-acetylamino]-3-methoxy-ceph-3-em-4-carbonsäure-natriumsalz, UV-Spektrum in 0.1 N HCl: 270 nm (ε= 7600) DC : $R_f$~0.05 ;

DC: Silicagel, Entwickeln mit Ninhydrin, System : sek. Butanol-Essigsäure-Wasser 67:10:23 .

<u>Beispiel 18:</u> Analog Beispiel 1-16 können folgende Verbindungen hergestellt werden:

7β-[D-2-Amino-2-(3,5-bis-(äthylsulfonylamino)-4-hydroxyphenyl)-acetylamino]-ceph-3-em-4-carbonsäure, UV-Spektrum in 0.1 N HCl : 209 nm (ε= 3900), 272 nm ( Schulter) DC : $R_f$~0.13;

7β-[D-2-Amino-2-(3,5-bis-(äthylsulfonylamino)-4-hydroxyphenyl)-acetylamino]-3-methoxy-ceph-3-em-4-carbonsäure, UV-Spektrum in 0,1 N HCl: 281 nm (ε= 8100), DC : Rf~0,18;

7β-[D-2-Amino-2-(3,5-bis-(äthylsulfonylamino)-4-hydroxyphenyl)-acetylamino]-3-chlor-ceph-3-em-4-carbonsäure, UV-Spektrum in 0.1 N HCl: 210 nm (ε= 37000), 270 nm (ε= 7500) ;

7β-[D-2-Amino-2-(3-acetylamino-4-hydroxyphenyl)-acetylamino]-ceph-3-em-4-carbonsäure, UV-Spektrum in 0.1 N HCl : 275 nm (ε= 6900) ;

7β-[D-2-Amino-2-(3-formylamino-4-hydroxyphenyl)-acetylamino]-3-methoxy-ceph-3-em-4-carbonsäure,UV Spektrum in 0.1 N HCl : 279 nm (ε= 7600) ;

7β-[D-2-Amino-2-(3-formylamino-4-hydroxyphenyl)-acetylamino]-3-chlor-ceph-3-em-4-carbonsäure, UV-Spektrum in 0.1 N HCl : 270 nm (ε= 8100) ;

7β-[D-2-Amino-2-(3-formylamino-4-hydroxyphenyl)-acetylamino]-ceph-3-em-4-carbonsäure, UV-Spektrum in 0.1 N HCl : 232 nm (ε= 11500), 270 nm (Schulter).

7β-[D-2-Amino-2-[3-(3-methylureido)-4-hydroxy-phenyl]-acetylamino]-3-methoxy-ceph-3-em-4-carbonsäure, UV-Spektrum in 0.1 N HCl : 244 nm (ε= 8500) 275 nm (Schulter) ;

7β-[D-2-Amino-2-[3-(3-methylureido)-4-hydroxy-phenyl]-acetylamino]-3-chlor-ceph-3-em-4-carbonsäure;

7β-[D-2-Amino-2-[3-(3-methylthioureido)4-hydroxy-phenyl]-acetylamino]-

ceph-3-em-4-carbonsäure;

7β-[D-2-Amino-2-[3-(3-methylthioureido)-4-hydroxy-phenyl]-acetylamino]-3-methoxy-ceph-3-em-4-carbonsäure;

7β-[D-2-Amino-2-[3-(3-methylthioureido)-4-hydroxy-phenyl]-acetylamino]-3-chlor-ceph-3-em-4-carbonsäure und ihre Salze, z.B. die Natriumsalze

DC : Dünnschichtchromatogramm auf Silicakel-Fertigplatten, System: sek. Butanol-Essigsäure-Wasser 67:10:23.

Beispiel 19: Trockenampullen oder Vials, enthaltend 0,5 g des inneren Salzes der 7β-[D-2-Amino-2-(3-methylsulfonylamino-4-hydroxyphenyl)-acetylamino]-3-methoxy-3-cephem-4-carbonsäure werden wie folgt hergestellt:

Zusammensetzung (für 1 Ampulle oder Vial)

| | |
|---|---|
| Inneres Salz der 7β-[D-2-Amino-2-(3-methylsulfonylamino-4-hydroxyphenyl)-acetylamino]-3-methoxy-3-cephem-4-carbonsäure | 0,5 g |
| Mannit | 0,05 g |

Eine sterile wässrige Lösung des inneren Salzes der 7β-[D-2-Amino-2-(3-methylsulfonylamino-4-hydroxyphenyl)-acetylamino]-3-methoxy-3-cephem-4-carbonsäure und des Mannits wird unter aseptischen Bedingungen in 5 ml.-Ampullen oder 5 ml.-Vials der Gefriertrocknung unterworfen und die Ampullen bzw. Vials verschlossen und geprüft.

Beispiel 20: Kapseln, enthaltend 0,25 g des inneren Salzes der 7β-[D-2-Amino-2-(3-methylsulfonylamino-4-hydroxyphenyl)-acetylamino]-3-methoxy-3-cephem-4-carbonsäure, werden wie folgt hergestellt:

Zusammensetzung (für 1000 Kapseln):

| | |
|---|---|
| Inneres Salz der 7β-[D-2-Amino-2-(3-methylsulfonylamino-4-hydroxyphenyl)-acetylamino]-3-methoxy-3-cephem-4-carbonsäure | 250.000 g |
| Maisstärke | 50.000 g |
| Polyvinylpyrrolidon | 15.000 g |
| Magnesiumstearat | 5.000 g |
| Aethanol | q.s. |

Das innere Salz der 7β-[D-2-Amino-2-(3-methylsulfonylamino-4-hydroxy-phenyl)-acetylamino]-3-methoxy-3-cephem-4-carbonsäure und die Maisstärke werden vermischt und mit einer Lösung des Polyvinylpyrrolidons in 50 g Aethanol befeuchtet. Die feuchte Masse wird durch ein Sieb mit einer Maschenweite von 3 mm gedrückt und bei 45° getrocknet. Das trockene Granulat wird durch ein Sieb mit einer Maschenweite von 1 mm getrieben und mit 5 g Magnesiumstearat vermischt. Die Mischung wird in Portionen von 0,320 g in Steckkapseln der Grösse 0 abgefüllt.

Patentansprüche (für alle benannten Länder ausser Oesterreich)

1. Verbindungen der Formel

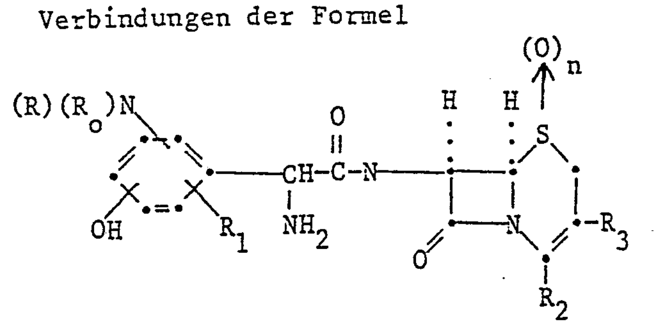

(I)

worin der Index n für 0 oder 1 steht, R Wasserstoff oder eine Acylgruppe, $R_o$ Wasserstoff oder Niederalkyl, $R_1$ Wasserstoff oder eine Gruppe der Teilformel $(R)(R_o)N-$, $R_2$ Carboxyl oder geschütztes Carboxyl und $R_3$ Wasserstoff, Halogen mit Ordnungszahl bis 35 oder Niederalkoxy bedeuten, sowie Hydrate und Salze von Verbindungen der Formel I.

2. Verbindungen der Formel I gemäss Anspruch 1, worin der Index n für 0 steht, R Wasserstoff oder eine Acylgruppe, z.B. Niederalkanoyl, Halogenniederalkanoyl, gegebenenfalls durch Halogen, Niederalkoxy oder Nitro substituiertes Benzoyl, tert.-Niederalkoxycarbonyl, Arylmethoxycarbonyl mit einem oder zwei Arylresten, die gegebenenfalls durch Niederalkyl, Niederalkoxy, Hydroxy, Halogen, und/oder Nitro mono- oder polysubstituiertes Phenyl darstellen, Aroylmethoxycarbonyl, worin die Aroylgruppe gegebenenfalls durch Halogen substituiertes Benzoyl darstellt, 2-Halogenniederalkoxycarbonyl, Carbamoyl, N-Niederalkylcarbamoyl, N,N-Diniederalkylcarbamoyl, Thiocarbamoyl, N-Niederalkylthiocarbamoyl, N,N-Diniederalkylthiocarbamoyl, Niederalkylsulfonyl, Arylsulfonyl, Sulfamoyl, N-Niederalkylsulfamoyl, oder N,N-Diniederalkylsulfamoyl, $R_o$ Wasserstoff oder Niederalkyl, $R_1$ Wasserstoff oder eine Gruppe der Teilformel $(R)(R_o)N-$, worin R und $R_o$ die vorstehend genannten Bedeutungen haben, $R_2$ Carboxyl oder Niederalkanoyl-methoxycarbonyl und $R_3$ Wasserstoff, Fluor, Chlor, Brom, Methoxy, Aethoxy, Isopropyloxy oder tert.-Butyloxy bedeuten, sowie Hydrate und Salze von Verbindungen (I), insbesondere pharmazeutisch verwendbare Salze von Verbindungen der Formel I.

3. Verbindungen der Formel I gemäss Anspruch 1, worin der Index n für 0 steht, R Wasserstoff, Niederalkanoyl mit bis zu 4 Kohlenstoffatomen, z.B. Formyl, Acetyl oder Propionyl, Halogenniederalkanoyl mit bis zu 4 Kohlenstoffatomen, z.B. 2-Chlor-, 2-Brom-, 2-Jod-, 2,2,2-Trifluor- oder 2,2,2-Trichloracetyl, gegebenenfalls durch Halogen, Niederalkoxy, z.B. Methoxy, oder Nitro substituiertes Benzoyl, z.B. 4-Chlorbenzoyl, 4-Methoxybenzoyl oder 4-Nitrobenzoyl, tert.-Niederalkoxycarbonyl, z.B. tert.-Butyloxycarbonyl, gegebenenfalls durch Nitro substituiertes Benzyloxycarbonyl, z.B. Benzyloxycarbonyl oder 4-Nitrobenzyloxycarbonyl, gegebenenfalls durch Niederalkoxy, z.B. Methoxy, substituiertes Diphenylmethoxycarbonyl, z.B. Benzhydryloxycarbonyl oder Bis-(4-methoxyphenyl)-methoxycarbonyl, Phenacyloxycarbonyl, 2-Halogenniederalkoxycarbonyl mit bis zu 4 Kohlenstoffatomen, z.B. 2,2,2-Trichlor-, 2-Chlor-, 2-Brom- oder 2-Jodäthoxycarbonyl, Carbamoyl, N-Niederalkylcarbamoyl, z.B. N-Methylcarbamoyl, N,N-Diniederalkylcarbamoyl, z.B. N,N-Dimethylcarbamoyl, Thiocarbamoyl, N-Niederalkylthiocarbamoyl, z.B. N-Methylthiocarbamoyl, N,N-Diniederalkylthiocarbamoyl, z.B. N,N-Dimethylthiocarbamoyl, Niederalkylsulfonyl, z.B. Methylsulfonyl oder Aethylsulfonyl, Arylsulfonyl, z.B. Phenylsulfonyl oder Toluylsulfonyl, Sulfamoyl, N-Niederalkylsulfamoyl, z.B. N-Methylsulfamoyl, oder N,N-Diniederalkylsulfamoyl, z.B. N,N-Dimethylsulfamoyl, $R_o$ Wasserstoff oder Niederalkyl, z.B. Methyl oder Aethyl, $R_1$ Wasserstoff oder eine Gruppe der Teilformel $(R)(R_o)N-$, worin R und $R_o$ die vorstehend genannten Bedeutungen haben, $R_2$ Carboxyl und $R_3$ Wasserstoff, Chlor oder Methoxy bedeuten, sowie Hydrate und Salze von Verbindungen (I), insbesondere pharmazeutisch verwendbare Salze von solchen Verbindungen der Formel I.

4. Verbindungen der Formel I gemäss Anspruch 1, worin der Index n für 0 steht, R Wasserstoff, Niederalkanoyl mit bis zu 4 Kohlenstoffatomen, z.B. Acetyl, Niederalkylsulfonyl mit bis zu 4 Kohlenstoffatomen, z.B. Methylsulfonyl oder Aethylsulfonyl, $R_o$ Wasserstoff, $R_1$ Wasserstoff oder eine Gruppe der Teilformel $(R)(R_o)N-$, worin R und $R_o$ die vorstehend genannten Bedeutungen haben, $R_2$ Carboxyl und

$R_3$ Wasserstoff, Chlor oder Methoxy bedeuten, sowie Salze von Verbindungen (I), insbesondere pharmazeutisch verwendbare Salze von Verbindungen der Formel I.

5. Verbindungen der Formel I gemäss Anspruch 1, worin der Index n für 0 steht, R Wasserstoff, Niederalkanoyl mit bis zu 4 Kohlenstoffatomen, z.B. Methylsulfonyl oder Aethylsulfonyl, $R_o$ Wasserstoff, $R_1$ Wasserstoff oder eine Gruppe der Teilformel $(R)(R_o)N-$, worin R und $R_o$ die vorstehend genannten Bedeutungen haben, $R_2$ Carboxyl und $R_3$ Wasserstoff, Chlor oder Methoxy bedeuten, und worin die beiden Gruppen der Teilformel $(R)(R_o)N-$ die 3- und 5-Stellung (meta) und die Hydroxygruppe die 4-Stellung (para) am Phenylring einnehmen, sowie Hydrate und Salze von solchen Verbindungen, insbesondere pharmazeutisch verwendbare Salze von solchen Verbindungen der Formel I.

6. 7β-[D-2-Amino-2-(3-methylsulfonylamino-4-hydroxyphenyl)-acetylamino]-3-methoxy-ceph-3-em-4-carbonsäure.

7. 7β-[D-2-Amino-2-(3-methylsulfonylamino-4-hydroxyphenyl)-acetylamino]-ceph-3-em-4-carbonsäure.

8. 7β-[D-2-Amino-2-(3-methylsulfonylamino-4-hydroxyphenyl)-acetylamino]-3-chlor-ceph-3-em-4-carbonsäure.

9. 7β-[D-2-Amino-2-(3-amino-4-hydroxyphenyl)-acetylamino]-3-methoxy-ceph-3-em-4-carbonsäure.

10. 7β-[D-2-Amino-2-(3-äthylsulfonylamino-4-hydroxyphenyl)-acetylamino]-ceph-3-em-4-carbonsäure.

11. 7β-[D-2-Amino-2-(3-äthylsulfonylamino-4-hydroxyphenyl)-acetylamino]-3-methoxy-ceph-3-em-4-carbonsäure.

12. 7β-[D-2-Amino-2-(3-äthylsulfonylamino-4-hydroxyphenyl)-acetyl-amino]-3-chlor-ceph-3-em-4-carbonsäure.

13. 7β-[D-2-Amino-2-(3-acetylamino-4-hydroxyphenyl)-acetylamino]-3-methoxy-ceph-3-em-4-carbonsäure.

14. Pharmazeutische Präparate enthaltend Verbindungen der Formel I, Hydrate oder pharmazeutisch verwendbare Salze von Verbindungen der Formel I mit salzbildenden Gruppen.

15. Verbindungen der Formel I, Hydrate oder pharmazeutisch verwendbare Salze von Verbindungen der Formel I zur Anwendung in einem Verfahren zur therapeutischen Behandlung des menschlichen oder tierischen Körpers.

16. Verbindungen der Formel I, Hydrate oder pharmazeutisch verwendbare Salze von Verbindungen der Formel I als antibiotisch wirksame Mittel.

17. Verwendung von Verbindungen der Formel I, Hydrate oder pharmazeutisch verwendbaren Salzen von Verbindungen der Formel I zur Herstellung von pharmazeutischen Präparaten.

18. Verfahren zur Herstellung von Verbindungen der Formel I und Salzen von Verbindungen der Formel I, die eine salzbildende Gruppe aufweisen, dadurch gekennzeichnet, dass man

a) in einer Verbindung der Formel

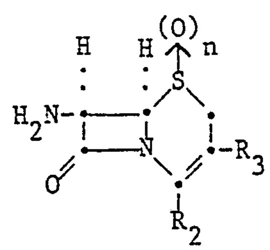

(II),

worin der Index n für 0 oder 1 steht und die 7β-Aminogruppe gegebenenfalls durch eine die Acylierung erlaubende Gruppe geschützt ist,

und $R_2$ und $R_3$ die unter Formel I genannten Bedeutungen haben, die 7β-Aminogruppe durch Umsetzung mit einem den Acylrest einer Carbonsäure der Formel

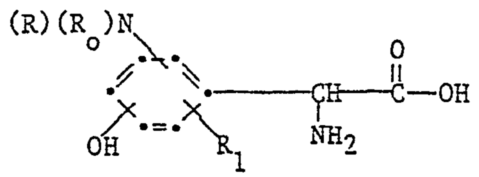

(III)

einführenden Acylierungsmittel, worin R, $R_o$ und $R_1$ die unter Formel I genannten Bedeutungen haben, und worin die Hydroxygruppe am Phenylring und/oder die Aminogruppe(n) gegebenenfalls in geschützter Form vorliegen und weitere vorhandene funktionelle Gruppen gegebenenfalls geschützt sind, acyliert, oder

b) eine 2-Cephemverbindung der Formel

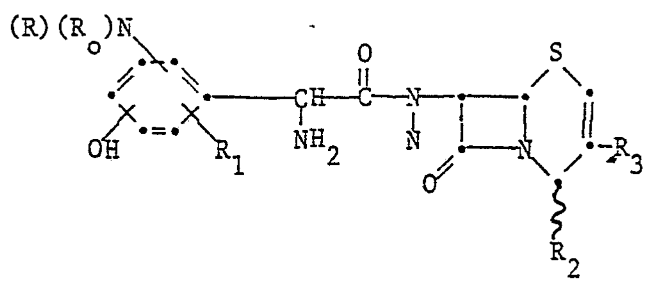

(IV),

worin R, $R_o$, $R_1$, $R_2$ und $R_3$ die unter Formel I genannten Bedeutungen haben und worin die Hydroxygruppe am Phenylring und/oder die Aminogruppe(n) gegebenenfalls in geschützter Form vorliegen und weitere vorhandene funktionelle Gruppen gegebenenfalls geschützt sind, isomerisiert, oder

c) in einer Verbindung der Formel

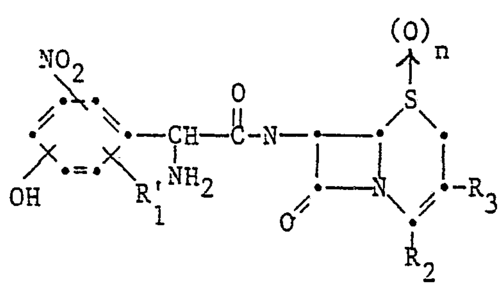

(V),

worin der Index n für 0 oder 1 steht und $R_1'$ Nitro oder den Rest $R_1$ darstellt, welcher die unter Formel I genannten Bedeutungen hat, $R_2$ und $R_3$ die unter Formel I genannten Bedeutungen haben, und worin die Hydroxygruppe am Phenylring und/oder die Aminogruppe(n) gegebenenfalls in geschützter Form vorliegen und weitere vorhandene funktionelle Gruppen gegebenenfalls geschützt sind, die Nitrogruppe(n) am Phenylring durch Umsetzen mit einem Nitrogruppen in Aminogruppen überführenden Mittel reduziert, und, wenn erwünscht, in einer erhältlichen Verbindung eine freie Aminogruppe der Teilformel (R)($R_o$)N-, worin R und $R_o$ Wasserstoff bedeuten, in eine substituierte Aminogruppe überführt und/oder, wenn erwünscht, eine erhältliche Verbindung, worin n 0 bedeutet, in eine Verbindung überführt, worin n 1 bedeutet, und/oder eine erhältliche Verbindung, worin n 1 bedeutet, in eine Verbindung überführt, worin n 0 bedeutet, und/oder in einer erhältlichen Verbindung in geschützter Form vorliegende funktionelle Gruppen in die freien funktionellen Gruppen überführt, und/oder ein erhältliches Salz in die freie Verbindung oder in ein anderes Salz überführt, und/oder eine erhältliche Verbindung mit einer salzbildenden Gruppe in ein Salz überführt, und/oder ein erhältliches Gemisch von isomeren Verbindungen in die einzelnen Isomeren auftrennt.

19. Die nach dem Verfahren gemäss Anspruch 18 herstellbaren Verbindungen.

20. Verbindungen der Formel

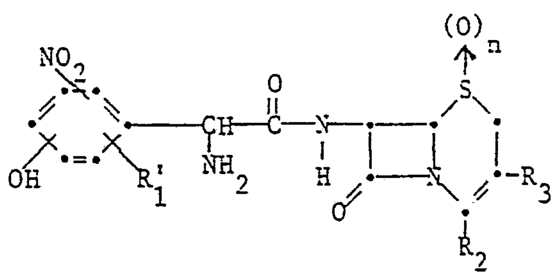

(V),

worin der Index n für 0 oder 1 steht und $R_1'$ Wasserstoff, Nitro oder eine Gruppe der Teilformel (R)($R_o$)N- darstellt, worin R Wasserstoff oder eine Acylgruppe und $R_o$ Wasserstoff oder Niederalkyl bedeuten,

$R_2$ Carboxyl oder geschütztes Carboxyl und $R_3$ Wasserstoff, Halogen mit Ordnungszahl bis 35 oder Niederalkoxy bedeuten, sowie Hydrate und Salze von Verbindungen der Formel V.

21. 7β-[D-2-Amino-2-(3-nitro-4-hydroxyphenyl)-acetylamino]-3-methoxy-ceph-3-em-4-carbonsäure.

22. Verfahren zur Herstellung von Verbindungen der Formel V und Salzen von Verbindungen der Formel V, die eine salzbildende Gruppe aufweisen, dadurch gekennzeichnet, dass man in einer Verbindung der Formel

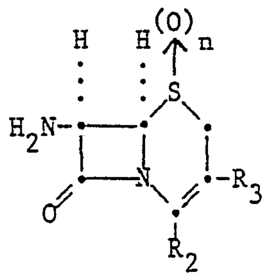

(II),

worin der Index n für 0 oder 1 steht und die 7β-Aminogruppe gegebenenfalls durch eine die Acylierung erlaubende Gruppe geschützt ist, und $R_2$ und $R_3$ die unter Formel V genannten Bedeutungen haben, die 7β-Aminogruppe durch Umsetzung mit einem den Acylrest einer Carbonsäure der Formel

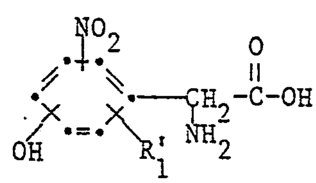

(VI)

einführenden Acylierungsmittel, worin die Hydroxygruppe am Phenylring und/oder die D-2-Aminogruppe gegebenenfalls in geschützter Form vorliegen und worin weitere vorhandene funktionelle Gruppen gegebenenfalls geschützt sind, acyliert und, wenn erwünscht, eine erhältliche Verbindung, worin der Index n 1 bedeutet, in eine Verbindung umwandelt, worin der Index n 0 bedeutet und/oder, wenn erwünscht, in einer erhältlichen Verbindung in geschützter Form vorliegende funktionelle Gruppen in freie funktionelle Gruppen und/oder ein erhältliches Salz

in die freie Verbindung oder in ein anderes Salz überführt und/oder eine erhältliche freie Verbindung mit einer salzbildenden Gruppe in ein Salz überführt und/oder ein erhältliches Gemisch von isomeren Verbindungen in die einzelnen Isomeren auftrennt.

Patentansprüche (für Oesterreich)

1. Verfahren zur Herstellung von Verbindungen der Formel

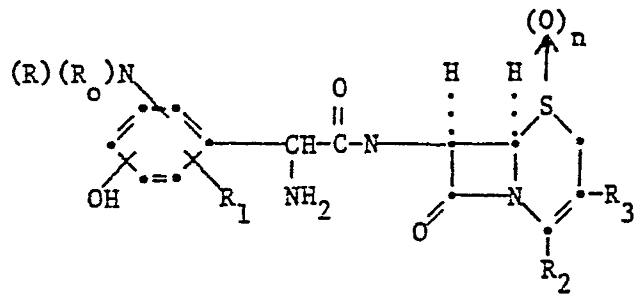

(I)

worin der Index n für 0 oder 1 steht, R Wasserstoff oder eine Acylgruppe, $R_o$ Wasserstoff oder Niederalkyl, $R_1$ Wasserstoff oder eine Gruppe der Teilformel $(R)(R_o)N-$, $R_2$ Carboxyl oder geschütztes Carboxyl und $R_3$ Wasserstoff, Halogen mit Ordnungszahl bis 35 oder Niederalkoxy bedeuten, sowie Hydrate und Salze von Verbindungen der Formel I bedeuten, sowie Hydraten und Salzen von Verbindungen der Formel I, dadurch gekennzeichnet, dass man

a) in einer Verbindung der Formel

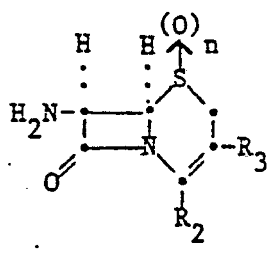

(II),

worin der Index n für 0 oder 1 steht und die 7β-Aminogruppe gegebenenfalls durch eine die Acylierung erlaubende Gruppe geschützt ist, und $R_2$ und $R_3$ die unter Formel I genannten Bedeutungen haben, die 7β-Aminogruppe durch Umsetzung mit einem den Acylrest einer Carbonsäure der Formel

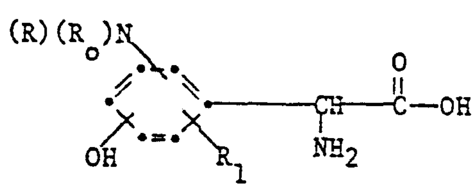

(III)

einführenden Acylierungsmittel, worin R, $R_o$ und $R_1$ die unter Formel I genannten Bedeutungen haben, und worin die Hydroxygruppe am Phenylring und/oder die Aminogruppe(n) gegebenenfalls in geschützter Form vorliegen und weitere vorhandene funktionelle Gruppen gegebenenfalls geschützt sind, acyliert, oder

b) eine 2-Cephemverbindung der Formel

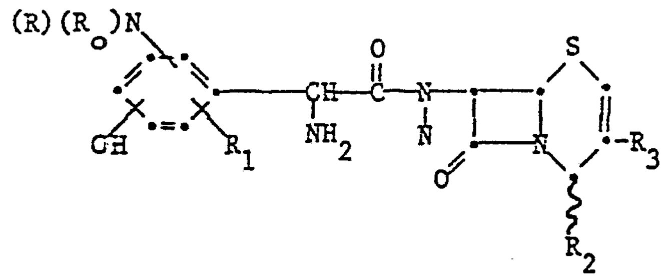

(IV),

worin R, $R_o$, $R_1$, $R_2$ und $R_3$ die unter Formel I genannten Bedeutungen haben und worin die Hydroxygruppe am Phenylring und/oder die Aminogruppe(n) gegebenenfalls in geschützter Form vorliegen und weitere vorhandene funktionelle Gruppen gegebenenfalls geschützt sind, isomerisiert, oder

c) in einer Verbindung der Formel

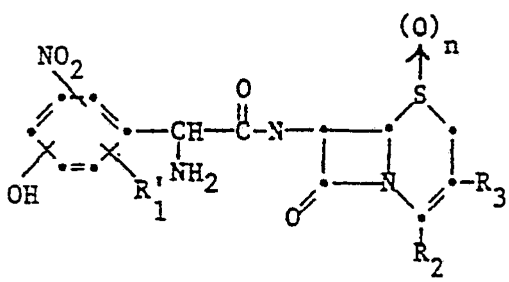

(V),

worin der Index n für 0 oder 1 steht und $R_1'$ Nitro oder den Rest $R_1$ darstellt, welcher die unter Formel I genannten Bedeutungen hat, $R_2$ und $R_3$ die unter Formel I genannten Bedeutungen haben, und worin die Hydroxygruppe am Phenylring und/oder die Aminogruppe(n) gegebenenfalls in geschützter Form vorliegen und weitere vorhandene funktionelle Gruppen gegebenenfalls geschützt sind, die Nitrogruppe(n) am Phenylring durch Umsetzen mit einem Nitrogruppen in Aminogruppen überführenden Mittel reduziert, und, wenn erwünscht, in einer erhältlichen

Verbindung eine freie Aminogruppe der Teilformel (R)($R_o$)N-, worin R und $R_o$ Wasserstoff bedeuten, in eine substituierte Aminogruppe überführt und/oder, wenn erwünscht, eine erhältliche Verbindung, worin n 0 bedeutet, in eine Verbindung überführt, worin n 1 bedeutet, und/oder eine erhältliche Verbindung, worin n 1 bedeutet, in eine Verbindung überführt, worin n 0 bedeutet, und/oder in einer erhältlichen Verbindung in geschützter Form vorliegende funktionelle Gruppen in die freien funktionellen Gruppen überführt, und/oder ein erhältliches Salz in die freie Verbindung oder in ein anderes Salz überführt, und/oder eine erhältliche Verbindung mit einer salzbildenden Gruppe in ein Salz überführt, und/oder ein erhältliches Gemisch von isomeren Verbindungen in die einzelnen Isomeren auftrennt.

2. Verfahren nach Anspruch 1 zur Herstellung von Verbindungen der Formel I, worin der Index n für 0 steht, R Wasserstoff oder eine Acylgruppe, z.B. Niederalkanoyl, Halogenniederalkanoyl, gegebenenfalls durch Halogen, Niederalkoxy oder Nitro substituiertes Benzoyl, tert.-Niederalkoxycarbonyl, Arylmethoxycarbonyl mit einem oder zwei Arylresten, die gegebenenfalls durch Niederalkyl, Niederalkoxy, Hydroxy, Halogen, und/oder Nitro mono- oder polysubstituiertes Phenyl darstellen, Aroylmethoxycarbonyl, worin die Aroylgruppe gegebenenfalls durch Halogen substituiertes Benzoyl darstellt, 2-Halogenniederalkoxycarbonyl, Carbamoyl, N-Niederalkylcarbamoyl, N,N-Diniederalkylcarbamoyl, Thiocarbamoyl, N-Niederalkylthiocarbamoyl, N,N-Diniederalkylthiocarbamoyl, Niederalkylsulfonyl, Arylsulfonyl, Sulfamoyl, N-Niederalkylsulfamoyl, oder N,N-Diniederalkylsulfamoyl, $R_o$ Wasserstoff oder Niederalkyl, $R_1$ Wasserstoff oder eine Gruppe der Teilformel (R)($R_o$)N-, worin R und $R_o$ die vorstehend genannten Bedeutungen haben, $R_2$ Carboxyl oder Niederalkanoyl-methoxycarbonyl und $R_3$ Wasserstoff, Fluor, Chlor, Brom, Methoxy, Aethoxy, Isopropyloxy oder tert.-Butyloxy bedeuten, sowie Hydraten undSalzen von Verbindungen der Formel I, insbesondere pharmazeutisch verwendbaren Salzen von Verbindungen der Formel I.

3. Verfahren nach Anspruch 2 zur Herstellung von Verbindungen der Formel I, worin der Index n für 0 steht, R Wasserstoff, Niederalkanoyl mit bis zu 4 Kohlenstoffatomen, z.B. Formyl, Acetyl oder Propionyl, Halogenniederalkanoyl mit bis zu 4 Kohlenstoffatomen, z.B. 2-Chlor-, 2-Brom-, 2-Jod-, 2,2,2-Trifluor- oder 2,2,2-Trichloracetyl, gegebenenfalls durch Halogen, Niederalkoxy, z.B. Methoxy, oder Nitro substituiertes Benzoyl, z.B. 4-Chlorbenzoyl, 4-Methoxybenzoyl oder 4-Nitrobenzoyl, tert.-Niederalkoxycarbonyl, z.B. tert.-Butyloxycarbonyl, gegebenenfalls durch Nitro substituiertes Benzyloxycarbonyl, z.B. Benzyloxycarbonyl oder 4-Nitrobenzyloxycarbonyl, gegebenenfalls durch Niederalkoxy, z.B. Methoxy, substituiertes Diphenylmethoxycarbonyl, z.B. Benzhydryloxycarbonyl oder Bis-(4-methoxyphenyl)-methoxycarbonyl, Phenacyloxycarbonyl, 2-Halogenniederalkoxycarbonyl mit bis zu 4 Kohlenstoffatomen, z.B. 2,2,2-Trichlor-, 2-Chlor-, 2-Brom- oder 2-Jodäthoxycarbonyl, Carbamoyl, N-Niederalkylcarbamoyl, z.B. N-Methylcarbamoyl, N,N-Diniederalkylcarbamoyl, z.B. N,N-Dimethylcarbamoyl, Thiocarbamoyl, N-Niederalkylthiocarbamoyl, z.B. N-Methylthiocarbamoyl, N,N-Diniederalkylthiocarbamoyl, z.B. N,N-Dimethylthiocarbamoyl, Niederalkylsulfonyl, z.B. Methylsulfonyl oder Aethylsulfonyl, Arylsulfonyl, z.B. Phenylsulfonyl oder Toluylsulfonyl, Sulfamoyl, N-Niederalkylsulfamoyl, z.B. N-Methylsulfamoyl, oder N,N-Diniederalkylsulfamoyl, z.B. N,N-Dimethylsulfamoyl, $R_o$ Wasserstoff oder Niederalkyl, z.B. Methyl oder Aethyl, $R_1$ Wasserstoff oder eine Gruppe der Teilformel $(R)(R_o)N-$, worin R und $R_o$ die vorstehend genannten Bedeutungen haben, $R_2$ Carboxyl und $R_3$ Wasserstoff, Chlor oder Methoxy bedeuten, sowie Hydrate und Salze von sowie Hydraten und Salzen von Verbindungen der Formel I, insbesondere pharmazeutisch verwendbaren Salzen von Verbindungen der Formel I.

4. Verfahren nach Anspruch 3 zur Herstellung von Verbindungen der Formel I, worin der Index n für 0 steht, R Wasserstoff, Niederalkanoyl mit bis zu 4 Kohlenstoffatomen, z.B. Acetyl, Niederalkylsulfonyl mit bis zu 4 Kohlenstoffatomen, z.B. Methylsulfonyl oder Aethylsulfonyl, $R_o$ Wasserstoff, $R_1$ Wasserstoff oder eine Gruppe

der Teilformel (R)($R_o$)N-, worin R und $R_o$ die vorstehend genannten Bedeutungen haben, $R_2$ Carboxyl und $R_3$ Wasserstoff, Chlor oder Methoxy bedeuten, sowie Hydraten und Salzen von Verbindungen der Formel I, insbesondere pharmazeutisch verwendbaren Salzen von Verbindungen der Formel I.

5. Verfahren nach Anspruch 4 zur Herstellung von Verbindungen der Formel I, worin der Index n für 0 steht, R Wasserstoff, Niederalkanoyl mit bis zu 4 Kohlenstoffatomen, z.B. Acetyl, Niederalkylsulfonyl mit bis zu 4 Kohlenstoffatomen, z.B. Methylsulfonyl oder Aethylsulfonyl, $R_o$ Wasserstoff, $R_1$ Wasserstoff oder eine Gruppe der Teilformel (R)($R_o$)N-, worin R und $R_o$ die vorstehend genannten Bedeutungen haben, $R_2$ Carboxyl und $R_3$ Wasserstoff, Chlor oder Methoxy bedeuten, und worin die beiden Gruppen der Teilformel (R)($R_o$)N- die 3- und 5-Stellung (meta) und die Hydroxygruppe die 4-Stellung (para) am Phenylring einnehmen, sowie Hydrate und Salze von solchen Verbindungen, insbesondere pharmazeutisch verwendbare Salze von solchen Verbindungen der Formel I.

6. Verfahren zur Herstellung von 7β-[D-2-Amino-2-(3-methylsulfonylamino-4-hydroxyphenyl)-acetylamino]-3-methoxy-ceph-3-em-4-carbonsäure, gemäss Anspruch 5.

7. Verfahren zur Herstellung von 7β-[D-2-Amino-2-(3-methylsulfonylamino-4-hydroxyphenyl)-acetylamino]-ceph-3-em-4-carbonsäure, gemäss Anspruch 5.

8. Verfahren zur Herstellung von 7β-[D-2-Amino-2-(3-methylsulfonylamino-4-hydroxyphenyl)-acetylamino]-3-chlor-ceph-3-em-4-carbonsäure, gemäss Anspruch 5.

9. Verfahren zur Herstellung von 7β-[D-2-Amino-2-(3-amino-4-hydroxyphenyl)-acetylamino]-3-methoxy-ceph-3-em-4-carbonsäure, gemäss Anspruch 5.

10. Verfahren zur Herstellung von 7β-[D-2-Amino-2-(3-äthylsulfonylamino-4-hydroxyphenyl)-acetylamino]-ceph-3-em-4-carbonsäure, gemäss Anspruch 5.

11. Verfahren zur Herstellung von 7β-[D-2-Amino-2-(3-äthylsulfonylamino-4-hydroxyphenyl)-acetylamino]-3-methoxy-ceph-3-em-4-carbonsäure, gemäss Anspruch 5.

12. Verfahren zur Herstellung von 7β-[D-2-Amino-2-(3-äthylsulfonylamino-4-hydroxyphenyl)-acetylamino]-3-chlor-ecph-3-em-4-carbonsäure, gemäss Anspruch 5.

13. Verfahren zur Herstellung von 7β-[D-2-Amino-2-(3-acetylamino-4-hydroxyphenyl)-acetylamino]-3-methoxy-ceph-3-em-4-carbonsäure, gemäss Anspruch 5.